(19) Europäisches Patentamt
European Patent Office
Office européen des brevets

(11) EP 3 632 914 B1

(12) EUROPEAN PATENT SPECIFICATION

(45) Date of publication and mention of the grant of the patent:
**31.08.2022 Bulletin 2022/35**

(21) Application number: **18805272.4**

(22) Date of filing: **22.05.2018**

(51) International Patent Classification (IPC):
***C07D 471/04** (2006.01)* ***A61K 31/55** (2006.01)*
***A61P 31/20** (2006.01)*

(52) Cooperative Patent Classification (CPC):
(C-Sets available)
**A61P 31/20; A61K 31/522; A61K 31/553; A61K 31/675; A61P 1/16; C07D 498/04** (Cont.)

(86) International application number:
**PCT/CN2018/087852**

(87) International publication number:
**WO 2018/214875 (29.11.2018 Gazette 2018/48)**

# (54) HEPATITIS B VIRUS SURFACE ANTIGEN INHIBITOR

HEPATITIS-B-VIRUS-OBERFLÄCHENANTIGENHEMMER

INHIBITEUR D'ANTIGÈNE DE SURFACE DU VIRUS DE L'HÉPATITE B

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.05.2017 CN 201710365328**

(43) Date of publication of application:
**08.04.2020 Bulletin 2020/15**

(73) Proprietor: **Fujian Akeylink Biotechnology Co., Ltd.**
**Ningde, Fujian 355300 (CN)**

(72) Inventors:
- **DING, Charles Z.**
  **Shanghai 200131 (CN)**
- **SUN, Fei**
  **Shanghai 200131 (CN)**
- **HU, Yanbin**
  **Shanghai 200131 (CN)**
- **CHEN, Shuhui**
  **Shanghai 200131 (CN)**

(74) Representative: **Mewburn Ellis LLP**
**Aurora Building**
**Counterslip**
**Bristol BS1 6BX (GB)**

(56) References cited:
**WO-A1-2017/013046**
**WO-A1-2018/022282**
**WO-A1-2018/085619**
**CN-A- 105 899 508**
**US-A1- 2011 065 687**

EP 3 632 914 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/522, A61K 2300/00;**
**A61K 31/553, A61K 2300/00;**
**A61K 31/675, A61K 2300/00**

## Description

### Field of invention

The present disclosure relates to a novel 11-oxo-7,11-dihydro-6H-benzo[t]pyrido[1,2-*d*][1,4]oxazepine-10-carboxylic acid derivative serving as a hepatitis B virus surface antigen inhibitor. The invention is set out in the appended set of claims.

### Prior arts

Viral hepatitis B, abbreviated as hepatitis B, is a disease caused by Hepatitis B Virus (HBV) infection in the body. Hepatitis B virus is a hepadnaviridae that mainly exists in hepatocytes and causes damages to the hepatocytes, resulting in inflammation, necrosis and fibrosis of the hepatocytes. Viral hepatitis B is divided into acute hepatitis B and chronic hepatitis B. Most adults with acute hepatitis B can recover through their inherent immune function. However, chronic hepatitis B (CHB) has become a major challenge for global health care, and it is also the main cause of chronic liver diseases, cirrhosis and hepatocellular carcinoma (HCC). It is estimated that 2 billion people worldwide are infected with chronic hepatitis B virus, and more than 350 million people have progressed to hepatitis B. Every year, approximately 600,000 people die from the complications of chronic hepatitis B. China is a high-prevalence area of hepatitis B, and there are many patients with hepatitis B, which is a serious hazard. According to the data, there are about 93 million people infected with hepatitis B virus in China, and about 20 million of them are diagnosed with chronic hepatitis B, 10%-20% of which can progress to cirrhosis, and 1%-5% of which can progress to hepatocellular carcinoma.

The key to the functional cure for hepatitis B is to clear HBsAg (hepatitis B virus surface antigen) and produce surface antibodies. Quantification of HBsAg is a very important biological indicator. In chronically infected patients, a decrease in HBsAg and seroconversion are rarely observed, which is the end point of current treatment.

The surface antigen protein of hepatitis B virus (HBV) plays a very important role in the process of HBV invasion into liver cells, and is of great significance for the prevention and treatment of HBV infection. Surface antigen proteins include large (L), medium (M) and small (S) surface antigen proteins that share a common C-terminal S region. They are expressed from an open reading frame, the different lengths of which are determined by the three AUG start codons of the reading frame. These three surface antigen proteins include pre-S1/pre-S2/S, pre-S2/S and S domains. The HBV surface antigen protein is integrated into the endoplasmic reticulum (ER) membrane, which is initiated by the N-terminal signal sequence. Not only do they form the basic structure of virions, but they also form globular and filamentous subviral particles (SVPs, HBsAg) that accumulate in ER, host ER and pre-Golgi apparatus, and SVP contains most S surface antigen proteins. The L protein is critical in the morphogenesis of the virus and the interaction of the nucleocapsid, but is not necessary for the formation of SVP. Because of their lack of nucleocapsids, the SVPs are non-infectious. SVPs are greatly involved in disease progression, especially in response to hepatitis B virus. In the blood of infected people, the content of SVPs is at least 10,000 times that of the virus, trapping the immune system and weakening the body's immune response to hepatitis B virus. HBsAg also inhibits human innate immunity, inhibits the production of cytokines induced by polysaccharides (LPS) and IL-2, and inhibits DC function of dendritic cells and the induction activity of interfering kinase-1/2 of ERK-1/2 and c-Jun *N*-terminus in monocytes by LPS. It is worth noting that disease progression in cirrhosis and hepatocellular carcinoma is also largely associated with persistent secretion of HBsAg. These findings suggest that HBsAg plays an important role in the progress of chronic hepatitis.

The currently approved anti-HBV drugs are mainly immunomodulators (interferon-a and peginterferon-$\alpha$-2$\alpha$) and antiviral therapeutic drugs (Lamivudine, Adefovir Dipivoxil, Entecavir, Telbivudine, Tenofovir, Clevudine, etc.). Among them, the antiviral therapeutic drugs belong to nucleotides, and the mechanism of action thereof is to inhibit the synthesis of HBV DNA, instead of directly reducing HBsAg levels. As the prolonged treatment, HBsAg clearance rate exhibited by nucleotide drugs is similar to natural observations.

Clinically available therapies exhibit a poor efficacy in reducing HBsAg. Therefore, the development of oral small molecule inhibitors that can effectively reduce HBsAg is currently required for clinical use.

Roche has developed a surface antigen inhibitor called RG7834 for the treatment of hepatitis B, and has reported the efficacy of this compound in the woodchuck model against hepatitis B: RG7834 as a single drug can reduce 2.57 Log surface antigens and 1.7 Log HBV-DNA. The compound exhibits good activity, but it is necessary to separate the isomers in the process of molecular synthesis, which reduces the yield and increases the cost. The present disclosure obtains novel compounds having higher anti-hepatitis B biological activity, a more compact synthetic process, and better drug-like properties by structural modification.

WO2017013046A1 discloses a series of 2-oxo-7,8-dihydro-6*H*-pyrido[2,1,*a*][2]benzazepine-3-carboxylic acid derivative (the general structure shown below) for the treatment or prevention of hepatitis B virus infection. Embodiment 3 exhibiting the highest activity among this series of fused ring compounds, has an $IC_{50}$ of 419 nM which can be further improved greatly. The chiral centers contained in this series of compounds are difficult to synthesize asymmetrically.

Usually, the 7-membered carbon ring has a poor aqueous solubility and is susceptible to oxidative metabolism.

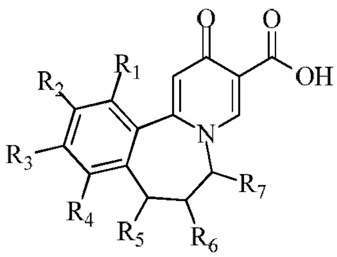

WO2018085619 published after the priority date of the present application. It relates to compounds described as useful for the treatment of HBV.

WO2018022282 published after the priority date of the present application. It relates to compounds described as useful for the treatment of HBV.

**Content of the present invention**

The present disclosure provides a compound of formula (I):

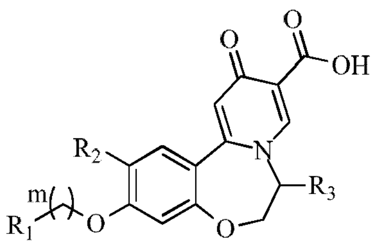

(I)

,

or a compound selected from the group consisting of:

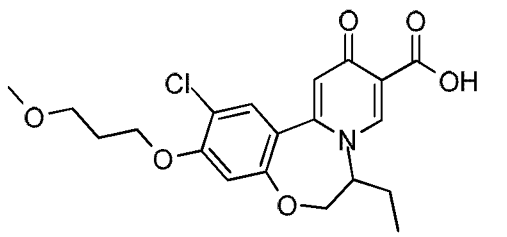

,

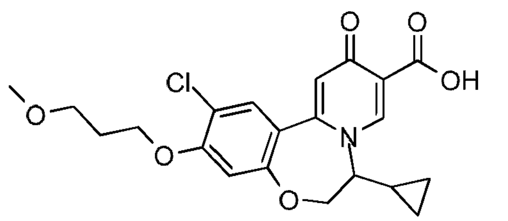

,

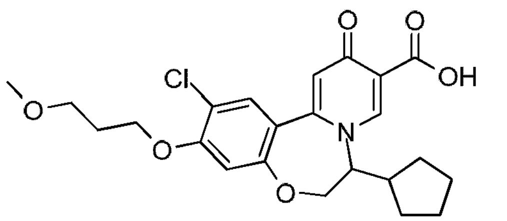

,

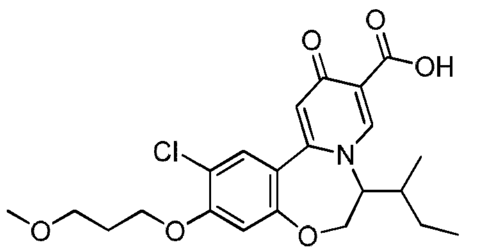

,

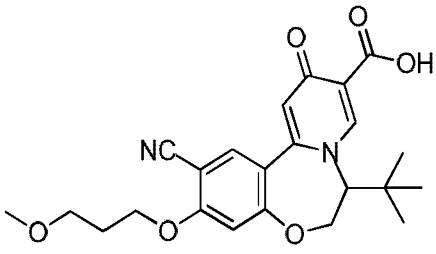,

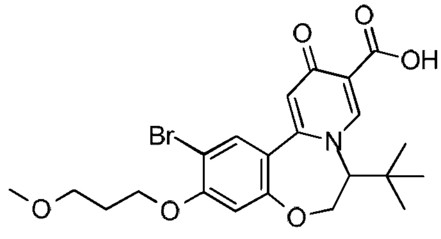,

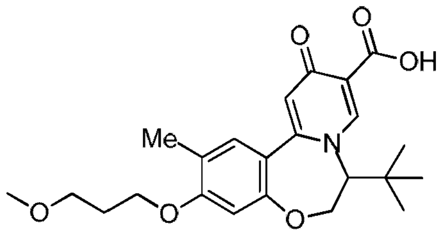,

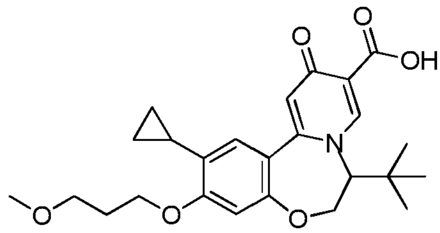,

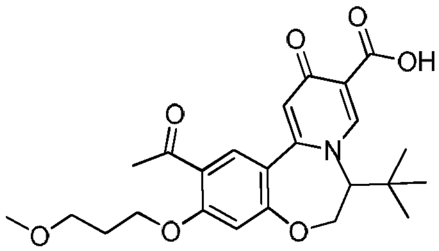;

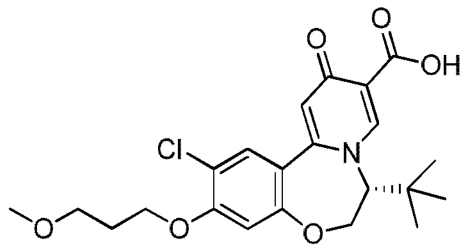

and

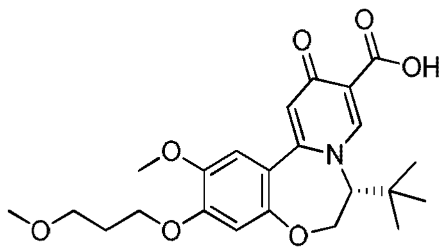;

or a pharmaceutically acceptable salt of the compound;
wherein, in the formula (I),
$R_1$ is OH, CN, $NH_2$,

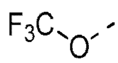,

or selected from the group consisting of

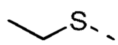,

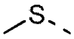, 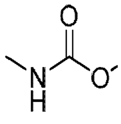, 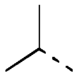, 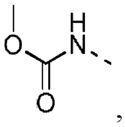, 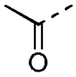, 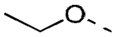, 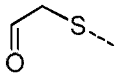, 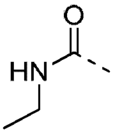,

$C_{2-5}$ alkenyl, $C_{2-5}$ heteroalkenyl, $C_{3-6}$ cycloalkyl and 3-6 membered heterocycloalkyl, wherein each of the

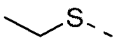, 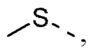, 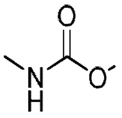, 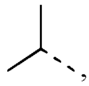, 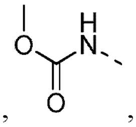, 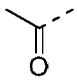, 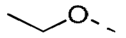, 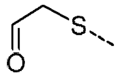, 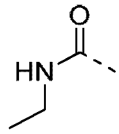,

$C_{2-5}$ alkenyl, $C_{2-5}$ heteroalkenyl, $C_{3-6}$ cycloalkyl and 3-6 membered heterocycloalkyl is optionally substituted by 1, 2 or 3 R;

m is 0, 1, 2, 3, 4 or 5; and

$R_1$ is not OH, CN, $NH_2$ provided m is 0;

or,

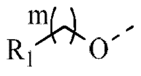 is 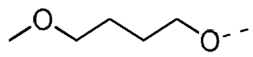;

$R_2$ is H, OH, CN, $NH_2$, halogen, or selected from the group consisting of $C_{1-3}$ alkyl, $C_{1-3}$ heteroalkyl, $C_{3-6}$ cycloalkyl and 3-6 membered heterocycloalkyl, wherein each of the $C_{1-3}$ alkyl, $C_{1-3}$ heteroalkyl, $C_{3-6}$ cycloalkyl and 3-6 membered heterocycloalkyl is optionally substituted by 1, 2 or 3 R;

$R_3$ is selected from the group consisting of $C_{1-6}$ alkyl and $C_{3-6}$ cycloalkyl, wherein each of the $C_{1-6}$ alkyl and $C_{3-6}$ cycloalkyl is optionally substituted by 1, 2 or 3 R;

Ris H, halogen, OH, CN, $NH_2$, or selected from the group consisting of $C_{1-3}$ alkyl and $C_{1-3}$ heteroalkyl, wherein each of the $C_{1-3}$ alkyl and $C_{1-3}$ heteroalkyl is optionally substituted by 1, 2 or 3 R';

R' is selected from the group consisting of F, Cl, Br, I, OH, CN, $NH_2$, $CH_3$, $CH_3CH_2$, $CH_3O$, $CF_3$, $CHF_2$ and $CH_2F$;

the "hetero" refers to heteroatom or heteroatomic group; the "hetero" in the $C_{2-5}$ heteroalkenyl, 3-6 membered heterocycloalkyl, $C_{1-3}$ heteroalkyl is independently selected from the group consisting of -C(=O)N(R)-, -N(R)-, -C(=NR)-, -(R)C=N-, $-S(=O)_2N(R)-$, -S(=O)N(R)-, N, - O-, -S-, =O, =S, -C(=O)O-, -C(=O)-, -C(=S)-, -S(=O)-, $-S(=O)_2-$ and -N(R)C(=O)N(R)-;

in any of the above cases, the number of the heteroatom or the heteroatomic group is independently 1, 2 or 3.

In some embodiments of the present disclosure, R is H, F, Cl, Br, I, OH, CN, $NH_2$, $CH_3$, $CH_3CH_2$, $CH_3O$, $CF_3$, $CHF_2$ or $CH_2F$.

In some embodiments of the disclosure, $R_1$ is OH, CN, $NH_2$, or selected from the group consisting of $C_2$-$_3$ alkenyl, $C_2$-$_3$ heteroalkenyl, $C_{3-6}$ cycloalkyl and 3-6 membered heterocycloalkyl, wherein each of the $C_2$-$_3$ alkenyl, $C_2$-$_3$ heteroalkenyl, $C_{3-6}$ cycloalkyl and 3-6 membered heterocycloalkyl is optionally substituted by 1, 2 or 3 R.

In some embodiments of the present disclosure, $R_1$ is OH, CN, $NH_2$, or selected from the group consisting of

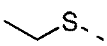, 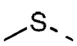, 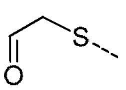, 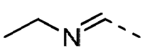, 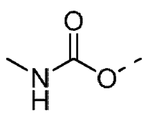, 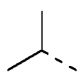,

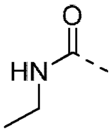, 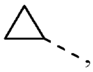, 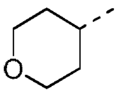, 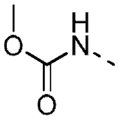, 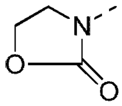, 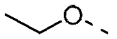 and 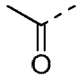,

wherein each of the

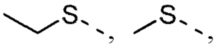, 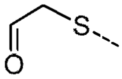, 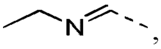, 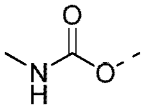, 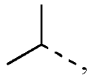, 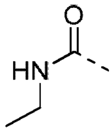, 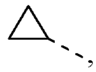,

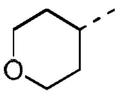, 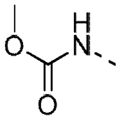, 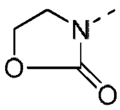, 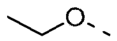 and 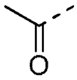

is optionally substituted by 1, 2 or 3 R.

In some embodiments of the present disclosure, $R_1$ is OH, CN, $NH_2$,

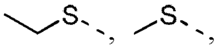,

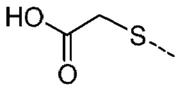, 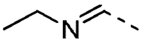, 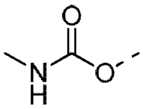, 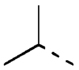, 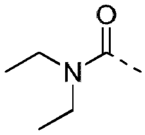, 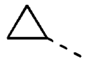, 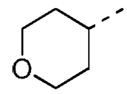,

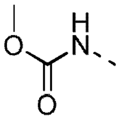, 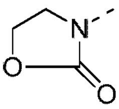, 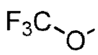, 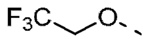, 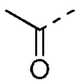.

In some embodiments of the present disclosure, $R_2$ is H, OH, CN, $NH_2$, halogen, or selected from the group consisting of $C_{1-3}$ alkyl, $C_{1-3}$ heteroalkyl and $C_{3-6}$ cycloalkyl, wherein each of the $C_{1-3}$ alkyl, $C_{1-3}$ heteroalkyl and $C_{3-6}$ cycloalkyl is optionally substituted by 1, 2 or 3 R.

In some embodiments of the present disclosure, $R_2$ is H, OH, CN, $NH_2$, F, Cl, Br, I, or selected from the group consisting of $CH_3$,

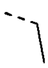, 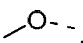, 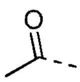 and 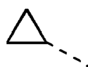,

wherein each of the $CH_3$,

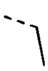, 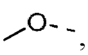, 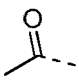 and 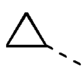

is optionally substituted by 1, 2 or 3 R.

In some embodiments of the present disclosure, $R_2$ is selected from the group consisting of Cl, Br, CN, $CH_3$,

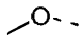, 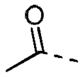 and 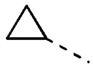.

In some embodiments of the present disclosure, $R_3$ is selected from the group consisting of $C_{l\text{-}4}$ alkyl and $C_{3\text{-}6}$ cycloalkyl, wherein each of the $C_{1\text{-}4}$ alkyl and $C_{3\text{-}6}$ cycloalkyl is optionally substituted by 1, 2 or 3 R.

In some embodiments of the present disclosure, $R_3$ is selected from the group consisting of

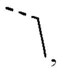, 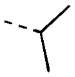, 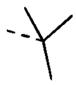, 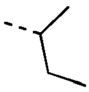, 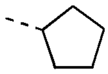 and 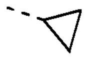.

In some embodiments of the present disclosure, m is selected from the group consisting of 0, 1, 2, 3 and 4; and $R_1$ is not OH, CN, $NH_2$ provided m is 0.

In some embodiments of the present disclosure, the moiety

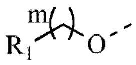

is selected from the group consisting of

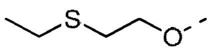, 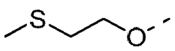, 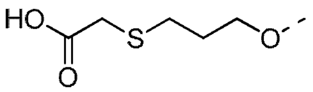,

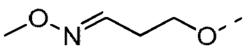, 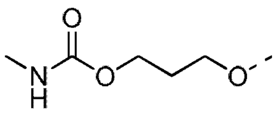, 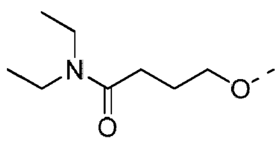,

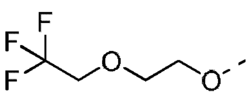, 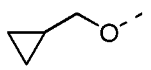, 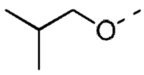, 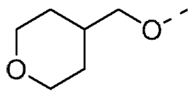, 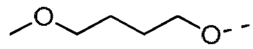,

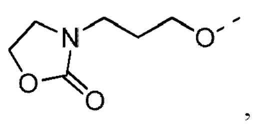, 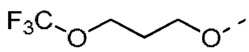, 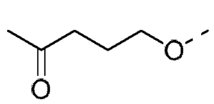 and 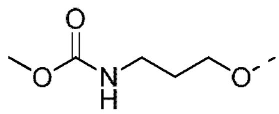.

In some embodiments of the present disclosure, provided herein is a compound of formula (II), (III) or (IV), or a pharmaceutically acceptable salt thereof,

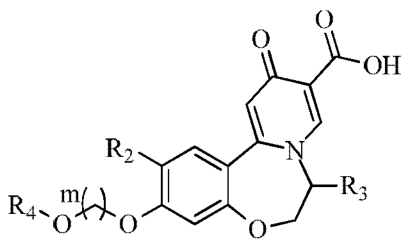

(II)

,

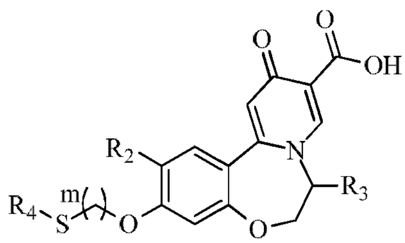

(III)

and

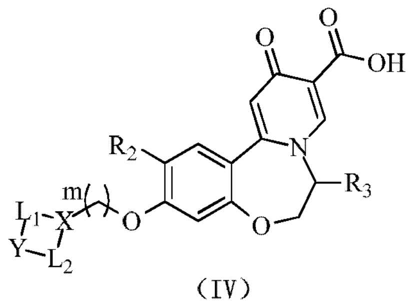

(IV)

,

wherein,

$R_4$ is H, or selected from the group consisting of $C_{1-3}$ alkyl and $C_{1-3}$ heteroalkyl, wherein each of the $C_{1-3}$ alkyl and $C_{1-3}$ heteroalkyl is optionally substituted by 1, 2 or 3 R;

X is selected from the group consisting of C and N;

Y is selected from the group consisting of O and C;

each of $L_1$ and $L_2$ is independently selected from the group consisting of a single bond, - $(CH_2)_n$- and -C(=O)-;

with the provision that $L_1$ and $L_2$ are not both a single bond;

n is 1 or 2;

m, R, $R_2$, $R_3$ and the "hetero" in $C_{1-3}$ heteroalkyl are as defined above; and $R_4$ is not H provided m is 0.

The present disclosure also provides a compound or a pharmaceutically acceptable salt thereof, wherein the compound is selected from the group consisting of

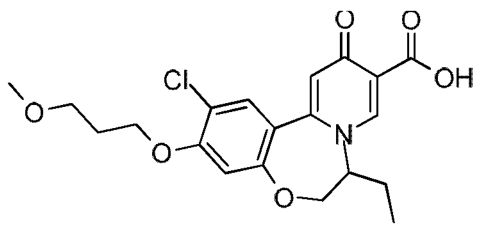

,

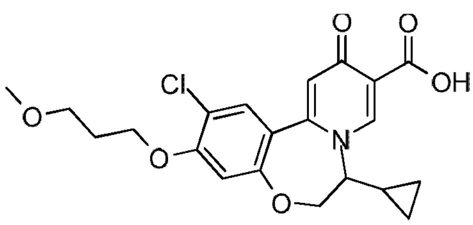

,

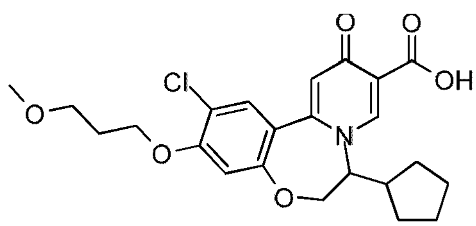

,

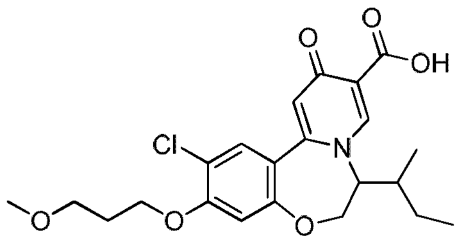,

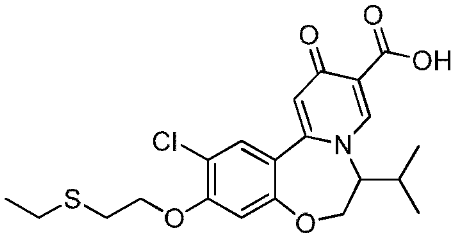, 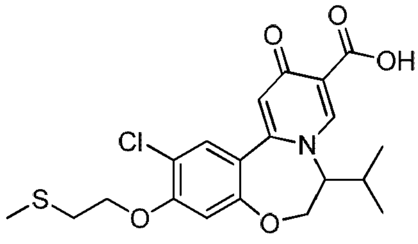,

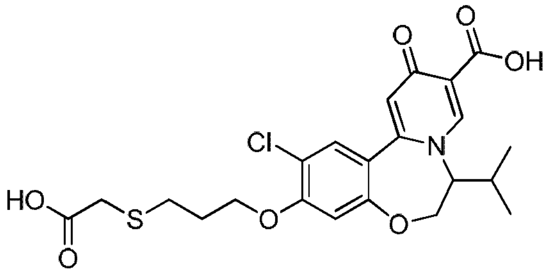, 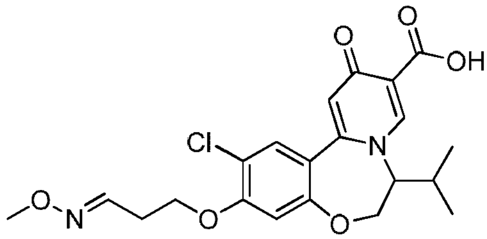,

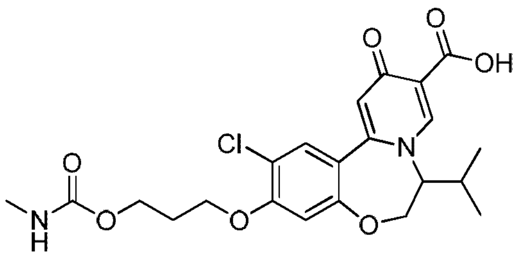, 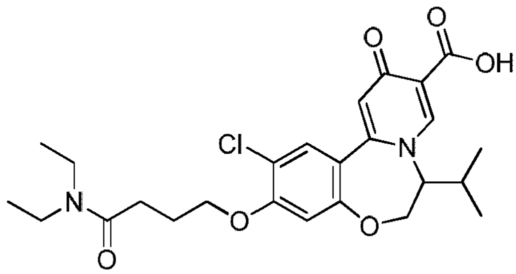,

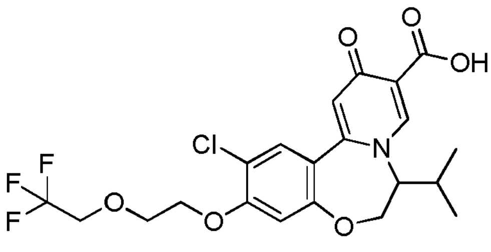,

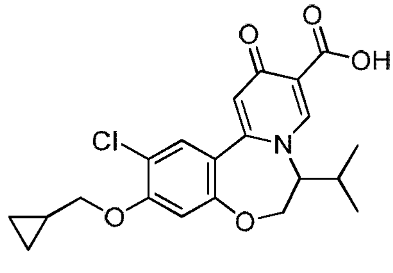, 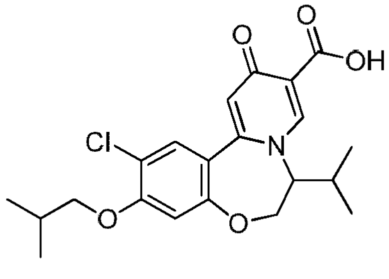,

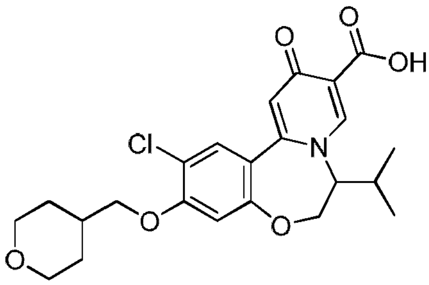

,

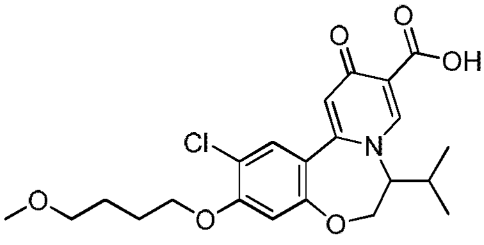

,

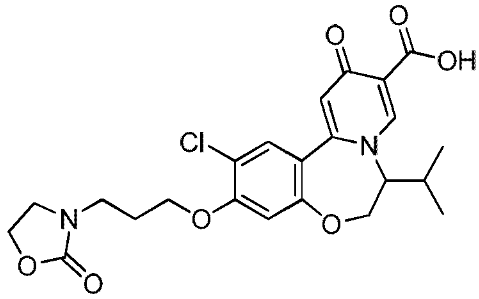

,

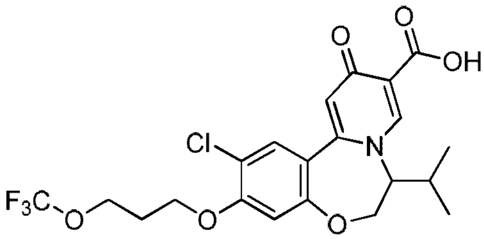

,

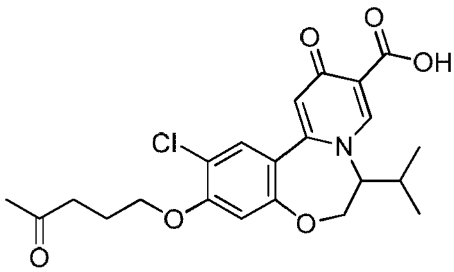

,

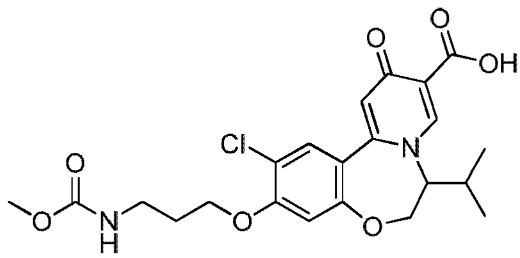

,

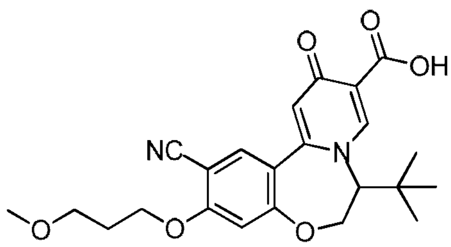

,

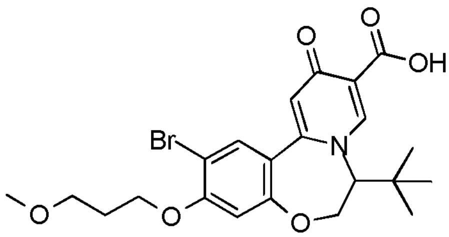

,

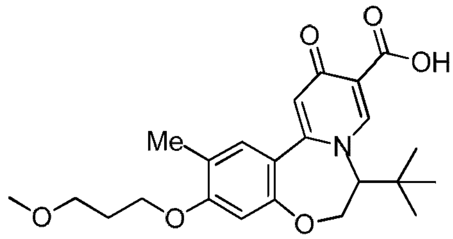,
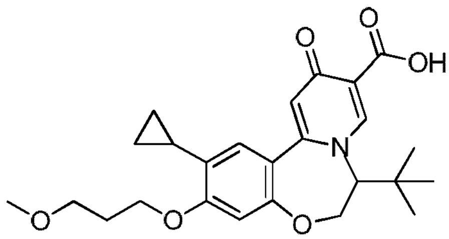

and

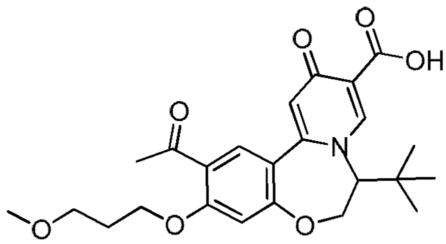.

In some embodiments of the present disclosure, the compound is selected from the group consisting of

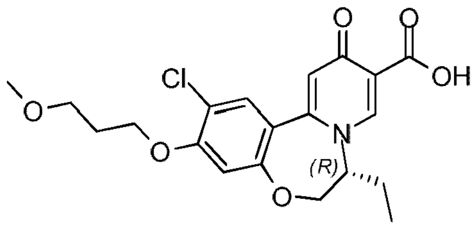,

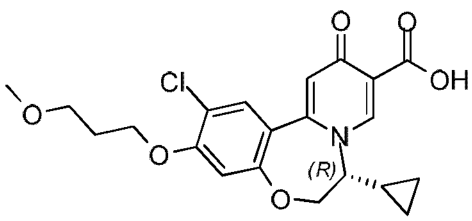,
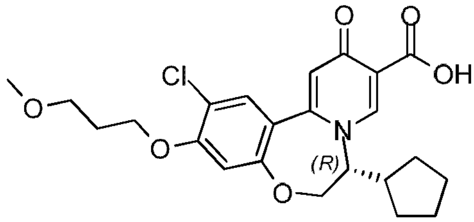,

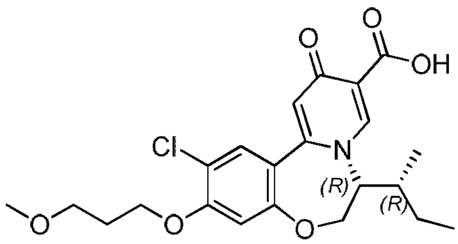,
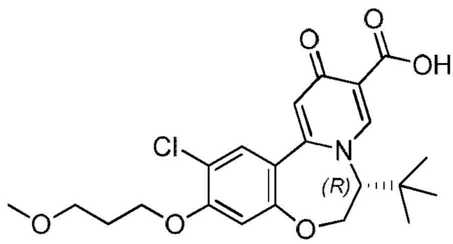,

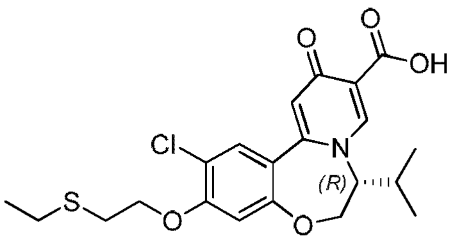,
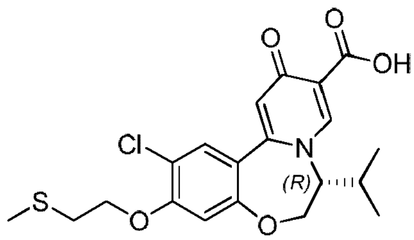,

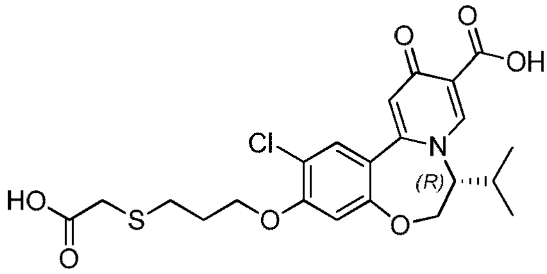
,
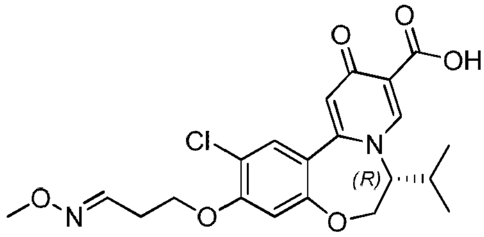
,

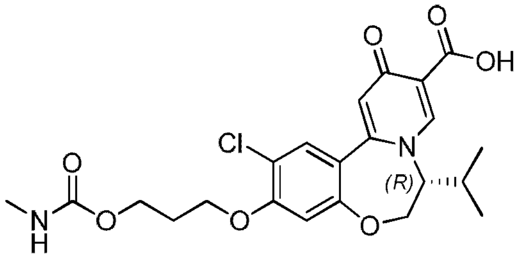
,
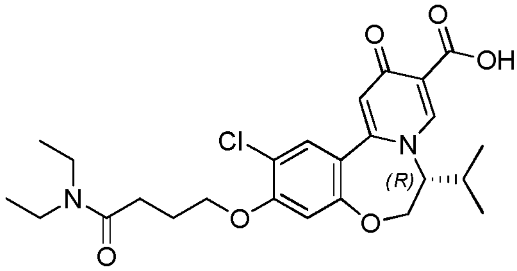
,

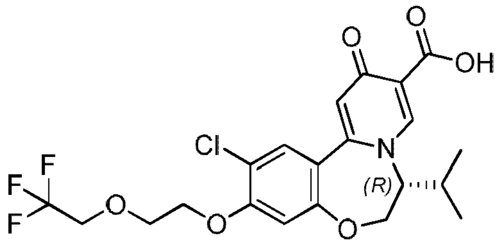
,

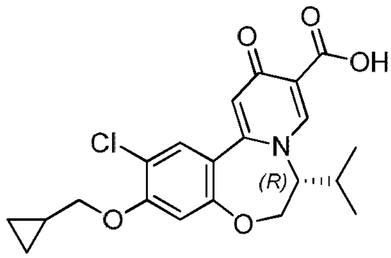
,
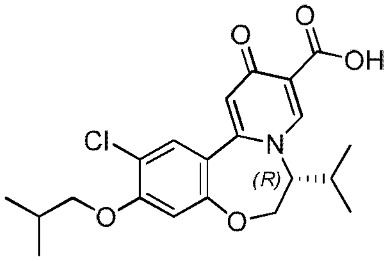
,

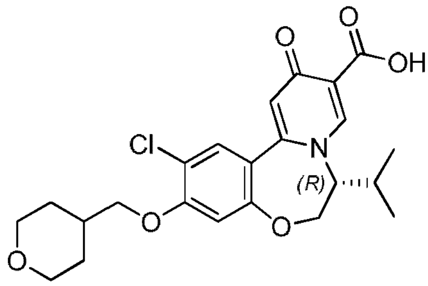
,

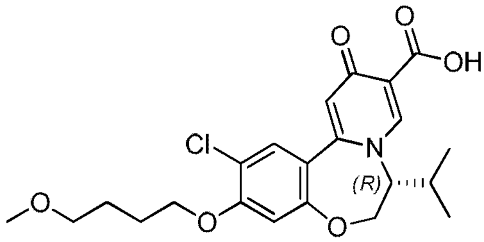 , 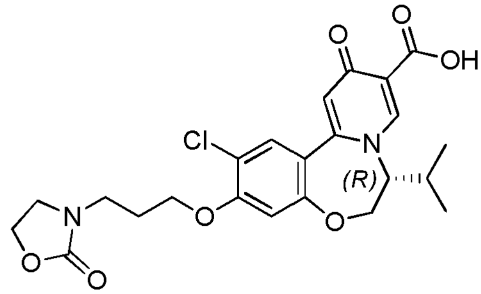 ,

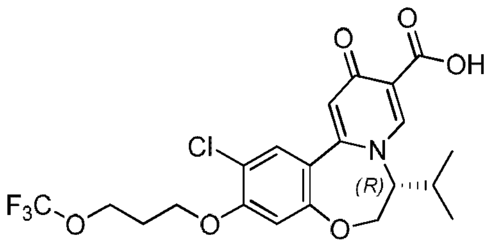 , 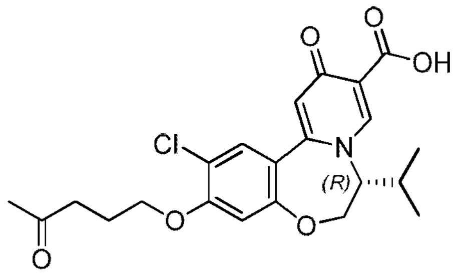 ,

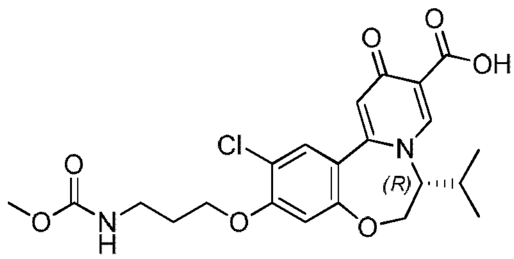 , 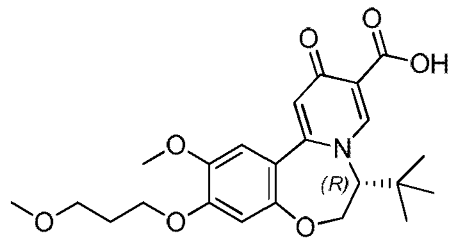 ,

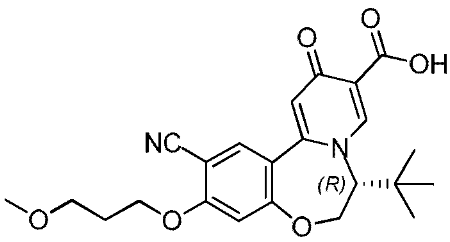 , 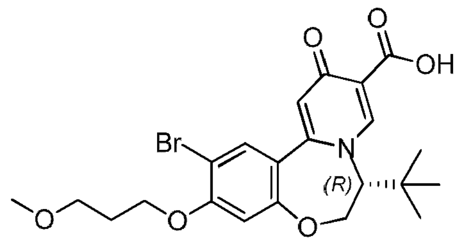 ,

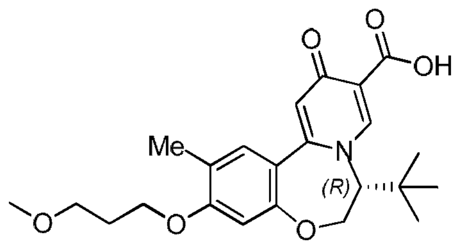 , 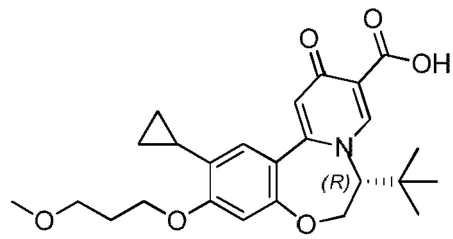

and

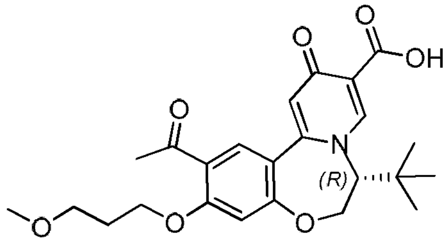

.

The present disclosure also provides a pharmaceutical composition comprising a therapeutically effective amount of the compound or the pharmaceutically acceptable salt thereof, and a pharmaceutically acceptable carrier.

The present disclosure also provides the compound or the pharmaceutically acceptable salt thereof, or the pharmaceutical composition for use in treating hepatitis B.

Other embodiments of the present disclosure can be obtained by the arbitrary combination of the above variables.

**Technical effect**

The present disclosure creatively designs and synthesizes a novel series of compounds having a seven-membered oxazepine as a core structure. The compounds of the present disclosure have high anti-HBV activity *in vitro,* and the $EC_{50}$ values of the most active compounds in inhibiting HBV-DNA and HBsAg are below 1 nM. The chiral center of the compound of the present disclosure is prepared by using commercial amino acid as a raw material, and the synthesis process is simple and economical. Compared with the prior art, after the carbon on the seven-membered ring is replaced by oxygen, the aqueous solubility of the compound is increased, and the risk of oxidative metabolism is lowered, thereby more excellent druggability is obtained.

The compounds of the present disclosure have a moderate plasma protein binding rate, and do not inhibit cytochrome P450 isoenzyme, exhibiting a low risk of drug-drug interaction. The compounds of the present disclosure exhibit excellent stability in the liver microsomes of three species of rats, human and mice, indicating that the compounds are not easily metabolized. The compounds of the present disclosure exhibit better exposure and bioavailability in the pharmacokinetic study on mice and rats. The compounds of the present disclosure exhibit a good anti-HBV activity in both *in vivo* pharmacodynamic study on hydrodynamic injection mouse HBV model (HDI-HBV) via tail vein and Hepatitis B virus mouse model (AAV-HBV) mediated by recombinant adeno-associated virus type 8 vector. The compounds of the present disclosure exhibit a good tolerance in the 14-day pre-toxicology test in rats and exhibit a good tolerance in a single-dose neurotoxicity test.

The (*R*)-configuration compound of the present disclosure has a 3 to 5-fold increase in anti-HBV activity compared to the racemic compound of the present disclosure, and has a 10-fold increase in anti-HBV activity compared to the (*S*)-configuration compound of the present disclosure.

**Definition and description**

Unless otherwise indicated, the following terms when used in the descriptions and the claims of the disclosure have the following meanings. A specific term or phrase should not be considered indefinite or unclear in the absence of a particular definition, but should be understood in the ordinary sense. When a trade name appears herein, it is intended to refer to its relative commodity or active ingredient thereof. The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" refers to a salt of the compound of the disclosure that is prepared by reacting the compound having a specific substituent of the disclosure with a relatively non-toxic acid or base. When the compound of the disclosure contains a relatively acidic functional group, a base addition salt can be obtained by bringing the neutral form of the compound into contact with a sufficient amount of base in a pure solution or a suitable inert solvent. The pharmaceutically acceptable base addition salt includes a salt of sodium, potassium, calcium, ammonium, organic amine or magnesium or similar salts. When the compound of the disclosure contains a relatively basic functional group, an acid addition salt can be obtained by bringing the neutral form of the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the

organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, and methanesulfonic acid, and the like; and an salt of amino acid (such as arginine and the like), and a salt of an organic acid such as glucuronic acid and the like (referred to Berge et al., "Pharmaceutical Salts", Journal of Pharmaceutical Science 66: 1-19 (1977)). Certain specific compounds of the disclosure that contain both basic and acidic functional groups can be converted to any base or acid addition salt.

Preferably, through bringing the salt into contact with a base or an acid in a conventional manner, then separating the parent compound, the neutral form of the compound is thereby regenerated. The difference between the parent form of the compound and its various salt forms lies in specific physical properties, such as different solubility in a polar solvent.

"Pharmaceutically acceptable salt" used herein belongs to a derivative of the compound of the present disclosure, wherein, the parent compound is modified by forming a salt with an acid or a base. Examples of the pharmaceutically acceptable salt include but are not limited to an inorganic acid or organic acid salt of a basic moiety such as amine, an alkali metal salt or an organic salt of an acidic moiety such as carboxylic acid, and the like. The pharmaceutically acceptable salt includes conventional non-toxic salt or quaternary ammonium salt of the parent compound, such as a salt formed by a non-toxic inorganic acid or an organic acid. The conventional non-toxic salt includes but is not limited to the salt derived from an inorganic acid and an organic acid, wherein the inorganic acid or organic acid is selected from the group consisting of 2-acetoxybenzoic acid, 2-hydroxyethanesulfonic acid, acetic acid, ascorbic acid, benzenesulfonic acid, benzoic acid, bicarbonate, carbonic acid, citric acid, edetic acid, ethanedisulfonic acid, ethanesulfonic acid, fumaric acid, glucoheptose, gluconic acid, glutamic acid, glycolic acid, hydrobromic acid, hydrochloric acid, hydroiodide, hydroxyl, hydroxynaphthalene, isethionic acid, lactic acid, lactose, dodecyl sulfonic acid, maleic acid, malic acid, mandelic acid, methanesulfonic acid, nitric acid, oxalic acid, pamoic acid, pantothenic acid, phenylacetic acid, phosphoric acid, polygalactanal acid, propionic acid, salicylic acid, stearic acid, subacetic acid, succinic acid, sulfamic acid, sulfanilic acid, sulfuric acid, tannin, tartaric acid and *p*-toluenesulfonic acid.

The pharmaceutically acceptable salt of the disclosure can be prepared from the parent compound that contains an acidic or basic moiety by conventional chemical method. Generally, such salt can be prepared by reacting the free acid or base form of the compound with a stoichiometric amount of an appropriate base or acid in water or an organic solvent or a mixture thereof. Generally, the non-aqueous media such as ether, ethyl acetate, ethanol, isopropanol or acetonitrile is preferred.

Certain compounds of the disclosure can exist in a nonsolvated form or a solvated form, including hydrated form. Generally, the solvated form is equivalent to the nonsolvated form, and both are encompassed within the scope of the disclosure.

Certain compounds of the present disclosure can have an asymmetric carbon atom (optical center) or a double bond. The racemate, diastereomer, geometric isomer and individual isomer are all encompassed within the scope of the present disclosure.

Unless otherwise specified, the absolute configuration of a stereogenic center is represented by a wedged solid bond

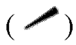

and a wedged dashed bond

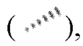

a wave line

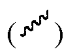

represents a wedged solid bond

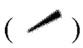

or a wedged dashed bond

( 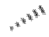 ),

and the relative configuration of a stereogenic center is represented by a straight solid bond

( 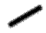 )

and a straight dashed bond

( 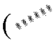 ).

When the compound described herein contains an olefinic double bond or other geometric asymmetric centers, *E* and *Z* geometric isomers are included unless otherwise specified. Likewise, all tautomeric forms are encompassed within the scope of the disclosure.

Unless otherwise specified, the terms "enriched in one isomer", "isomer enriched", "enriched in one enantiomer" or "enantiomer enriched" refer to the content of one of the isomers or enantiomers is less than 100%, and the content of the isomer or enantiomer is 60% or more, or 70% or more, or 80% or more, or 90% or more, or 95% or more, or 96% or more, or 97% or more, or 98% or more, or 99% or more, or 99.5% or more, or 99.6% or more, or 99.7% or more, or 99.8% or more, or 99.9% or more.

Unless otherwise specified, the terms "excess of isomer" or "excess of enantiomer" refers to the difference between the relative percentages of the two isomers or enantiomers. For example, wherein, the content of one of the isomers or enantiomers is 90%, and the other one is 10%, then the excess of isomer or enantiomer (ee value) is 80%.

The compound of the disclosure may have a specific geometric or stereoisomeric form. The disclosure contemplates all such compounds, including *cis* and *trans* isomer, (-)- and (+)-enantiomer, (*R*)- and (*S*)-enantiomer, diastereoisomer, (*D*)-isomer, (*L*)-isomer, and racemic mixture and other mixtures, for example, an enantiomer or diastereoisomer enriched mixture, all of which are encompassed within the scope of the disclosure. The substituent such as alkyl may have an additional asymmetric carbon atom. All these isomers and mixtures thereof are encompassed within the scope of the disclosure.

Optically active (*R*)- and (*S*)-isomer, or *D* and *L* isomer can be prepared using chiral synthesis or chiral reagents or other conventional techniques. If one kind of enantiomer of certain compound of the disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as amino) or an acidic functional group (such as carboxyl), the compound reacts with an appropriate optically active acid or base to form a salt of the diastereomeric isomer which is then subjected to diastereomeric resolution through the conventional method in the art to give the pure enantiomer. In addition, the enantiomer and the diastereoisomer are generally isolated through chromatography which uses a chiral stationary phase and optionally combines with a chemical derivative method (such as carbamate generated from amine).

The compound of the disclosure may contain an unnatural proportion of atomic isotope at one or more than one atom(s) that constitute the compound. For example, the compound can be radiolabeled with a radioactive isotope, such as tritium ($^{3}H$), iodine-125 ($^{125}I$) or C-14 ($^{14}C$). All isotopic variations of the compound of the disclosure, whether radioactive or not, are encompassed within the scope of the disclosure.

The term "pharmaceutically acceptable carrier" refers to any agent or carrier medium which is capable of delivering an effective amount of the active substance of the present disclosure, does not interfere with the biological activity of the active substance and has no toxic side effect on the host or patient. The representative carrier includes water, oil, vegetable and mineral, cream base, lotion base, ointment base and the like. The base includes a suspending agent, a thickener, a penetration enhancer and the like. Their formulations are well known to the skilled in the cosmetic field or the topical pharmaceutical field. The additional information about the carrier can be referred to Remington: The Science and Practice of Pharmacy, 21st Ed., Lippincott, Williams & Wilkins (2005).

The term "excipient" generally refers to a carrier, diluent and/or vehicle required to formulate an effective pharmaceutical composition.

For a medicament or a pharmacologically active agent, the term "effective amount" or "therapeutically effective amount" refers to a nontoxic but sufficient amount to achieve a desired effect of the medicament or the agent. For the oral dosage form of the present disclosure, an "effective amount" of the active substance in the composition refers to an amount required for achieving a desired effect when combining with another active substance in the composition. The effective amount varies from person to person and is determined depending on the age and general condition of the recipient as well as the specific active substance. The appropriate effective amount in an individual case can be

determined by the skilled in the art based on routine experiment.

The term "active ingredient", "therapeutic agent", "active substance" or "active agent" refers to a chemical entity which can effectively treat the target disorder, disease or condition.

"Optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted with the substituent, including deuterium and hydrogen variants, as long as the valence of the specific atom is normal and the substituted compound is stable. When the substituent is a ketone (i.e., =O), it means two hydrogen atoms are substituted. Positions on an aromatic ring cannot be substituted with a ketone. The term "optionally substituted" means an atom can be substituted with a substituent or not, unless otherwise specified, the type and number of the substituent may be arbitrary as long as being chemically achievable.

When any variable (such as R) occurs in the constitution or structure of the compound more than once, the definition of the variable at each occurrence is independent. Thus, for example, if a group is substituted with 0-2 R, the group can be optionally substituted with up to two R, wherein the definition of R at each occurrence is independent. Moreover, a combination of the substituent and/or the variant thereof is allowed only when the combination results in a stable compound.

When the number of a linking group is 0, such as -(CRR)o-, it means that the linking group is a single bond.

When one of the variables is selected from a single bond, it means that the two groups linked by the single bond are connected directly. For example, when L in A-L-Z represents a single bond, the structure of A-L-Z is actually A-Z.

When a substituent is vacant, it means that the substituent does not exist. For example, when X is vacant in A-X, the structure of A-X is actually A. When a bond of a substituent can be cross-linked to more than one atom on a ring, such substituent can be bonded to any atom of the ring. For example, the structural unit

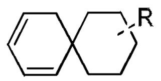 or 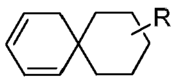

means that the substituent R can be located at any position on cyclohexyl or cyclohexadiene. When the enumerative substituent does not indicate by which atom it is linked to the group to be substituted, such substituent can be bonded by any atom thereof. For example, when pyridyl acts as a substituent, it can be linked to the group to be substituted by any carbon atom on the pyridine ring. When the enumerative linking group does not indicate the direction for linking, the direction for linking is arbitrary, for example, the linking group L contained in

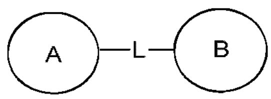

is -M-W-, then -M-W- can link ring A and ring B to form

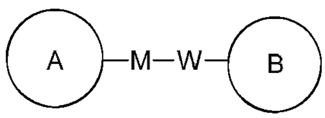

in the direction same as left-to-right reading order, and form

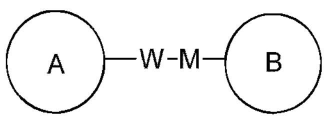

in the direction contrary to left-to-right reading order. A combination of substituents and/or variants thereof is allowed only when such combination can result in a stable compound.

Unless otherwise specified, the term "hetero" represents a heteroatom or a heteroatomic group (e.g., an atomic group containing a heteroatom), including the atom except carbon (C) and hydrogen (H) and the atomic group containing the above heteroatom, for example, including oxygen (O), nitrogen (N), sulfur (S), silicon (Si), germanium (Ge), aluminum

(Al), boron (B), -O-, -S-, =O, =S, -C(=O)O-, -C(=O)-, -C(=S)-, -S(=O), $-S(=O)_2-$, and the group consisting of -C(=O)N(H)-, - N(H)-, -C(=NH)-, $-S(=O)_2N(H)-$ and -S(=O)N(H)-, each of which is optionally substituted.

Unless otherwise specified, the term "ring" refers to a substituted or unsubstituted cycloalkyl, heterocycloalkyl, cycloalkenyl, heterocycloalkenyl, cycloalkynyl, heterocycloalkynyl, aryl or heteroaryl. The so-called ring includes a single ring, a double ring, a spiral ring, a fused ring or a bridged ring. The number of the atom on the ring is usually defined as the member number of the ring, for example, a "5-7 membered ring" means that 5 to 7 atoms are arranged on a ring. Unless otherwise specified, the ring optionally contains 1 to 3 heteroatoms. Therefore, a "5-7 membered ring" includes, for example, phenyl, pyridinyl and piperidinyl; on the other hand, the term "5-7 membered heterocycloalkyl ring" includes pyridyl and piperidinyl, but excluding phenyl. The term "ring" also includes a ring system containing at least one ring, wherein each ring independently meets the above definition.

Unless otherwise specified, the term "heterocycle" or "heterocyclo" refers to a stable monocyclic, bicyclic or tricyclic ring containing a heteroatom or a heteroatom group, which can be saturated, partially unsaturated or unsaturated (aromatic) and can contain carbon atoms and 1, 2, 3 or 4 ring heteroatoms independently selected from N, O and S, wherein any of the above heterocycle can be fused to a benzene ring to form a bicyclic ring. Nitrogen and sulfur heteroatoms can optionally be oxidized (i.e., NO and S(O)p, p is 1 or 2). Nitrogen atom can be substituted or unsubstituted (i.e., N or NR, wherein R is H or other substituents already defined herein). The heterocycle can be attached to the pendant group of any heteroatom or carbon atom to form a stable structure. If the resulting compound is stable, the heterocycle described herein may have a substitution at a carbon or nitrogen position. Nitrogen atom on the heterocycle is optionally quaternized. In a preferred embodiment, when the total number of S and O atom of the heterocycle is more than 1, the heteroatom is not adjacent to each other. In another preferred embodiment, the total number of S and O atom of the heterocycle is not more than 1. As used herein, the term "aromatic heterocyclic group" or "heteroaryl" refers to a stable 5-, 6- or 7-membered monocyclic or bicyclic or 7-, 8-, 9- or 10-membered bicyclic heterocyclic aromatic ring which contains carbon atoms and 1, 2, 3 or 4 ring heteroatoms independently selected from N, O and S. Nitrogen atom can be substituted or unsubstituted (i.e., N or NR, wherein R is H or other substituents already defined herein). Nitrogen and sulfur heteroatoms may optionally be oxidized (i.e., NO and $S(O)_p$, p is 1 or 2). It is worth noting that the total number of S and O atom of an aromatic heterocycle is not more than one. The bridged ring is also included in the definition of the heterocycle. A bridged ring is formed when one or more than one atom (i.e., C, O, N or S) link two non-adjacent carbon or nitrogen atoms. A preferred bridged ring includes, but not limited to one carbon atom, two carbon atoms, one nitrogen atom, two nitrogen atoms and one carbon-nitrogen group. It is worth noting that a bridge always converts a monocyclic ring to a tricyclic ring. In a bridged ring, the substituent on the ring may also be present on the bridge.

Examples of the heterocyclic compound include, but are not limited to: acridinyl, azocinyl, benzimidazolyl, benzofuranyl, benzomercaptofuranyl, benzomercaptophenyl, benzoxazolyl, benzoxazolinyl, benzothiazolyl, benzotriazolyl, benzotetrazolyl, benzoisoxazolyl, benzoisothiazolyl, benzoimidazolinyl, carbazolyl, 4a*H*-carbazolyl, carbolinyl, chromanyl, chromene, cinnolinyl decahydroquinolinyl, 2*H*,6*H*-1,5,2-dithiazinyl, dihydrofuro[2,3-*b*]tetrahydrofuranyl, furanyl, furazanyl, imidazolidinyl, imidazolinyl, imidazolyl, 1*H*-indazolyl, indolenyl, indolinyl, indolizinyl, indolyl, 3*H*-indolyl, isobenzofuranyl, isoindolyl, isoindolinyl, isoquinolinyl, isothiazolyl, isoxazolyl, methylenedioxyphenyl, morpholinyl, naphthyridinyl, octahydro-isoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, hydroxindolyl, pyrimidinyl, phenanthridinyl, phenanthrolinyl, phenazine, phenothiazine, benzoxanthinyl, phenoloxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolyl, pyridazinyl, pyrido-oxazolyl, pyrido-imidazolyl, pyrido-thiazolyl, pyridinyl, pyrrolidinyl, pyrrolinyl, 2*H*-pyrrolyl, pyrrolyl, quinazolinyl, quinolinyl, 4*H*-quinolizinyl, quinoxalinyl, quinuclidinyl, tetrahydrofuranyl, tetrahydroisoquinolinyl, tetrahydroquinolinyl, tetrazolyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, isothiazolylthienyl, thieno-oxazolyl, thieno-thiazolyl, thieno-imidazolyl, thienyl, triazinyl, 1*H*-1,2,3-triazolyl, 2*H*-1,2,3-triazolyl, 1*H*-1,2,4-triazolyl, 4*H*-1,2,4-triazolyl and xanthenyl. Also included are fused-ring compounds and spiro compounds.

Unless otherwise specified, the term "hydrocarbyl" or its hyponyms (e.g., alkyl, alkenyl, alkynyl, and aryl, etc.), by itself or as part of another substituent, refers to a linear, branched chain or cyclic hydrocarbon radical or any combination thereof. They can be fully saturated (e.g., alkyl), mono- or polyunsaturated (e.g., alkenyl, alkynyl, and aryl), can be mono-, di- or poly-substituted, can be monovalent (e.g., methyl), divalent (e.g., methylene) or multivalent (e.g., methenyl), can also include a divalent or multivalent group, have a specified number of carbon atom (for example, $C_1$-$C_{12}$ indicates 1 to 12 carbon atoms, $C_{1-12}$ is selected from $C_1$, $C_2$, $C_3$, $C_4$, $C_5$, $C_6$, $C_7$, $C_8$, $C_9$, $C_{10}$, $C_{11}$ and $C_{12}$; $C_{3-12}$ is selected from $C_3$, $C_4$, $C_5$, $C_6$, $C_7$, $C_8$, $C_9$, $C_{10}$, $C_{11}$ and $C_{12}$). The term "hydrocarbyl" includes, but is not limited to aliphatic hydrocarbyl and aromatic hydrocarbyl. The aliphatic hydrocarbyl includes linear and cyclic hydrocarbyl, specifically includes but not limited to alkyl, alkenyl, and alkynyl. The aromatic hydrocarbyl includes but is not limited to 6-12 membered aromatic hydrocarbyl such as phenyl, naphthyl and the like. In some embodiments, the term "hydrocarbyl" refers to a linear or branched group or a combination thereof which can be fully saturated, mono- or polyunsaturated, and can include a divalent or multivalent group. Examples of the saturated hydrocarbyl group include, but are not limited to, methyl, ethyl, *n*-propyl, isopropyl, *n*-butyl, *tert*-butyl, isobutyl, sec-butyl, cyclohexyl, (cyclohexyl)methyl, cyclopropylmethyl, and the

homolog or isomer of *n*-amyl, *n*-hexyl, *n*-heptyl, *n*-octyl and other atom groups. The unsaturated hydrocarbyl has one or more than one double or triple bonds. Examples of the unsaturated alkyl include but are not limited to, vinyl, 2-propenyl, butenyl, crotyl, 2-isopentenyl, 2-(butadienyl), 2,4-pentadienyl, 3-(1,4-pentadienyl), ethynyl, 1- and 3-propynyl, 3-butynyl, and more higher homologs and isomers.

Unless otherwise specified, the term "heterohydrocarbyl" or its hyponyms (such as heteroalkyl, heteroalkenyl, heteroalkynyl, and heteroaryl, etc.), by itself or as part of another substituent, refers to a stable linear, branched or cyclic hydrocarbon group or any combination thereof, which has a specified number of carbon atoms and at least one heteroatom. In some embodiments, the term "heteroalkyl" by itself or in combination with another term refers to a stable linear chain, branched hydrocarbon radical or a combination thereof which has a specified number of carbon atoms and at least one heteroatom. In a specific embodiment, a heteroatom is selected from B, O, N and S, wherein nitrogen and sulfur atoms are optionally oxidized and the nitrogen atom is optionally quaternized. The heteroatom or heteroatom group can be located at any interior position of a heterohydrocarbyl, including the position where the hydrocarbyl attaches to the rest part of the molecule. But the terms "alkoxy", "alkylamino" and "alkylthio" (or thioalkyl) are used by the conventional meaning and refer to an alkyl group connected to the rest part of the molecule via an oxygen atom, an amino or a sulfur atom respectively. Examples include, but are not limited to, $-CH_2-CH_2-O-CH_3$, $-CH_2-CH_2-NH-CH_3$, $-CH_2-CH_2-N(CH_3)-CH_3$, $-CH_2-S-CH_2-CH_3$, $-CH_2-CH_2$, $-S(O)-CH_3$, $-CH_2-CH_2-S(O)_2-CH_3$, $-CH=CH-O-CH_3$, $-CH_2-CH=N-OCH_3$ and $-CH=CH-N(CH_3)-CH_3$. Up to two consecutive heteroatoms can be present, such as, $-CH_2-NH-OCH_3$.

Unless otherwise specified, the term "cyclohydrocarbyl", "heterocyclohydrocarbyl" or its hyponyms (such as aryl, heteroaryl, cycloalkyl, heterocycloalkyl, cycloalkenyl, heterocycloalkenyl, cycloalkynyl, heterocycloalkynyl, etc.) by itself or in combination with another term refers to cyclized "hydrocarbyl" or "heterohydrocarbyl". Furthermore, for heterohydrocarbyl or heterocyclohydrocarbyl (e.g., heteroalkyl, and heterocycloalkyl), one heteroatom can occupy the position where the heterocycle attaches to the remainder position of the molecule. Examples of the cycloalkyl include, but are not limited to, cyclopentyl, cyclohexyl, 1-cyclohexenyl, 3-cyclohexenyl, cycloheptyl and the like. Non-limiting examples of heterocycloalkyl include 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydro-thiophen-2-yl, tetrahydro-thiophen-3-yl, 1-piperazinyl and 2-piperazinyl.

Unless otherwise specified, the term "alkyl" refers to a linear chain or branched saturated hydrocarbon group, can be mono-substituted (e.g., $-CH_2F$) or poly-substituted (e.g., $-CF_3$), can be monovalent (e.g. methyl), divalent (e.g., methylene) or multivalent (e.g., methenyl). Examples of alkyl include methyl (Me), ethyl (Et), propyl (such as *n*-propyl and isopropyl), butyl (such as *n*-butyl, isobutyl, sec-butyl, *tert*-butyl), pentyl (such as *n*-pentyl, isopentyl, neopentyl) and the like.

Unless otherwise specified, the term "alkenyl" refers to an alkyl group having one or more than one carbon-carbon double bonds at any position on the chain, can be mono-substituted or poly-substituted, and can be monovalent, divalent or multivalent. Examples of alkenyl include ethenyl, propenyl, butenyl, pentenyl, hexenyl, butadienyl, pentadienyl, hexadienyl, and the like.

Unless otherwise specified, the term "alkynyl" refers to an alkyl group having one or more than one carbon-carbon triple bonds at any position on the chain, can be mono-substituted or poly-substituted, and can be monovalent, divalent or multivalent. Examples of alkynyl include ethynyl, propynyl, butynyl, pentynyl, and the like.

Unless otherwise specified, cycloalkyl includes any stable cyclic or polycyclic hydrocarbyl, and any carbon atom is saturated, can be mono-substituted or poly-substituted, and can be monovalent, divalent or multivalent. Examples of cycloalkyl include, but are not limited to, cyclopropyl, norbornanyl, [2.2.2]bicyclooctane, [4.4.0]bicyclodecanyl and the like.

Unless otherwise specified, cycloalkenyl includes any stable cyclic or polycyclic hydrocarbyl having one or more than one unsaturated carbon-carbon single bonds at any position on the ring, can be mono-substituted or poly-substituted, and can be monovalent, divalent or multivalent. Examples of the cycloalkenyl include, but are not limited to, cyclopentenyl, cyclohexenyl and the like.

Unless otherwise specified, cycloalkynyl includes any stable cyclic or polycyclic hydrocarbyl having one or more carbon-carbon triple bonds at any position on the ring, can be mono-substituted or poly-substituted, and can be monovalent, divalent or multivalent.

Unless otherwise specified, the term "halo" or "halogen" by itself or as part of another substituent refers to fluorine, chlorine, bromine or iodine atom. Furthermore, the term "haloalkyl" is meant to include monohaloalkyl and polyhaloalkyl. For example, the term "halo($C_1$-$C_4$)alkyl" is meant to include, but not limited to, trifluoromethyl, 2,2,2-trifluoroethyl, 4-chlorobutyl, 3-bromopropyl and the like. Examples of haloalkyl include, but not limited to trifluoromethyl, trichloromethyl, pentafluoroethyl and pentachloroethyl.

The term "alkoxy" represents any alkyl defined above having a specified number of carbon atoms attached by an oxygen bridge. Unless otherwise specified, $C_{1-6}$ alkoxy includes $C_1$, $C_2$, $C_3$, $C_4$, $C_5$ and $C_6$ alkoxy. Examples of alkoxy include, but not limited to methoxy, ethoxy, *n*-propoxy, isopropoxy, *n*-butoxy, *sec*-butoxy, tert-butoxy, *n*-pentyloxy and S-pentoxy.

Unless otherwise specified, the term "aryl" refers to a polyunsaturated aromatic substituent, can be mono-, di- or poly-substituted, can be a monovalent, divalent or multivalent, can be a single ring or a multiple ring (e.g. one to three rings; wherein at least one ring is aromatic), which are fused together or connected covalently. The term "heteroaryl" refers to an aryl (or ring) containing one to four heteroatoms. In an illustrative example, the heteroatom is selected from B, O, N and S, wherein nitrogen and sulfur atoms are optionally oxidized and nitrogen atom is optionally quaternized. A heteroaryl may attach to the rest part of a molecule via a heteroatom. Non-limiting examples of aryl or heteroaryl include phenyl, naphthyl, biphenyl, pyrrolyl, pyrazolyl, imidazolyl, pyrazinyl, oxazolyl, phenyl-oxazolyl, isoxazolyl, thiazolyl, furanyl, thienyl, pyridyl, pyrimidinyl, benzothiazolyl, purinyl, benzimidazolyl, indolyl, isoquinolyl, quinoxalinyl, quinolyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl and 6-quinolyl. The substituent of any of the above aryl and heteroaryl ring system is selected from the acceptable substituent described below.

Unless otherwise specified, when aryl combines with other terms (such as aryloxy, arylthio, aralkyl), the aryl includes the aryl and heteroaryl ring as defined above. Thus, the term "aralkyl" is meant to include the group (e.g., benzyl, phenethyl, pyridylmethyl, etc.) where an aryl is attached to an alkyl, including an alkyl where the carbon atom (e.g., methylene) has been replaced by an atom such as oxygen, for example, phenoxymethyl, 2-pyridyloxy, 3-(1-naphthyloxy)propyl, and the like.

The term "leaving group" refers to a functional group or atom which can be replaced by another functional group or atom through a substitution reaction (such as affinity substitution reaction). For example, representative leaving groups include triflate; chlorine, bromine and iodine; sulfonate group, such as mesylate, tosylate, p-bromobenzenesulfonate, p-toluenesulfonates and the like; acyloxy, such as acetoxy, trifluoroacetoxy and the like.

The term "protecting group" includes, but is not limited to "amino protecting group", "hydroxy protecting group" or "mercapto protecting group". The term "amino protecting group" refers to a protecting group suitable for blocking the side reaction on the nitrogen of an amino. Representative amino protecting groups include, but are not limited to: formyl; acyl, such as alkanoyl (e.g., acetyl, trichloroacetyl or trifluoroacetyl); alkoxycarbonyl, such as *tert*-butoxycarbonyl (Boc); arylmethoxycarbonyl such as benzyloxycarbonyl (Cbz) and 9-fluorenylmethoxycarbonyl (Fmoc); arylmethyl such as benzyl (Bn), trityl (Tr), 1,1-bis-(4'-methoxyphenyl)methyl; silyl such as trimethylsilyl (TMS) and *tert*-butyl dimethyl silyl (TBS) and the like. The term "hydroxy protecting group" refers to a protecting group suitable for blocking the side reaction on hydroxy. Representative hydroxy protecting groups include, but are not limited to: alkyl such as methyl, ethyl and *tert*-butyl; acyl such as alkanoyl (e.g., acetyl); arylmethyl such as benzyl (Bn), *p*-methoxybenzyl (PMB), 9-fluorenylmethyl (Fm), and diphenylmethyl (benzhydryl, DPM); silyl such as trimethylsilyl (TMS) and *tert*-butyl dimethyl silyl (TBS) and the like.

The compound of the present disclosure can be prepared by a variety of synthetic methods well known to the skilled in the art, including the following enumerative embodiment, the embodiment formed by the following enumerative embodiment in combination with other chemical synthesis methods and the equivalent replacement well known to the skilled in the art. The preferred embodiment includes, but is not limited to the embodiment of the present disclosure.

The solvent used in the present disclosure is commercially available.

Compounds are named manually or by ChemDraw® software, the commercially available compounds use their vendor directory names.

## Brief description of the drawings

Figure 1 is the changes in HBsAg content of the plasma of the mice after administration at different days.

Figure 2 is the changes in HBsAg content of the mice after administration at different dates.

Figure 3 is the changes in body weight of the mice after administration every day.

## Detailed description of the embodiment

The following examples further illustrate the present disclosure. Embodiments 1, 3-13, 15-17 and 19-29 are according to the present invention. Embodiments 2, 14 and 18 are for reference.

**Embodiment 1**

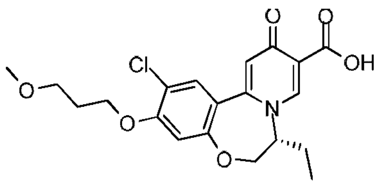

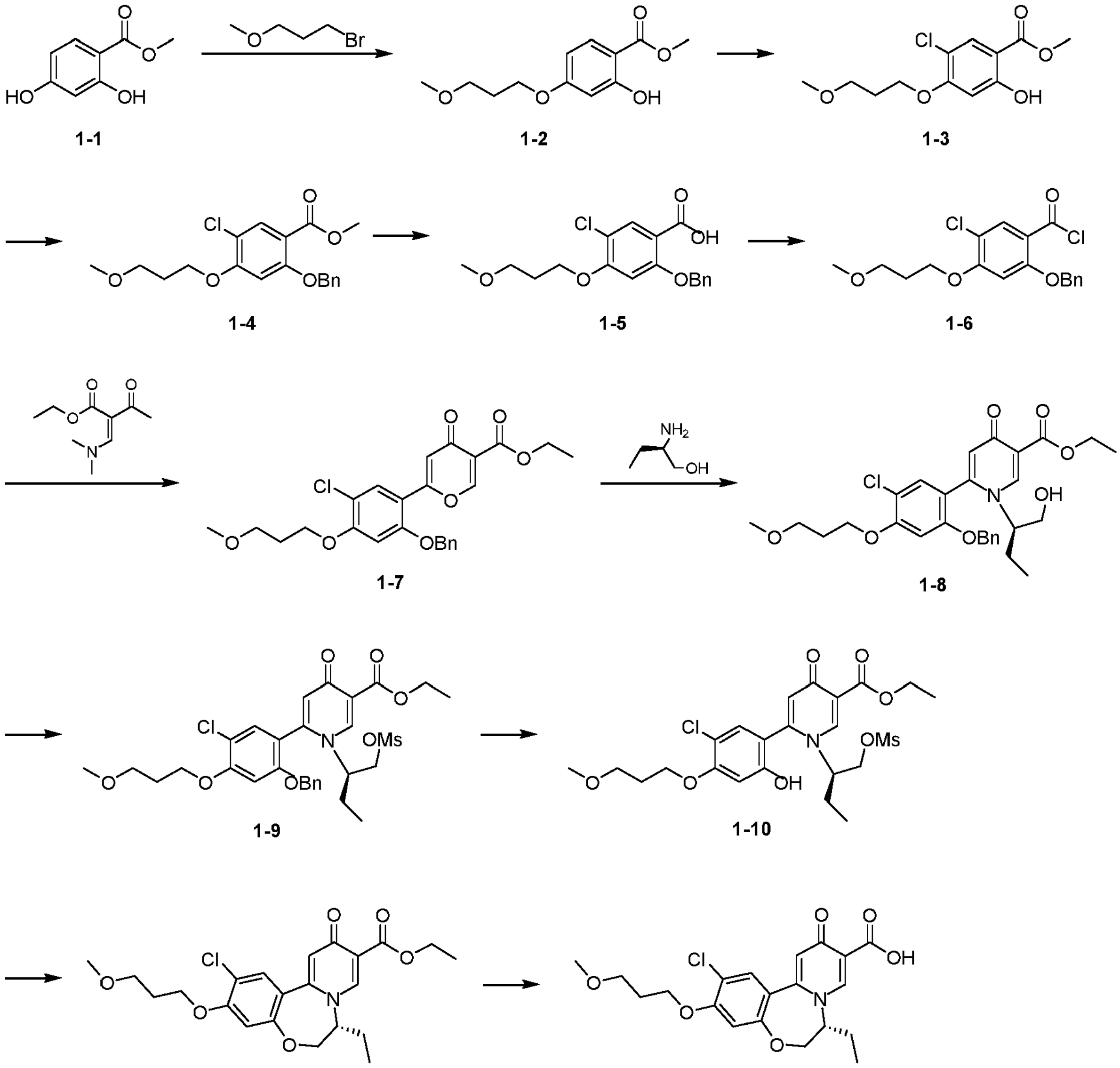

1-1 1-2 1-3 1-4 1-5 1-6 1-7 1-8 1-9 1-10 1-11 Embodiment 1

**Step A:** 1-1 (200.00 g, 1.19 mol) was dissolved in *N*,*N*-dimethylformamide (1000.00 mL), followed by addition of potassium carbonate (164.39 g, 1.19 mol). 1-Bromo-3-methoxy-propane (182.01 g, 1.19 mol) was added dropwise at 90°C within one hour. The mixture was stirred at 90°C for 15 hours. 3 L of ethyl acetate (1 L × 3) was added to the reaction mixture. The organic phases were combined, washed with 15.00 L of water (3.00 L × 5) and 3.00 L of saturated brine (1.00 L × 3), dried over anhydrous sodium sulfate, and concentrated under reduced pressure to give a crude product. The crude product was purified by silica gel column chromatography (eluent: petroleum ether/ethyl acetate = 1/0) to give Compound 1-2.

**Step B:** 1-2 (80.00 g, 332.98 mmol) was dissolved in acetonitrile (800.00 mL), followed by addition of chlorosuccinimide (44.46 g, 332.98 mmol), and the mixture was stirred at 90°C for three hours. Half volume of acetonitrile was first rotary evaporated, followed by addition of 200.00 mL of water and 300.00 mL of ethyl acetate. The isolated organic phase was washed with ammonium chloride solution (100.00 mL), dried over anhydrous sodium, and concentrated under reduced pressure to give a yellow oily compound. The compound was triturated with methanol to give 1-3.

**Step C:** Potassium carbonate (76.48 g, 553.34 mmol) was added to a solution of 1-3 (76.00 g, 276.67 mmol), benzyl bromide (52.05 g, 304.34 mmol, 36.15 mL) in *N,N*-dimethylformamide (800.00 mL). The mixed solution was stirred at 25°C for 16 hours. Ethyl acetate (900.00 mL) and water (1000.00 mL) were added to the solution. The organic phase was isolated and washed with water (1000.00 mL $\times$ 2) and saturated brine (300.00 mL $\times$ 2), dried over anhydrous sodium sulfate and concentrated under reduced pressure to give Compound 1-4.

**Step D:** 1-4 (92.00 g, 252.18 mmol) was dissolved in methanol (500.00 mL) followed by addition of potassium hydroxide solution (6 M, 239.99 mL). The mixture was stirred at 45°C for 2 hours. The pH of the reaction mixture was adjusted to 3 with 6 M hydrochloric acid, and a white solid was precipitated, followed by filtration to obtain the solid. The solid was dissolved in dichloromethane (80 mmol), dried over anhydrous sodium sulfate, filtered and concentrated under reduced pressure to give Compound 1-5.

**Step E:** 1-5 (76.58 g, 218.31 mmol) was dissolved in dichloromethane (500.00 mL), followed by addition of oxalyl chloride (41.56 g, 327.46 mmol, 28.66 mL) and *N,N*-dimethylformamide (159.56 mg, 2.18 mmol, 167.96 $\mu$L). The mixture was stirred at 25°C for 3 hours. After completion of the reaction, the mixture was concentrated under reduced pressure to give Compound 1-6.

**Step F:** 1-6 (63.00 g, 170.62 mmol) and methyl ethyl-2-(dimethylaminomethylidene)-3-oxo-butanoate (44.24 g, 238.87 mmol) were dissolved in tetrahydrofuran (600.00 mL), which was then added dropwise to a solution of lithium hexamethyldisilazide in tetrahydrofuran (1.00 mol, 409.49 mL) at -70°C to -60°C. After completion of the dropwise addition, the dry ice acetone bath was removed, and a hydrochloric acid solution (3.00 mol, 832.06 mL) was added in one portion. The reaction mixture was stirred at 20°C for one hour. The mixture was filtered to give a solid. The solid was dissolved in dichloromethane, dried over anhydrous sodium sulfate, and concentrated under reduced pressure to give a solid. Methyl *tert*-butyl ether (60.00 mL) was added to the solid, stirred for 30 minutes and filtered to give Compound 1-7.

$^1$H NMR (400 MHz, $CDCl_3$) $\delta$8.55 (s, 1H), 7.73-7.79 (m, 1H), 7.34-7.43 (m, 4H), 7.20 (s, 1H), 6.63 (s, 1H), 5.24 (s, 2H), 4.39 (q, *J*=7.15 Hz, 2H), 4.11 (t, *J*=6.21 Hz, 2H), 3.59 (t, *J*=5.90 Hz, 2H), 3.38 (s, 3H), 2.05-2.12 (m, 2H), 1.40 (t, *J*=7.15 Hz, 3H).

**Step G:** 1-7 (2.00 g, 4.23 mmol) was dissolved in ethanol (30.00 mL), followed by addition of acetic acid (10.00 mL) and (*R*)-(-)-2-amino-1-butanol (565.59 mg, 6.35 mmol, 595.36 $\mu$L). The reaction mixture was stirred at 80°C for 16 hours. The reaction mixture was concentrated under reduced pressure and extracted with water (50.00 mL) and 60.00 mL of ethyl acetate (20.00 mL$\times$3). The combined organic phase was washed with 20.00 mL (10.00 mL$\times$2) of saturated sodium bicarbonate, dried over anhydrous sodium sulfate and concentrated under reduced pressure to give a crude product. The crude product was purified by column chromatography (silica, eluent: petroleum ether/ethyl acetate = 10/1 to 1/1) to give Compound 1-8.

**Step H:** 1-8 (1.80 g, 3.31 mmol) was dissolved in dichloromethane (20.00 mL), followed by addition of triethylamine (502.41 mg, 4.97 mmol, 688.23 $\mu$L) and addition of methanesulfonyl chloride (454.99 mg, 3.97 mmol, 307.43 $\mu$L) at 0°C. The reaction mixture was stirred at 25°C for 2 hours. The reaction mixture was quenched by the addition of 30.00 mL of water at 25°C, and then extracted with 100.00 mL of dichloromethane (50 mL $\times$ 2). The combined organic phase was washed with 60.00 mL (30.00 mL$\times$2) of saturated brine, dried over anhydrous sulfate, filtered, concentrated under reduced pressure to give Compound 1-9.

**Step I:** 1-9 (2.00 g, 3.21 mmol) was dissolved in tetrahydrofuran (100.00 mL), followed by addition of palladium carbon (300.00 mg, purity 10%) under nitrogen atmosphere. The suspension was purged with hydrogen three times. The reaction mixture was stirred at 25°C for 2 hours under hydrogen (15 Psi) atmosphere. The reaction mixture was filtered and concentrated under reduced pressure to give Compound 1-10.

**Step J:** 1-10 (1.70 g, 3.20 mmol) was dissolved in *N*,*N*-dimethylformamide (20.00 mL), followed by addition of potassium carbonate (884.54 mg, 6.40 mmol) and potassium iodide (5.31 mg, 32.00 $\mu$mol). The reaction mixture was stirred at 100°C for 16 hours. The reaction mixture was extracted with 100.00 mL (50.00 mL $\times$ 2) of ethyl acetate. The combined organic phase was washed with 100.00 mL of saturated brine (50.00 mL $\times$ 2), dried over anhydrous sodium sulfate, filtered and concentrated under reduced pressure to give Compound 1-11.

**Step K:** 1-11 (1.00 g, 2.29 mmol) was dissolved in methanol (47.40 mL), followed by addition of sodium hydroxide solution (4 M, 10.32 mL). The reaction mixture was stirred at 25°C for 20 minutes. The reaction mixture was quenched with hydrochloric acid (1 mol), and the pH was adjusted to 3 and a solid precipitated. The mixture was extracted with 40.00 mL of ethyl acetate (20.00 mL $\times$ 2). The combined organic phase was washed with 30.00 mL of saturated sodium bicarbonate solution and 30.00 mL of saturated brine (15.00 mL $\times$ 2), dried over anhydrous sodium sulfate, filtered and concentrated under reduced pressure to give a crude product. The crude product was subjected to preparative thin layer chromatography (silica, petroleum ether: ethyl acetate = 0:1) to give the compound of Embodiment 1.

ee value (enantiomeric excess): 100%.

SFC (Supercritical Fluid Chromatography) method: Column: Chiralcel OD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400 MHz, $CDCl_3$) $\delta$ 15.67 (s, 1H), 8.50 (br s, 1H), 7.43 (s, 1H), 6.71 (s, 1H), 6.67 (s, 1H), 4.19-4.40 (m, 2H), 4.10 (br t, *J*=6.11 Hz, 2H), 3.46-3.63 (m, 2H), 3.42 (s, 1H), 3.30 (s, 3H), 2.07 (quin, *J*=6.02 Hz, 2H), 1.77-1.98 (m, 2H), 0.96 (br s, 3H).

**Embodiments 2 to 5 can be prepared by the method referring to the preparation method of Embodiment 1:**

**Embodiment 2 (reference)**

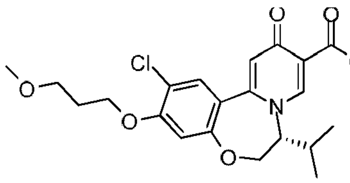

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400 MHz, $CDCl_3$) $\delta$ 15.77 (br s, 1H), 8.49 (s, 1H), 7.52 (s, 1H), 6.87 (s, 1H), 6.68 (s, 1H), 4.49-4.72 (m, 2H), 4.12-4.28 (m, 2H), 3.92 (br d, *J*=6.40 Hz, 1H), 3.63 (t, *J*=5.90 Hz, 2H), 3.39 (s, 3H), 2.06-2.24 (m, 3H), 1.10 (d, *J*=6.53 Hz, 3H), 0.89 (d, *J*=6.53 Hz, 3H).

**Embodiment 3**

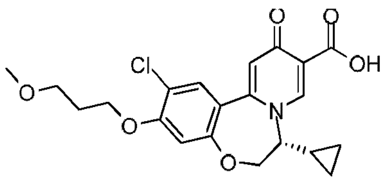

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400 MHz, $CDCl_3$) $\delta$ 15.81 (s, 1H), 8.88-9.18 (m, 1H), 7.47 (s, 1H), 6.78 (s, 1H), 6.76 (s, 1H), 4.48-4.59 (m, 2H), 4.19 (dt, *J*=2.07, 6.12 Hz, 2H), 3.59-3.67 (m, 2H), 3.45-3.52 (m, 1H), 3.39 (s, 3H), 2.15 (quin, *J*=6.05 Hz, 2H), 1.14-1.31 (m, 2H), 0.29-0.55 (m, 2H), 0.09 (s, 1H).

Embodiment 4

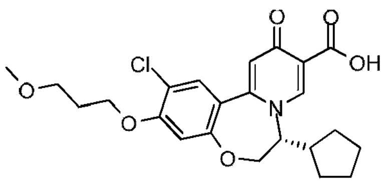

ee value (enantiomeric excess): 97%.

SFC (Supercritical Fluid Chromatography) method: Column: Chiral pak AD-3 100 mm × 4.6 mm I.D., 3 μm.

Mobile phase: 5%-40% methanol (0.05% diethylamine) in carbon dioxide. Flow rate: 3 mL/min. Wavelength: 220 nm.

$^1H$ NMR (400 MHz, $CDCl_3$) $\delta$ 15.74 (s,1H), 8.61 (br s, 1H), 7.52 (s, 1H), 6.79 (s, 1H), 6.74 (s, 1H), 4.46 (br s, 2H), 4.20 (t, *J*=6.15 Hz, 2H), 4.04 (br s, 1H), 3.63 (dt, *J*=2.20, 5.87 Hz, 2H), 3.39 (s, 3H), 2.38 (br s, 1H), 2.16 (quin, *J*=5.96 Hz, 2H), 1.60-2.00 (m, 6H), 1.09-1.25 (m, 2H).

## Embodiment 5

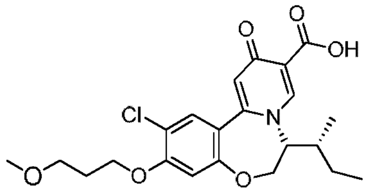

ee value (enantiomeric excess): 89%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1H$ NMR (400 MHz, $CDCl_3$) $\delta$ = 15.73 (br s, 1H), 8.42 (s, 1H), 7.43 (s, 1H), 6.78 (s, 1H), 6.57 (s, 1H), 4.64 - 4.45 (m, 2H), 4.13 - 4.05 (m, 2H), 3.97 (ddd, *J*=2.4, 5.6, 10.9 Hz, 1H), 3.53 (t, *J*=6.0 Hz, 2H), 3.30 (s, 3H), 2.06 (quin, *J*=6.1 Hz, 2H), 1.64 - 1.52 (m, 1H), 1.42 - 1.01 (m, 2H), 0.85 (t, *J*=7.4 Hz, 3H), 0.74 (d, *J*=6.6 Hz, 3H).

## Embodiment 6

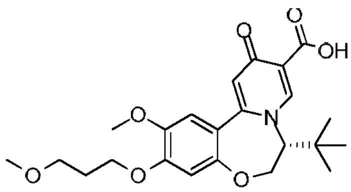

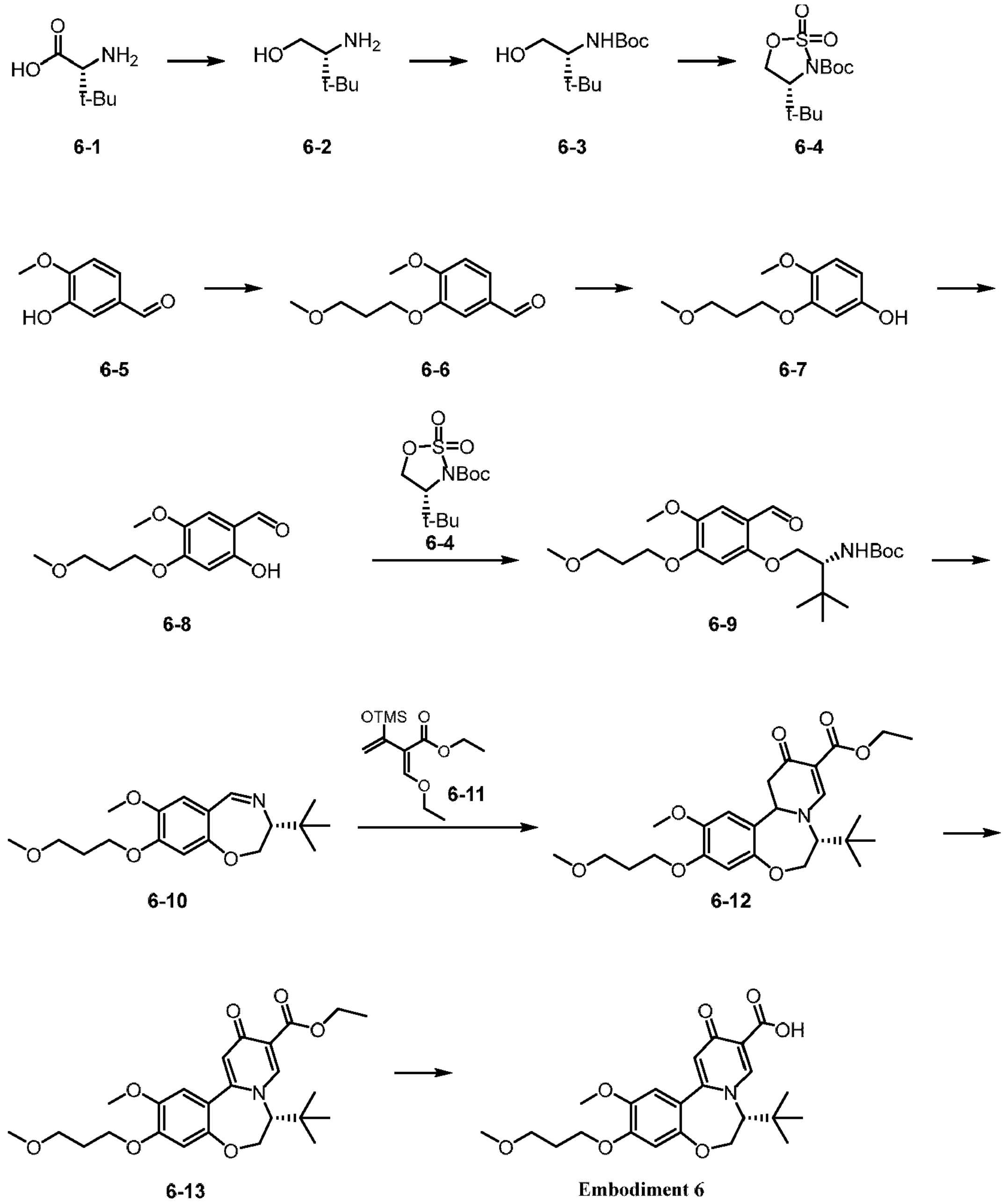

6-1 6-2 6-3 6-4

6-5 6-6 6-7

6-4

6-8 6-9

6-11

6-10 6-12

6-13 Embodiment 6

**Step A:** Lithium aluminum tetrahydride (80.00 g, 2.11 mol, 2.77 eq) was added to a solution of 6-1 (100.00 g, 762.36 mmol, 1.00 eq) in tetrahydrofuran (400.00 mL) while maintaining the temperature below 0°C. The solution was stirred at 10°C for 10 hours. Afterwards, 80.00 mL of water was added to the reaction mixture under stirring, and 240.00 mL of 15% aqueous sodium hydroxide solution was added, followed by addition of 80.00 mL of water. The suspension was stirred at 10°C for 20 minutes, followed by filtration to give a colorless liquid. The colorless liquid was concentrated under reduced pressure to give compound 6-2.

$^1$H NMR (400 MHz, $CDCl_3$) $\delta$ = 3.72 (dd, *J*=3.9, 10.2 Hz, 1H), 3.21 (t, *J*=10.2 Hz, 1H), 2.51 (dd, *J*=3.9, 10.2 Hz, 1H), 0.91 (s, 9H).

**Step B:** 6-2 (50.00 g, 426.66 mmol) and triethylamine (59.39 mL, 426.66 mmol) were dissolved in dichloromethane (500.00 mL). Di-*tert*-butyl dicarbonate (92.19 g, 422.40 mmol) was dissolved in dichloromethane (100.00 mL), and was added dropwise to the above reaction mixture at 0°C. The reaction mixture was then stirred at 25°C for 12 hours. The reaction mixture was washed with saturated brine (600.00 mL), dried over anhydrous sodium sulfate. The organic phase was concentrated under reduced pressure, and rotary evaporated to dryness, followed by recrystallization with methyl *tert-butyl* ether/petroleum ether (50.00/100.00) to give compound 6-3.

$^1$H NMR (400 MHz, $CDCl_3$) $\delta$ 4.64 (br s, 1H), 3.80-3.92 (m, 1H), 3.51 (br d, *J*=7.09 Hz, 2H), 2.17 (br s, 1H), 1.48 (s, 9H), 0.96 (s, 9H).

**Step C:** Thionyl chloride (100.98 mL, 1.39 mmol) was dissolved in acetonitrile (707.50 mL), 6-3 (121.00 g, 556.82 mmol) was dissolved in acetonitrile (282.90 mL), and added dropwise to the above reaction mixture at -40°C. After completion of the addition, pyridine (224.72 mL, 2.78 mol) was added to the reaction mixture in one portion. The ice bath was removed and the reaction mixture was stirred at 5-10°C for 1 hour. After the solvent was rotary evaporated under reduced pressure to dryness, ethyl acetate (800.00 mL) was added and a solid precipitated. The mixture was filtered and the filtrate was concentrated under reduced pressure. Secondary, the obtained oil, water and ruthenium trichloride (12.55 g, 55.68 mmol) were dissolved in acetonitrile (153.80 mL). Sodium periodate (142.92 g, 668.19 mmol) was suspended in water (153.80 mL), and was slowly added to the above reaction mixture. The final reaction mixture was stirred at 5-10°C for 0.15 hour. The reaction mixture was filtered, and the filtrate was extracted with ethyl acetate (800.00 mL×2). The organic phase was washed with saturated brine (800.00 mL), dried over anhydrous sodium sulfate, filtered, concentrated under reduced pressure, rotary evaporated under reduced pressure to dryness, and purified by column chromatograph (silica, petroleum ether/ethyl acetate = 50/1 to 20/1) to give compound 6-4.

$^1$H NMR (400 MHz, $CDCl_3$) $\delta$ 4.49-4.55 (m, 1H), 4.40-4.44 (m, 1H), 4.10 (d, *J*=6.15 Hz, 1H), 1.49 (s, 9H), 0.94 (s, 9H).

**Step D:** 6-5 (100.00 g, 657.26 mmol) was dissolved in acetonitrile (1300.00 mL), followed by addition of potassium carbonate (227.10 g, 1.64 mol) and 1-bromo-3-methoxypropane (110.63 g, 722.99 mmol). The reaction mixture was stirred at 85°C for 6 hours. The reaction solution was extracted with 600.00 mL of ethyl acetate (200.00 mL×3), dried over anhydrous sodium sulfate, filtered and concentrated under reduced pressure to give compound 6-6.

$^1$H NMR (400 MHz, $CDCl_3$) $\delta$ 9.76-9.94 (m, 1H), 7.42-7.48 (m, 2H), 6.98 (d, *J*=8.03 Hz, 1H), 4.18 (t, *J*=6.53 Hz, 2H), 3.95 (s, 3H), 3.57 (t, *J*=6.09 Hz, 2H), 3.33-3.39 (m, 3H), 2.13 (quin, *J*=6.34 Hz, 2H).

**Step E:** 6-6 (70.00 g, 312.15 mmol) was dissolved in dichloromethane, followed by addition of *m*-chloroperoxybenzoic acid (94.27 g, 437.01 mmol). The reaction mixture was stirred at 50°C for 2 hours. The reaction mixture was cooled down, followed by filtration, and the filtrate was extracted with dichloromethane. The organic phase was washed with 2000.00 mL of saturated sodium bicarbonate solution (400.00 mL × 5), dried over anhydrous sodium sulfate and concentrated under reduced pressure to give a brown oil. The brown oil was dissolved with as little methanol as possible, followed by slow addition of 2 M potassium hydroxide (350.00 mL) solution (the course was exothermal). The reaction mixture in dark color was stirred at room temperature for 20 minutes, and the pH of the reaction mixture was adjusted to 5 with 37% hydrochloric acid, followed by extraction with 400.00 mL of ethyl acetate (200.00 mL × 2). The organic phase was washed with 200.00 mL of saturated brine (100.00 mL × 2), dried over anhydrous sodium sulfate and concentrated under reduced pressure to give Compound 6-7.

$^1$H NMR (400 MHz, $CDCl_3$) $\delta$ 6.75 (d, *J*=8.53 Hz, 1H), 6.49 (d, *J*=2.89 Hz, 1H), 6.36 (dd, *J*=2.82, 8.60 Hz, 1H), 4.07 (t, *J*=6.40 Hz, 2H), 3.82 (s, 3H), 3.60 (t, *J*=6.15 Hz, 2H), 3.38 (s, 3H), 2.06-2.14 (m, 2H).

**Step F:** 6-7 (33.00 g, 155.48 mmol) was dissolved in tetrahydrofuran (330.00 mL), followed by addition of paraformaldehyde (42.02 g, 466.45 mmol), magnesium chloride (29.61 g, 310.97 mmol) and triethylamine (47.20 g, 466.45 mmol, 64.92 mL). The reaction mixture was stirred at 80°C for 8 hours. After completion of the reaction, the reaction mixture was quenched with 2 mol of hydrochloric acid solution (200.00 mL) at 25°C, and then extracted with 600.00 mL of ethyl acetate (200.00 mL × 3). The organic phase was washed with 400.00 mL of saturated brine (200.00 mL × 2), dried over anhydrous sodium sulfate, filtered and concentrated under reduced pressure to give a residue. The residue was washed with ethanol (30.00 mL) and filtered to give a filter cake, thereby giving Compound 6-8.

$^1$H NMR (400 MHz, $CDCl_3$) $\delta$ 11.29 (s, 1H), 9.55-9.67 (m, 1H), 6.83 (s, 1H), 6.42 (s, 1H), 4.10 (t, *J*=6.48 Hz, 2H), 3.79 (s, 3H), 3.49 (t, *J*=6.05 Hz, 2H), 3.28 (s, 3H), 2.06 (quin, J=6.27 Hz, 2H).

**Step G:** 6-8 (8.70 g, 36.21 mmol) was dissolved in *N*,*N*-dimethylformamide (80.00 mL), followed by addition of potassium carbonate (10.01 g, 72.42 mmol) and 6-4 (11.13 g, 39.83 mol). The reaction mixture was stirred at 50°C for 2 hours. The reaction mixture was quenched with 1.00 mol/L aqueous hydrochloric acid solution (200.00 mL) and extracted with ethyl acetate (150.00 mL × 2). The organic phases were combined, washed with water (150.00 mL × 3), dried over anhydrous sodium sulfate, filtered and concentrated under reduced pressure to give Compound 6-9.

$^1$H NMR (400 MHz, $CDCl_3$) $\delta$ 10.31 (s, 1H), 7.34 (s, 1H), 6.57 (s, 1H), 4.18-4.26 (m, 3H), 4.07 (dd, *J*=5.33, 9.60 Hz, 1H), 3.88 (s, 4H), 3.60 (t, *J*=5.96 Hz, 2H), 3.39 (s, 3H), 2.17 (quin, *J*=6.21 Hz, 2H), 1.47 (s, 9H), 1.06 (s, 9H).

**Step H:** 6-9 (15.80 g, 35.95 mmol) was dissolved in dichloromethane (150.00 mL), followed by addition of trifluoroacetic acid (43.91 mL, 593.12 mmol). The reaction mixture was stirred at 10°C for 3 hours. The reaction mixture was concentrated under reduced pressure and rotary evaporated to dryness, followed by addition of aqueous sodium bicarbonate solution (100.00 mL) and extraction with dichloromethane (100.00 mL). The organic phase was dried over anhydrous sodium sulfate, filtered and concentrated under reduced pressure to give Compound 6-10.

$^1$H NMR (400 MHz, $CDCl_3$) $\delta$ 8.40 (s, 1H), 6.80 (s, 1H), 6.51 (s, 1H), 4.30 (br d, *J*=12.35 Hz, 1H), 4.04-4.11 (m, 3H), 3.79 (s, 3H), 3.49 (t, *J*=5.99 Hz, 2H), 3.36 (br d, *J*=2.93 Hz, 1H), 3.28 (s, 3H), 2.06 (quin, *J*=6.24 Hz, 2H), 1.02 (s, 9H).

**Step I:** 6-10 (5.00 g, 15.56 mmol) was dissolved in toluene (20.00 mL), followed by addition of 6-11 (8.04 g, 31.11 mmol). The reaction mixture was stirred at 120°C for 12 hours under nitrogen atmosphere. The reaction mixture

was quenched with water (100.00 mL) and extracted with ethyl acetate (100.00 mL × 2). The organic phases were combined, washed with water (80.00 mL × 2), dried over anhydrous sodium sulfate, filtered and concentrated under reduced pressure. The residue was purified by reverse phase column, and then purified by high performance preparative liquid chromatography (column: Phenomenex luna C18 250*50 mm*10 μm; mobile phase: [water (0.225% formic acid)-acetonitrile]; elution gradient: 35%-70%, 25 min) to give Compound 6-12.

$^1$H NMR (400 MHz, $CDCl_3$) $\delta$ 7.95 (s, 1H), 6.59 (s, 1H), 6.40 (s, 1H), 5.15-5.23 (m, 1H), 4.35-4.41 (m, 2H), 4.08-4.19 (m, 2H), 3.94-4.00 (m, 2H), 3.72 (s, 3H), 3.61-3.67 (m, 1H), 3.46 (dt, *J*=1.96, 5.99 Hz, 2H), 3.27 (s, 3H), 3.01-3.08 (m, 1H), 2.85-2.94 (m, 1H), 1.97-2.01 (m, 2H), 1.18-1.22 (m, 3H), 1.04 (s, 9H).

**Step J:** 6-12 (875.00 mg, 1.90 mmol) was dissolved in toluene (20.00 mL) and ethylene glycol dimethyl ether (20.00 mL), followed by addition of tetra-chloro-benzoquinone (1.40 g, 5.69 mmol). The reaction mixture was stirred at 120°C for 12 hours. The reaction mixture was cooled to room temperature, followed by addition of saturated aqueous sodium carbonate solution (50.00 mL) and ethyl acetate (60.00 mL). The mixture was stirred at 10-15°C for 20 minutes and separated to give an organic phase. The organic phase was added to 2.00 mol/L aqueous hydrochloric acid solution (60.00 mL), and stirred at 10-15°C for 20 minutes, followed by partition. The organic phase was washed with 2 mol/L aqueous hydrochloric acid solution (60.00 mL × 2), followed by partition. 2 mol/L aqueous sodium hydroxide solution (200.00 mL) and dichloromethane (200.00 mL) were added to the aqueous phase, followed by partition. The organic phase was dried over anhydrous sodium sulfate, filtered and concentrated under reduced pressure to give Compound 6-13.

$^1$H NMR (400 MHz, $CDCl_3$) $\delta$ 7.98-8.78 (m, 1H), 6.86 (s, 1H), 6.43-6.73 (m, 2H), 4.41-4.48 (m, 1H), 4.28-4.38 (m, 2H), 4.03-4.11 (m, 2H), 3.93 (br s, 1H), 3.80 (s, 3H), 3.47-3.52 (m, 3H), 3.29 (s, 3H), 2.06 (quin, *J*=6.24 Hz, 2H), 1.33 (t, *J*=7.15 Hz, 2H), 0.70-1.25 (m, 10H).

**Step K:** 6-13 (600.00 mg, 1.31 mmol) was dissolved in methanol (6.00 mL), followed by addition of 4.00 mol/L of aqueous sodium hydroxide solution (2.00 mL, 6.39 eq). The reaction mixture was stirred at 15°C for 0.25 hours. The pH of the reaction mixture was adjusted to 3-4 with 1.00 mol/L aqueous hydrochloric acid solution, followed by extraction with dichloromethane (50.00 mL × 3). The organic phases were combined, washed with saturated brine (50.00 mL), dried over anhydrous sodium sulfate, filtered and concentrated under reduced pressure to give Embodiment 6.

ee value (enantiomeric excess): 100%.

SFC (Supercritical Fluid Chromatography) method: Column: Chiralcel OD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400 MHz, $CDCl_3$) $\delta$ 15.72 (br s, 1H), 8.32-8.93 (m, 1H), 6.60-6.93 (m, 2H), 6.51 (br s, 1H), 4.38-4.63 (m, 2H), 4.11 (br dd, *J*=4.52, 12.23 Hz, 3H), 3.79-3.87 (m, 3H), 3.46-3.54 (m, 2H), 3.29 (s, 3H), 2.07 (quin, *J*=6.24 Hz, 2H), 0.77-1.21 (m, 9H).

**Embodiment 7**

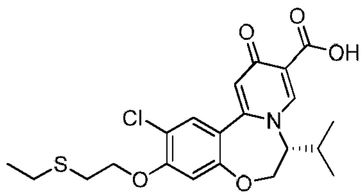

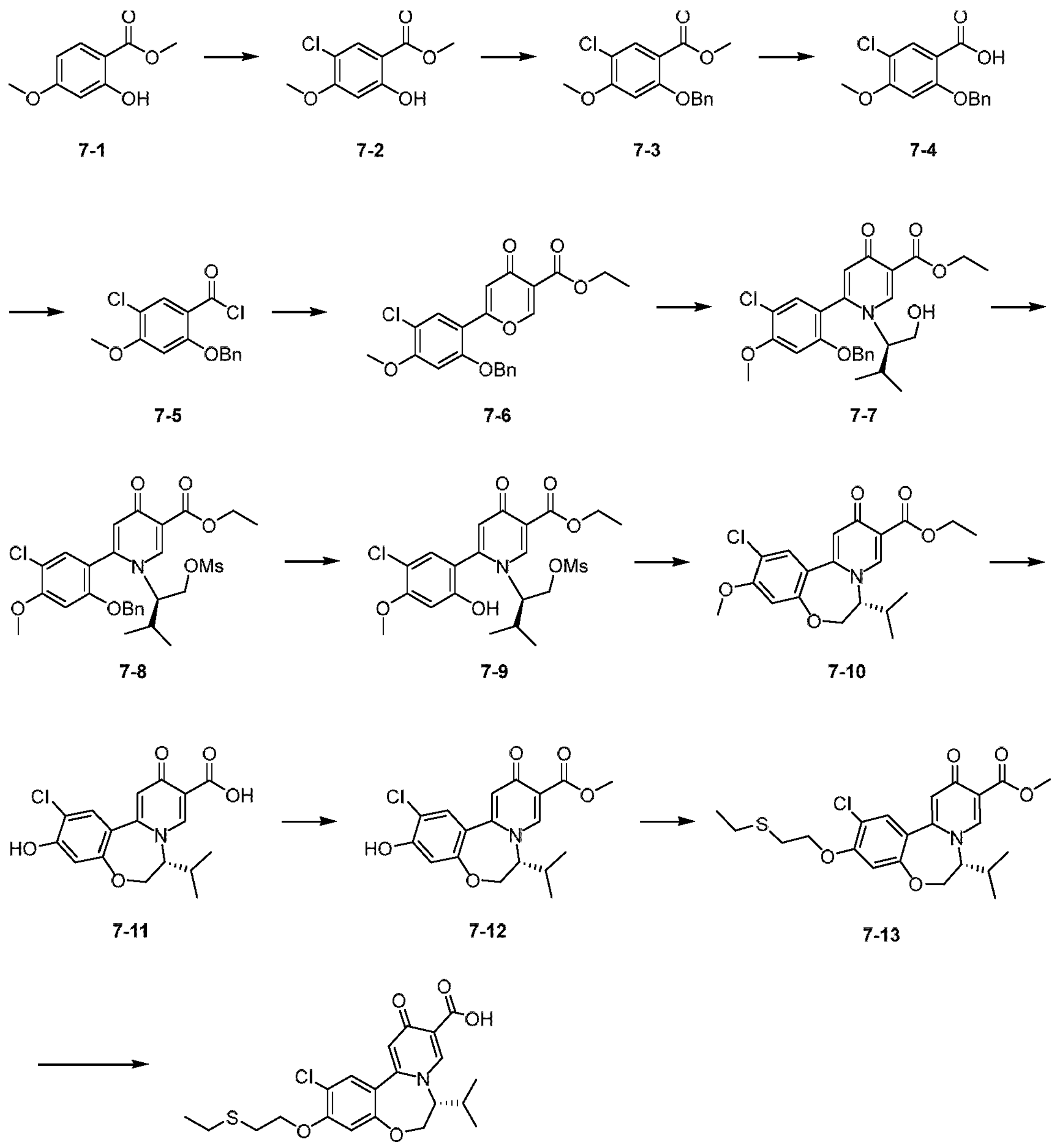

7-1 7-2 7-3 7-4

7-5 7-6 7-7

7-8 7-9 7-10

7-11 7-12 7-13

Embodiment 7

**Step A:** 7-1 (50.00 g, 274.47 mmol, 1.00 eq) was dissolved in acetonitrile (500.00 mL) and cooled to 0°C, followed by addition of chlorosuccinimide (37.75 g, 282.70 mmol, 1.03 eq). The mixture was heated to 25°C and stirred for 10 hours. Afterwards, the reaction mixture was concentrated under reduced pressure to give a colorless liquid, and ethyl acetate (500 mL) was added to the liquid. The solution was washed with water (100.00 mL*3) and saturated brine (100.00 mL*3). The organic phase was dried over anhydrous sodium sulfate and concentrated under reduced pressure to give a colorless liquid. The colorless liquid was purified by silica gel column to give 7-2.

**Step B:** Potassium carbonate (70.18 g, 507.80 mmol, 2.20 eq) was added to a solution of 7-2 (50.00 g, 230.82 mmol, 1.00 eq) and benzyl bromide (43.42 g, 253.90 mmol, 30.16 mL, 1.10 eq) in *N,N*-dimethylformamide (500.00 mL) in one portion. The mixture was then stirred at 25°C for 10 hours. Ethyl acetate (700.00 mL * 2) and water (200.00 mL) were added to the solution. The solution was stirred at 20°C for 10 minutes. The organic phase was separated, washed

with water (200.00 mL * 2) and saturated brine (200.00 mL * 2), dried over anhydrous sodium sulfate and concentrate under reduced pressure to give 7-3.

**Step C:** Potassium hydroxide (6.00 mol/L, 41.57 mL, 3.06 eq) was added to a mixed solution of 7-3 (25.00 g, 81.50 mmol, 1.00 eq) in water (20.00 mL) and methanol (60.00 mL) in one portion. The solution was stirred at 50°C for two hours. 50.00 mL of water was added to the solution. The solution was concentrated under reduced pressure to 70.00 mL and washed with ethyl acetate/petroleum ether (4/1, 20.00 mL * 2). The aqueous phase was separated, and the pH was adjusted to 1-2 with 1.00 mol/L dilute hydrochloric acid to give a suspension. The suspension was filtered to give a white solid, which was then triturated with water (30.00 mL) to give 7-4.

**Step D:** Oxalyl chloride (17.35 g, 136.65 mmol, 11.96 mL, 2.00 eq) was added dropwise to a solution of 7-4 (20.00 g, 68.33 mmol, 1.00 eq) in dichloromethane (200.00 mL). The solution was stirred at 28°C for two hours, and then concentrated to give 7-5.

**Step E:** A mixed solution of 7-5 (19.00 g, 61.06 mmol, 1.00 eq) and methyl ethyl-2-(dimethylaminomethylidene)-3-oxo-butanoate (11.88 g, 64.11 mmol, 1.05 eq) in tetrahydrofuran (50.00 mL) was dropwise added to a solution of lithium hexamethyldisilazide (1.00 mol/L, 152.65 mL, 2.50 eq) in tetrahydrofuran (10.00 mL) at -70°C in 5 minutes. The dry ice/acetone bath was removed and the solution was stirred for 5 minutes. Dilute hydrochloric acid (1.00 mol/L, 125.37 mL, 57.44 eq) was added to the mixture. The mixture was stirred vigorously, and then rotary evaporated at 60°C to remove most of tetrahydrofuran. The residue was kept at 60 to 65°C for 1.5 hours. 200.00 mL of water was added to the mixture to give a suspension, which was stirred for 30 minutes and filtered to give a yellow solid. The yellow solid was further triturated with water (40.00 mL) and methyl *tert-butyl* ether (40.00 mL) to give Compound 7-6.

**Step F:** A mixed solution of 7-6 (10.00 g, 24.11 mmol, 1.00 eq) and valinol (3.73 g, 36.17 mmol, 4.01 mL, 1.50 eq) in acetic acid (30.00 mL) and ethanol (90.00 mL) was stirred at 90°C for 10 hours. The mixture was cooled to 20°C and concentrated under reduced pressure at 40°C to give a yellow liquid. The yellow liquid was purified by silica gel column chromatography (silica, petroleum ether/ethyl acetate = 10/1) to give Compound 7-7.

**Step G:** Triethylamine (4.86 g, 48.06 mmol, 6.66 mL, 3.00 eq) was added to a solution of 7-7 (8.00 g, 15.36 mmol, 63.71%) in dichloromethane (10.00 mL) in one portion, and methanesulfonyl chloride (3.67 g, 32.04 mmol, 2.84 mL, 2.00 eq) was then added thereto. The solution was stirred at 20°C for two hours. The solution was concentrated under reduced pressure to give a brown residue. The residue was purified by silica gel chromatography (silica, petroleum ether / ethanol = 100/1 to 10/1) twice to give Compound 7-8.

**Step H:** Palladium on carbon (1.00 g, 10%) was added to a solution of 7-8 (8.01 g, 13.86 mmol, 1.00 eq) in tetrahydrofuran (15.00 mL) under nitrogen atmosphere. After the suspension was purged with hydrogen (2.80 g, 1.39 mol, 100.00 eq, 15 psi) several times. Afterwards, the mixture was then stirred at 15°C for two hours under hydrogen atmosphere. The reaction mixture was filtered and concentrated under reduced pressure to give a yellow gum, which was triturated with petroleum ether (30.00 mL * 2) and filtered to give Compound 7-9.

**Step I:** Potassium carbonate (0.45 g, 3.24 mmol, 2.00 eq) and potassium iodide (2.69 mg, 16.21 μmol, 0.01 eq) were added to a solution of 7-9 (0.79 g, 1.62 mmol, 1.00 eq) in *N,N*-dimethylformamide (4.00 mL). The resulting mixture was stirred at 100°C for 10 hours. The solution was then poured into 10.00 mL of water and extracted with ethyl acetate (30.00 mL*2). The organic phases were combined, washed with water (5.00 mL * 3) and saturated brine (5.00 mL * 3), dried over anhydrous sodium sulfate and concentrated under reduced pressure to give Compound 7-10.

**Step J:** Boron tribromide (1.15 g, 4.59 mmol, 442.31 μL, 6.00 eq) was added dropwise to a solution of 7-10 (300.00 mg, 765.62 μmol, 1.00 eq) in dichloromethane (30.00 mL) while maintaining the temperature below -78°C. After completion of the addition, the solution was stirred between -78°C and 0°C for 10 hours. The reaction solution was quenched with MeOH and concentrated under reduced pressure to give a yellow liquid. A solution of dichloromethane/methanol (10/1, 100.00 mL) was added to the yellow liquid. The organic phase was separated, dried over anhydrous sodium sulfate and concentrated under reduced pressure to give Compound 7-11.

**Step K:** Thionyl chloride (687.08 mg, 5.78 mmol, 418.95 μL, 10.00 eq) was added to a solution of 7-11 (202.00 mg, 577.52 μmol, 1.00 eq) in methanol (21.18 g, 661.15 mmol, 26.81 mL, 1144.80 eq) under nitrogen atmosphere in one portion. The mixture was stirred at 50°C for 4 hours. The reaction mixture was concentrated under reduced pressure to give Compound 7-12.

**Step L:** 7-12 (70.00 mg, 192.22 μmol) was dissolved in *N,N*-dimethylformamide (2.00 mL), followed by addition of potassium carbonate (34.57 mg, 250.15 μmol) and 2-chloroethylethyl sulfide (31.18 mg, 250.15 μmol). The mixture was stirred at 100°C for 12 hours to give 7-13. The reaction mixture was directly used in the next step without purification.

**Step M:** 7-13 (86.97 mg, 192.43 μmol) was dissolved in water (1.00 mL), and potassium carbonate (26.59 mg, 192.43 μmol) was added thereto. The mixture was stirred at 100°C for 12 hours. The pH of the reaction mixture was adjusted to 3-4 and the reaction mixture was purified by high performance preparative liquid chromatography (column: Boston Green ODS 150*30 4 μm; mobile phase: [water (0.225% formic acid)-acetonitrile]; elution gradient: 55%-85%, 10.5 minutes) to give Embodiment 7.

ee value (enantiomeric excess): 100%.

SFC (Supercritical Fluid Chromatography) method: Column: Chiral pak AD-3 100 mm × 4.6 mm I.D., 3 μm.

Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$ 8.78 (s, 1H), 7.75 (s, 1H), 7.03 (s, 1H), 6.92 (s, 1H), 4.71 (br s, 2H), 4.55 (br d, *J*=9.2 Hz, 1H), 4.34 - 4.25 (m, 2H), 2.92 (t, *J*=6.3 Hz, 2H), 2.67 (q, *J*=7.3 Hz, 2H), 1.83 (br s, 1H), 1.21 (t, *J*=7.4 Hz, 3H), 0.98 (d, *J*=6.5 Hz, 3H), 0.71 (d, *J*=6.5 Hz, 3H).

**Embodiments 8 can be prepared by the method referring to the preparation method of Embodiment 7:**

**Embodiment 8**

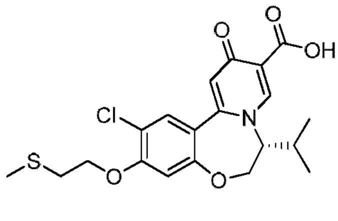

ee value (enantiomeric excess): 100%.

SFC (Supercritical Fluid Chromatography) method: Column: Chiral pad AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$ 8.79 (s, 1H), 7.76 (s, 1H), 7.03 (s, 1H), 6.93 (s, 1H), 4.71 (br s, 2H), 4.55 (br d, *J*=10.7 Hz, 1H), 4.35 - 4.27 (m, 2H), 2.90 (t, *J*=6.3 Hz, 2H), 2.20 (s, 3H), 2.08 (s, 1H), 0.98 (d, *J*=6.5 Hz, 3H), 0.71 (d, *J*=6.5 Hz, 3H).

**Embodiment 9**

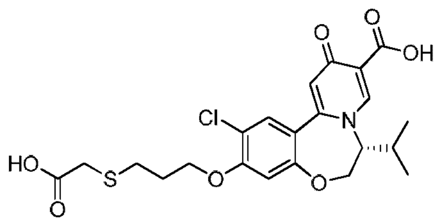

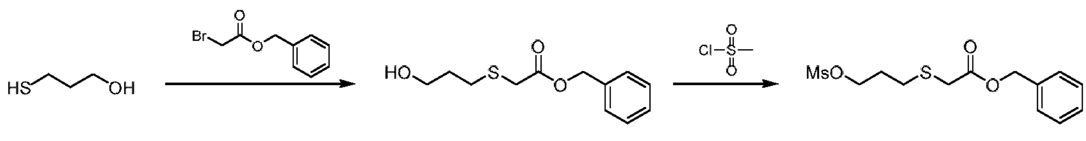

9-1 9-2 9-3

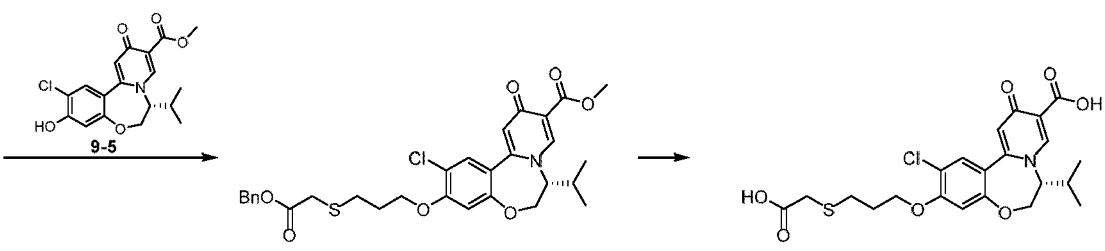


9-4 Embodiment 9

Compound 9-5 can be prepared by the method referring to the preparation method of Compound 7-12:

**Step A:** 9-1 (5.00 g, 54.25 mmol) was dissolved in *N*,*N*-dimethylformamide (20.00 mL), followed by addition of diisopropylethylamine (10.52 g, 81.38 mmol) and benzyl 2-bromoacetate (12.43 g, 54.25 mmol). The mixture was stirred at 15°C

for 16 hours. The reaction mixture was filtered to give a filtrate, followed by addition of water (50.00 mL) and extraction with ethyl acetate (50.00 mL * 2). The combined organic phases were washed with saturated brine (30.00 mL), dried over anhydrous sodium sulfate and evaporated under reduced pressure to give Compound 9-2.

$^1$H NMR (400MHz, $CDCl_3$) $\delta$7.24-7.32 (m, 5H), 5.10 (s, 2H), 3.64 (br d, *J*=3.06 Hz, 2H), 3.21 (s, 2H), 2.66 (t, *J*=7.09 Hz, 2H), 1.70-1.80 (m, 2H).

**Step B:** 9-2 (5.00 g, 20.81 mmol) was dissolved in dichloromethane (50.00 mL), followed by addition of triethylamine (3.16 g, 31.22 mmol) and methanesulfonyl chloride (2.62 g, 22.89 mmol). The mixture was stirred at 10°C for 3 hours. The reaction solution was directly used in the next step without purification. The reaction mixture was quenched with water (50.00 mL) and extracted with dichloromethane (50.00 mL * 2). The combined organic phases were then washed with water (50.00 mL) and saturated brine (50.00 mL), dried over anhydrous sodium sulfate and evaporated under reduced pressure to give Compound 9-3.

$^1$H NMR (400MHz, $CDCl_3$) $\delta$7.34-7.42 (m, 5H), 5.20 (s, 2H), 4.31 (t, *J*=6.09 Hz, 2H), 3.29 (s, 2H), 3.02 (s, 3H), 2.76 (t, *J*=7.03 Hz, 2H), 1.99-2.07 (m, 2H).

**Step C:** 9-3 (91.90 mg, 288.63 μmol) was dissolved in *N*,*N*-dimethylformamide (2.00 mL), followed by addition of potassium carbonate (45.21 mg, 327.11 μmol). The mixture was stirred at 70°C for 12 hours to give 9-4. The reaction mixture was directly used in the next step without purification.

**Step D:** 9-4 (112.78 mg, 192.43 μmol) was dissolved in water (2.00 mL), and potassium carbonate (26.59 mg, 192.43 μmol) was added thereto. The mixture was stirred at 100°C for 12 hours. The pH of the reaction mixture was adjusted to 3-4 and the reaction mixture was purified by high performance preparative liquid chromatography (column: Boston Green ODS 150*25 10 μm; mobile phase: [water (0.225% formic acid)-acetonitrile]; elution gradient: 30%-60%, 11 min) to give Embodiment 9.

ee value (enantiomeric excess): 81.8%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$8.78 (s, 1H), 7.75 (s, 1H), 7.02 (s, 1H), 6.90 (s, 1H), 4.70 (br s, 2H), 4.55 (br d, *J*=10.3 Hz, 1H), 4.27 - 4.11 (m, 2H), 3.27 (s, 2H), 2.76 (t, *J*=7.2 Hz, 2H), 2.07 - 1.97 (m, 2H), 1.84 (br s, 1H), 0.98 (d, *J*=6.5 Hz, 3H), 0.71 (br d, *J*=6.4 Hz, 3H).

**Embodiment 10**

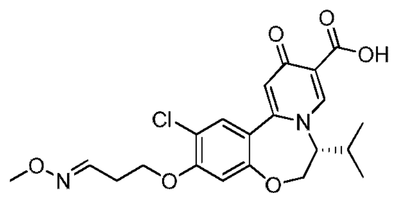

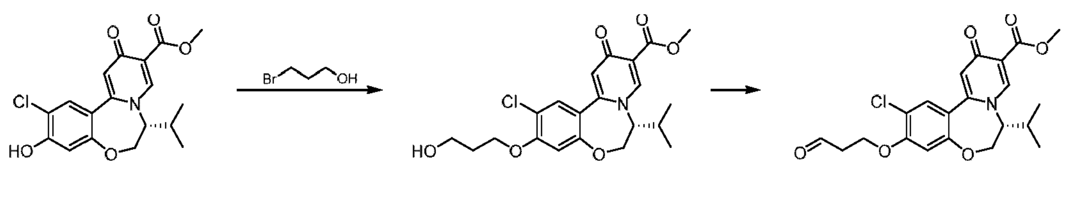

10-1 10-2 10-3

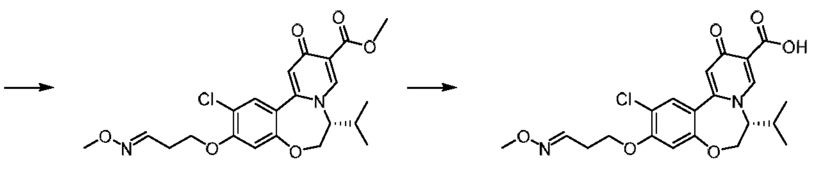

10-4 Embodiment 10

Compound 10-1 can be prepared by the method referring to the preparation method of Compound 7-12:

**Step A:** 10-1 (700.00 mg, 1.92 mmol) was dissolved in *N*,*N*-dimethylformamide (20.00 mL), followed by addition of potassium carbonate (452.09 mg, 3.27 mmol) and 3-bromo-1-propanol (401.13 mg, 2.89 mmol). The mixture was stirred at 50°C for 5 hours, followed by addition of water (45.00 mL) and extraction with 150.00 mL (50.00 mL *3) of dichloromethane. The combined organic phases were dried over anhydrous sodium sulfate and evaporated under reduced pressure to give a crude product. The crude product was purified by high performance preparative liquid chromatography (column: Boston Green ODS 250*50 mm, 10 μm; mobile phase: [water (0.225% formic acid)-acetonitrile]; elution gradient: 15%-45%; 30 min) to give Compound 10-2.

$^1$H NMR (400MHz, $CDCl_3$) $\delta$ 8.21 (s, 1H), 7.53 (s, 1H), 6.73 (s, 1H), 6.64 (s, 1H), 4.68 - 4.46 (m, 2H), 4.28 - 4.20 (m, 2H), 3.95 (s, 3H), 3.73 (ddd, *J*=2.9, 5.3, 10.9 Hz, 1H), 2.15 (quin, *J*=5.8 Hz, 2H), 2.10 - 2.03 (m, 1H), 1.28 (s, 2H), 1.08 (d, *J*=6.5 Hz, 3H), 0.90 (d, *J*=6.5 Hz, 3H).

**Step B:** 10-2 (100.00 mg, 237.04 μmol) was dissolved in dichloromethane (20.00 mL), and Dess-Martin oxidant (110.59 mg, 260.74 μmol) was added thereto at 0°C. The mixture was stirred at 20°C for 2 hours. Saturated sodium bicarbonate (20.00 mL) and sodium thiosulfate (20.00 mL) were added thereto, followed by filtration to give a filtrate. The organic phase was washed with saturated sodium bicarbonate (20.00 mL * 3). The organic phases were combined, dried over anhydrous sodium sulfate and evaporated under reduced pressure to give a crude product. The crude product was purified by silica gel column chromatography (eluent: dichloromethane/methanol = 10/1) to give Compound 10-3.

**Step C:** 10-3 (40.00 mg, 95.27 μmol) and methoxylamine hydrochloride (9.55 mg, 114.33 μmol) were dissolved in dichloromethane (10.00 mL), and pyridine (9.04 mg, 114.33μmol) was added thereto. The mixture was stirred at 15°C for 12 hours, and rotary evaporated to remove the solvent, followed by dilution with 15.00 mL of water and extraction with 30.00 mL of ethyl acetate (10.00 mL*3). The combined organic phases were then dried over anhydrous sodium sulfate and evaporated under reduced pressure to give a crude product. The crude product was purified by silica gel plate (eluent: dichloromethane/methanol = 10/1) to give Compound 10-4.

**Step D:** 10-4 (30.00 mg, 66.30 μmol) was dissolved in methanol (9.00 mL), and sodium hydroxide solution (4.00 mol, 3.00 mL) was added thereto. The mixture was stirred at 15°C for 0.5 hour. The pH of the reaction mixture was adjusted to 3-4 and the reaction mixture was purified by high performance preparative liquid chromatography (column: Boston Green ODS 150*30 4 μm; mobile phase: [water (0.225% formic acid)-acetonitrile]; elution gradient: 50%-74%, 10.5 min) to give Embodiment 10.

ee value (enantiomeric excess): 5.8%.

SFC (Supercritical Fluid Chromatography) method: Column: Chiral OD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$ 8.77 (br s, 1H), 7.74 (br s, 1H), 7.49 (t, *J*=5.8 Hz, 1H), 7.01 (br s, 1H), 6.94 - 6.86 (m, 1H), 4.69 (br s, 2H), 4.54 (br s, 1H), 4.36 - 4.20 (m, 2H), 3.83 - 3.71 (m, 3H), 2.78 - 2.61 (m, 2H), 1.83 (br s, 1H), 0.98 (d, *J*=6.4 Hz, 3H), 0.71 (br d, *J*=6.4 Hz, 3H).

**Embodiment 11**

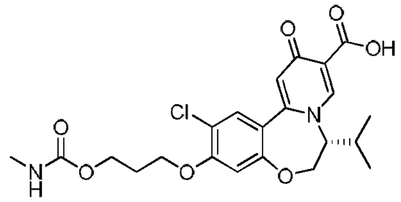

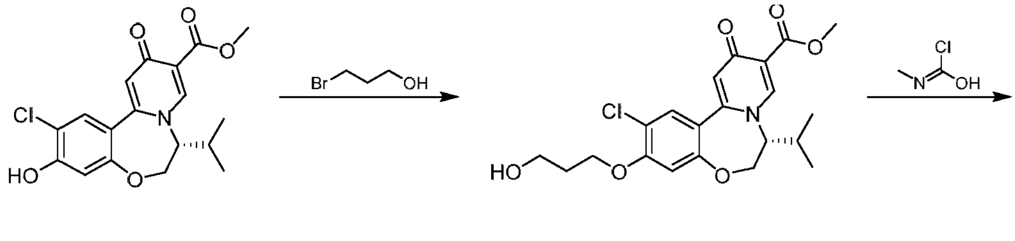

11-1 11-2

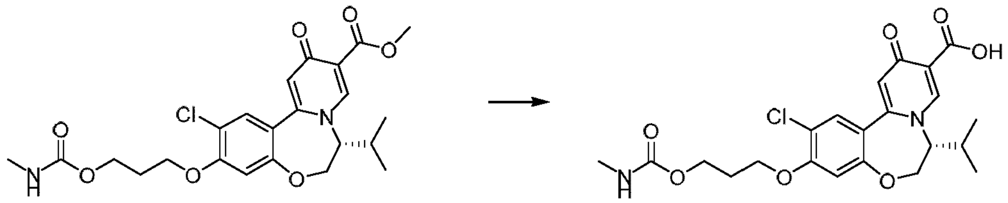

11-3 Embodiment 11

Compound 11-1 can be prepared by the method referring to the preparation method of Compound 7-12:

**Step A:** 11-1 (700.00 mg, 1.92 mmol) was dissolved in *N*,*N*-dimethylformamide (20.00 mL), followed by addition of potassium carbonate (452.09 mg, 3.27 mmol) and 3-bromo-1-propanol (401.13 mg, 2.89 mmol). The mixture was stirred at 50°C for 5 hours, followed by addition of water (45.00 mL) and extraction with 150.00 mL (50.00 mL *3) of dichloromethane. The combined organic phases were dried over anhydrous sodium sulfate and evaporated under reduced pressure to give a crude product. The crude product was purified by high performance preparative liquid chromatography (column: Boston Green ODS 250*50 mm, 10 μm; mobile phase: [water (0.225% formic acid)-acetonitrile]; elution gradient: 15%-45%; 30 min) to give Compound 11-2.

$^1H$ NMR (400MHz, $CDCl_3$) $\delta$ 8.21 (s, 1H), 7.53 (s, 1H), 6.73 (s, 1H), 6.64 (s, 1H), 4.68 - 4.46 (m, 2H), 4.28 - 4.20 (m, 2H), 3.95 (s, 3H), 3.73 (ddd, *J*=2.9, 5.3, 10.9 Hz, 1H), 2.15 (quin, *J*=5.8 Hz, 2H), 2.10 - 2.03 (m, 1H), 1.28 (s, 2H), 1.08 (d, *J*=6.5 Hz, 3H), 0.90 (d, *J*=6.5 Hz, 3H).

**Step B:** 11-2 (80.00 mg, 189.63 mmol), 4-dimethylaminopyridine (231.67 μg, 1.90 μmol) and triethylamine (57.57 mg, 568.89 μmol) were dissolved in dichloromethane (20.00 mL), followed by addition of methylaminoformyl chloride (35.47 mg, 379.26 μmol). The mixture was stirred at 15°C for 5 hours, followed by addition of water (15.00 mL) and extraction with 45.00 mL (15.00 mL *3) of dichloromethane. The combined organic phases were then dried over anhydrous sodium sulfate and evaporated under reduced pressure to give Compound 11-3.

**Step C:** 11-3 (90.00 mg, 187.92 μmol) was dissolved in methanol (10.00 mL), tetrahydrofuran (10.00 mL) and water (10.00 mL), and lithium hydroxide monohydrate (23.66 mol, 563.77 μmol) was added thereto. The mixture was stirred at 20°C for 12 hours. The pH of the reaction mixture was adjusted to 3-4, and the reaction mixture was diluted with 20.00 mL of water and extracted with 45.00 mL (15.00 mL *3) of dichloromethane. The combined organic phases were then dried over anhydrous sodium sulfate and evaporated under reduced pressure to give a crude product. The crude product was purified by high performance preparative liquid chromatography (column: Boston Green ODS 150*30 4 μm; mobile phase: [water (0.225% formic acid)-acetonitrile]; elution gradient: 35%-65%, 10.5 min) to give Embodiment 11.

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1H$ NMR (400MHz, DMSO-$d_6$) $\delta$ 8.79 (s, 1H), 7.76 (s, 1H), 7.11 - 6.95 (m, 2H), 6.89 (s, 1H), 4.71 (br s, 2H), 4.56 (br d, *J*=7.5 Hz, 1H), 4.23 - 4.07 (m, 4H), 2.56 (d, *J*=4.5 Hz, 3H), 2.08 - 2.02 (m, 2H), 1.96 - 1.71 (m, 1H), 0.98 (d,

*J*=6.5 Hz, 3H), 0.71 (br d, *J*=6.5 Hz, 3H).

**Embodiment 12**

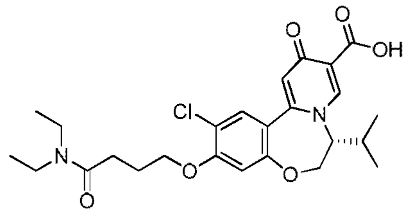

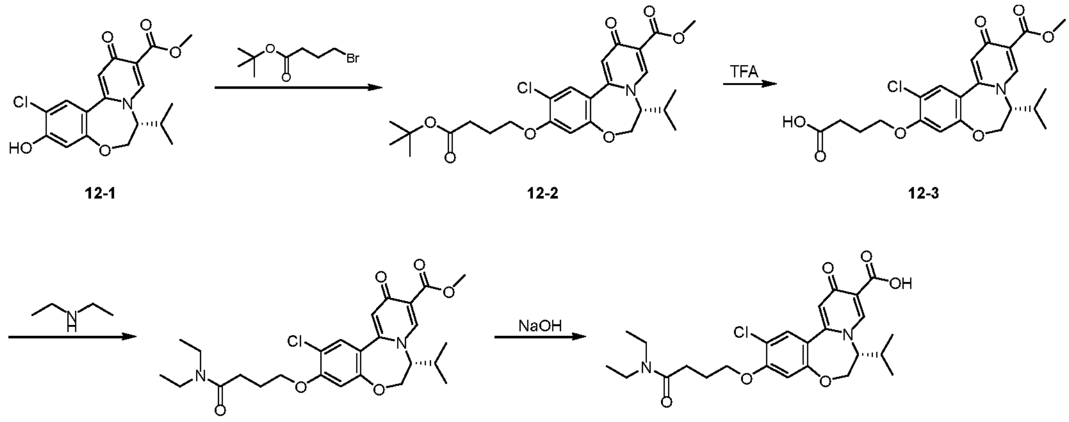


12-1 12-2 12-3

12-4 Embodiment 12

Compound 12-1 can be prepared by the method referring to the preparation method of Compound 7-12:

**Step A:** 12-1 (100.00 mg, 274.88 μmol) was dissolved in *N*,*N*-dimethylformamide (2.00 mL), followed by addition of potassium carbonate (49.39 mg, 357.34 μmol) and 4-bromobutyl *tert*-butyl ester (79.73 mg, 357.34 μmol). The mixture was stirred at 100°C for 12 hours, followed by addition of water (30.00 mL) and extraction with 45.00 mL (15.00 mL *3) of dichloromethane. The combined organic phases were dried over anhydrous sodium sulfate and evaporated under reduced pressure to give a crude product 12-2.

**Step B:** 12-2 (128.00 mg, 252.97 μmol) was dissolved in dichloromethane (3.00 mL), followed by addition of trifluoroacetic acid (4.62 mg, 40.52 μmol). The mixture was stirred at 15°C for 1 hour and rotary evaporated to remove the solvent to give a crude product 12-3.

**Step C:** 12-3 (80.00 mg, 177.83 μmol) was dissolved in dichloromethane (10.00 mL), followed by addition of 2-(7-azabenzotriazol)-*N*,*N*,*N'*,*N'*-tetramethyluronium hexafluorophosphate (HATU) (81.14 mg, 213.39 μmol) and triethylamine (26.99 mg, 266.74 μmol). The mixture was stirred at 25°C for 30 minutes, followed by addition of diethylamine (15.61 mg, 213.39 μmol). The mixture was stirred at 25°C for 12 hours, followed by addition of water (20.00 mL) and extraction with 45.00 mL (15.00 mL *3) of dichloromethane. The combined organic phases were then dried over anhydrous sodium sulfate and evaporated under reduced pressure to give 12-4.

**Step D:** 12-4 (80.00 mg, 158.22 μmol) was dissolved in methanol (4.50 mL), and sodium hydroxide solution (4.00 mol, 1.71 mL) was added thereto. The mixture was stirred at 20°C for 5 minutes. The pH of the reaction mixture was adjusted to 2-3 and the reaction mixture was purified by high performance preparative liquid chromatography (column: Boston Green ODS 150*25 mm, 10 μm; mobile phase: [water (0.225% formic acid)-acetonitrile]; elution gradient: 40%-70%, 11 min) to give Embodiment 12.

ee value (enantiomeric excess): 99.5%.

SFC (Supercritical Fluid Chromatography) method: Column: Chiralcel OD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1H$ NMR (00MHz, DMSO-$d_6$) δ 8.77 (br s, 1H), 7.74 (s, 1H), 7.12 - 6.79 (m, 2H), 4.77 - 4.48 (m, 1H), 4.80 - 4.45 (m, 2H), 4.22 - 4.10 (m, 2H), 3.29 (br dd, *J*=7.0, 11.7 Hz, 4H), 2.49 - 2.45 (m, 2H), 1.98 (quin, *J*=6.6 Hz, 2H), 1.82 (br s, 1H), 1.10 (t, *J*=7.1 Hz, 3H), 1.04 - 0.96 (m, 6H), 0.71 (br d, *J*=6.4 Hz, 3H).

Embodiment 13

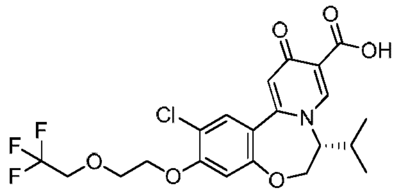

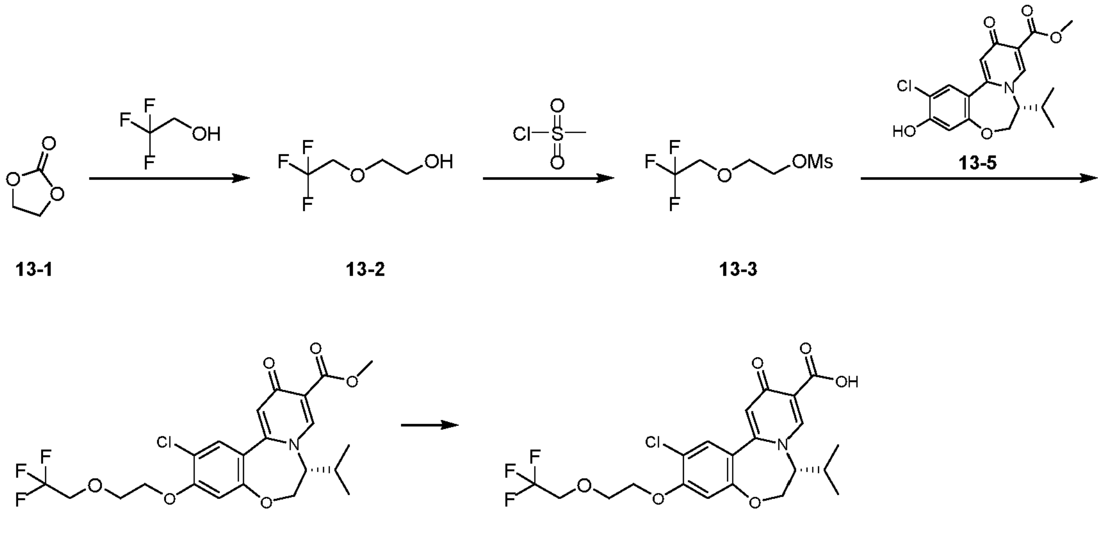


13-1 13-2 13-3

13-4 Embodiment 13

Compound 13-5 can be prepared by the method referring to the preparation method of Compound 7-12:

**Step A:** A mixture of 13-1 (13.20 g, 150.00 mmol), 2,2,2,-trifluoroethanol (10.00 g, 99.96 mmol), triethylamine (10.11 g, 100.00 mmol) and tetrabutylammonium iodide (738.24 mg, 2.00 mmol) was purged with nitrogen gas. The mixture was stirred at 100°C for 24 hours. The reaction mixture was distilled to give Compound 13-2.

$^1$H NMR (400MHz, $CDCl_3$) $\delta$ 3.91 (q, *J*=8.74 Hz, 2H), 3.72-3.82 (m, 4H), 2.21 (br s, 1H).

**Step B:** 13-2 (1.00 g, 6.94 mmol) was dissolved in dichloromethane (15.00 mL), and triethylamine (912.94 mg, 9.02 mmol) was added thereto, followed by dropwise addition of methanesulfonyl chloride (1.15 g, 10.04 mmol) at 0°C. The mixture was stirred at 20°C for 2 hours. The reaction mixture was directly used in the next step without purification. The reaction mixture was quenched with saturated sodium carbonate (20.00 mL), diluted with water (10.00 mL), extracted with 60.00 mL (20.00 mL * 3) of dichloromethane, dried over anhydrous sodium sulfate and distilled under reduced pressure to give Compound 13-3.

$^1$H NMR (400MHz, $CDCl_3$) $\delta$ 4.43 - 4.39 (m, 2H), 3.96 - 3.88 (m, 4H), 3.07 (s, 3H).

**Step C:** 13-3 (85.50 mg, 384.84 μmol) was dissolved in *N*,*N*-dimethylformamide (2.00 mL), followed by addition of potassium carbonate (53.19 mg, 384.84 μmol). The mixture was stirred at 100°C for 12 hours to give 13-4. The reaction mixture was directly used in the next step without purification.

**Step D:** 13-4 (94.26 mg, 192.22 μmol) was dissolved in water (0.50 mL) and the mixture was stirred at 100°C for 12 hours. The pH of the reaction mixture was adjusted to 3-4 and the reaction mixture was purified by high performance preparative liquid chromatography (column: Boston Green ODS 150*30 4 μm; mobile phase: [water (0.225% formic acid)-acetonitrile]; elution gradient: 50%-80%, 10.5 minutes) to give Embodiment 13.

ee value (enantiomeric excess): 83.8%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$ 8.76 (s, 1H), 7.75 (s, 1H), 6.99 (br s, 1H), 6.92 (s, 1H), 4.75 - 4.63 (m, 2H), 4.53 (br d, *J*=9.8 Hz, 1H), 4.36 - 4.27 (m, 2H), 4.19 (q, *J*=9.3 Hz, 2H), 3.98 (t, *J*=4.2 Hz, 2H), 1.93 - 1.74 (m, 1H), 0.98 (d, *J*=6.4 Hz, 3H), 0.71 (d, *J*=6.5 Hz, 3H).

**Embodiments 14 to 21 can be prepared by the method referring to the preparation method of Embodiment 7:**

**Embodiment 14 (reference)**

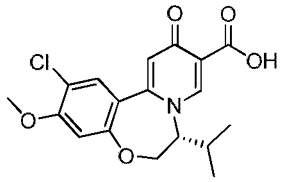

ee value (enantiomeric excess): 67.6%.
SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.
$^{1}$H NMR (400MHz, DMSO-$d_6$) $\delta$ 8.78 (s, 1H), 7.75 (s, 1H), 7.02 (s, 1H), 6.89 (s, 1H), 4.71 (br d, *J*=3.4 Hz, 2H), 4.55 (br d, *J*=10.4 Hz, 1H), 3.92 (s, 3H), 1.91 - 1.71 (m, 1H), 0.99 (d, *J*=6.5 Hz, 3H), 0.71 (d, *J*=6.5 Hz, 3H).

**Embodiment 15**

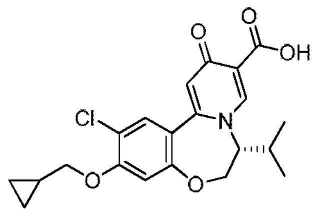

ee value (enantiomeric excess): 100%.
SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.
$^{1}$H NMR (400MHz, DMSO-$d_6$) $\delta$ 8.76 (br s, 1H), 7.73 (s, 1H), 7.11 - 6.75 (m, 2H), 4.73 - 4.47 (m, 3H), 3.98 (dd, *J*=4.7, 6.8 Hz, 2H), 1.99 - 1.67 (m, 1H), 1.32 - 1.22 (m, 1H), 0.97 (d, *J*=6.5 Hz, 3H), 0.71 (br d, *J*=6.5 Hz, 3H), 0.60 (br dd, *J*=1.5, 7.9 Hz, 2H), 0.37 (q, *J*=4.5 Hz, 2H).

**Embodiment 16**

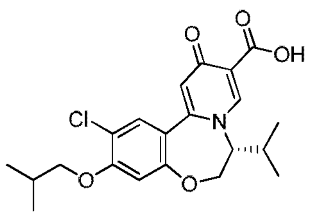

ee value (enantiomeric excess): 100%.
SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.
$^{1}$H NMR (400MHz, DMSO-$d_6$) $\delta$ 8.76 (s, 1H), 7.73 (s, 1H), 6.99 (br s, 1H), 6.87 (s, 1H), 4.69 (br d, *J*=2.9 Hz, 2H), 4.53 (br d, *J*=11.3 Hz, 1H), 3.90 (dd, *J*=2.7, 6.5 Hz, 2H), 2.10 - 2.04 (m, 1H), 1.92 - 1.75 (m, 1H), 1.01 (d, *J*=6.7 Hz, 6H), 0.98 (d, *J*=6.5 Hz, 3H), 0.71 (d, *J*=6.5 Hz, 3H).

**Embodiment 17**

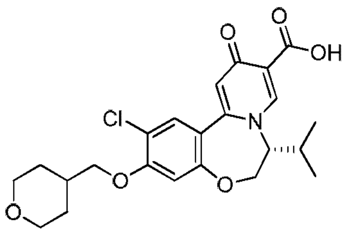

ee value (enantiomeric excess): 100%.
SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.
$^1$H NMR (400MHz, DMSO-$d_6$) δ 8.76 (s, 1H), 7.73 (s, 1H), 6.98 (br s, 1H), 6.89 (s, 1H), 4.71 - 4.49 (m, 3H), 4.00 (br d, *J*=5.7 Hz, 2H), 3.91 - 3.87 (m, 2H), 3.35 (br s, 2H), 2.12 - 2.01 (m, 2H), 1.73 - 1.62 (m, 2H), 1.43 - 1.32 (m, 2H), 0.98 (br d, *J*=6.5 Hz, 3H), 0.71 (br d, *J*=6.5 Hz, 3H).

**Embodiment 18 (reference)**

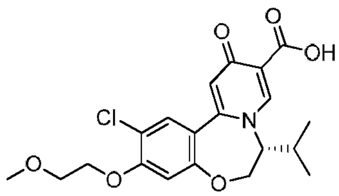

ee value (enantiomeric excess): 100%.
SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.
$^1$H NMR (400MHz, DMSO-$d_6$) δ 8.76 (br s, 1H), 7.74 (s, 1H), 7.16 - 6.83 (m, 2H), 4.70 (br s, 2H), 4.52 (br s, 1H), 4.31 - 4.21 (m, 2H), 3.70 (t, *J*=4.4 Hz, 2H), 3.34 (s, 3H), 1.94 - 1.68 (m, 1H), 0.98 (d, *J*=6.4 Hz, 3H), 0.71 (d, *J*=6.5 Hz, 3H).

**Embodiment 19**

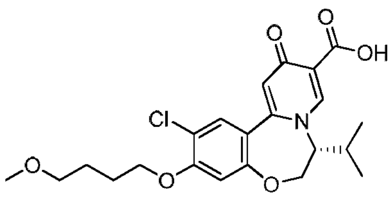

ee value (enantiomeric excess): 100%.
SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.
$^1$H NMR (400MHz, DMSO-$d_6$) δ 8.78 (s, 1H), 7.74 (s, 1H), 7.01 (br s, 1H), 6.88 (s, 1H), 4.70 (br d, *J*=2.4 Hz, 2H), 4.55 (br d, *J*=8.9 Hz, 1H), 4.21 - 4.06 (m, 2H), 3.40 (br s, 2H), 3.24 (s, 3H), 1.90 - 1.72 (m, 3H), 1.71 - 1.63 (m, 2H), 0.98 (d, *J*=6.5 Hz, 3H), 0.71 (br d, *J*=6.5 Hz, 3H).

**Embodiment 20**

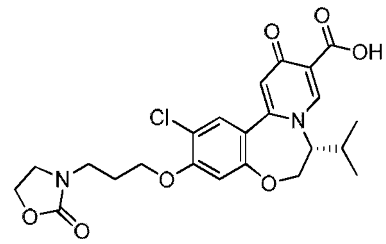

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$ 8.78 (s, 1H), 7.76 (s, 1H), 7.03 (s, 1H), 6.88 (s, 1H), 4.70 (br d, *J*=2.8 Hz, 2H), 4.55 (br d, *J*=8.4 Hz, 1H), 4.30 - 4.23 (m, 2H), 4.21 - 4.11 (m, 2H), 3.69 - 3.50 (m, 2H), 3.35 - 3.33 (m, 2H), 2.00 (quin, *J*=6.4 Hz, 2H), 1.94 - 1.73 (m, 1H), 0.98 (d, *J*=6.5 Hz, 3H), 0.71 (br d, *J*=6.5 Hz, 3H).

**Embodiment 21**

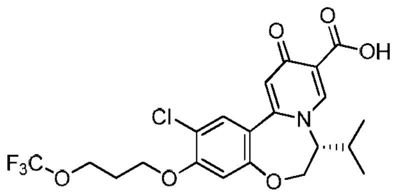

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, DMSO-$d_6$) $\delta$ 8.78 (s, 1H), 7.76 (s, 1H), 7.03 (s, 1H), 6.93 (s, 1H), 4.70 (br d, *J*=3.1 Hz, 2H), 4.55 (br d, *J*=10.0 Hz, 1H), 4.29 - 4.20 (m, 4H), 2.18 (quin, *J*=6.0 Hz, 2H), 1.83 (br s, 1H), 0.98 (d, *J*=6.4 Hz, 3H), 0.71 (d, *J*=6.5 Hz, 3H).

**Embodiment 22**

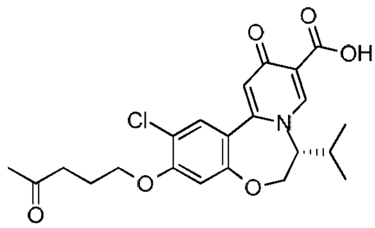

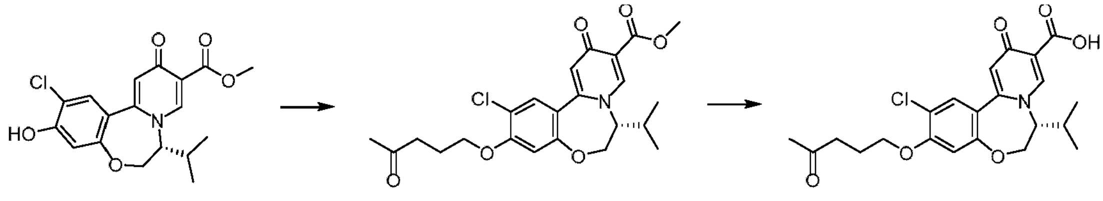

22-1 22-2 Embodiment 22

**Compound 22-1 can be prepared by the method referring to the preparation method of Compound 7-12:**

**Step A:** Potassium carbonate (182.36 mg, 1.32 mmol, 1.60 eq) was added to a solution of 22-1 (300.00 mg, 824.65 μmol, 1.00 eq) and 5-bromo-2-pentanone (176.92 mg, 1.07 mmol, 1.30 eq) in *N*,*N*-dimethylformamide (3.00 mL) in one portion. The solution was stirred at 110°C for 10 hours, and 1.00 mol/L dilute hydrochloric acid (5.00 mL) was added thereto. The mixture was stirred at 10°C for 10 minutes, and extracted with ethyl acetate (30.00 mL *3). The combined organic phases were washed with water (10.00 mL*3) and saturated brine (10.00 mL*3), dried over anhydrous sodium sulfate, and concentrated under reduced pressure to give a yellow solid, which was separated by silica gel plate chromatography (dichloromethane / methanol 10/1) (twice) to give Compound 22-2.

**Step B:** 4 mol/L aqueous sodium hydroxide solution (0.50 mL) was added to a solution of 22-2 (50.00 mg, 111.63 μmol, 1.00 eq) in methanol (3.00 mL) in one portion. The mixed solution was stirred at 40°C for 10 minutes, followed by addition of 1.00 mL of 1.00 mol/L dilute aqueous hydrochloric acid solution and concentration under reduced pressure to give a yellow solid. The yellow solid was purified by high performance preparative liquid chromatography (column: Phenomenex Gemini 150 mm * 25 mm * 10 μm; mobile phase: [water (0.05% ammonia) - acetonitrile]; elution gradient: 9% - 39%, 10 minutes) to give Embodiment 22.

ee value (enantiomeric excess): 93%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, $CD_3OD$) $\delta$ = 8.43 (br s, 1H), 7.57 (br s, 1H), 6.84 - 6.67 (m, 2H), 4.75 - 4.51 (m, 2H), 4.13 (br d, *J*=5.6 Hz, 3H), 2.75 (br t, *J*=6.8 Hz, 2H), 2.20 (s, 3H), 2.13 - 2.08 (m, 2H), 1.95 (br s, 1H), 1.07 (br d, *J*=6.2 Hz, 3H), 0.82 (br d, *J*=6.0 Hz, 3H).

**Embodiment 23**

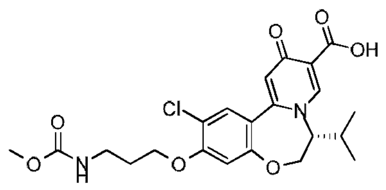

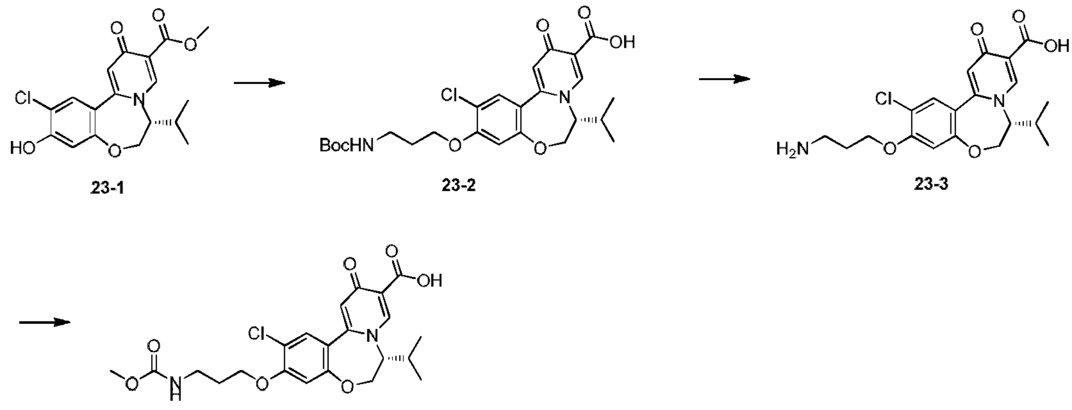

23-1 23-2 23-3

Embodiment 23

**Compound 23-1 can be prepared by the method referring to the preparation method of Compound 7-12:**

**Step A:** Potassium carbonate (121.57 mg, 879.63 μmol, 1.60 eq) was added to a solution of 23-1 (200.00 mg, 549.77 μmol, 1.00 eq) in *N,N*-dimethylformamide (8.00 mL). The solution was stirred at 110°C for 1 hour. 3.00 mL of water was added to the solution, and the suspension was filtered to give a brown solid. The brown solid was separated by preparative silica gel plate (dichloromethane / methanol 10/1) twice to give Compound 23-2.

**Step B:** Trifluoroacetic acid (950.29 mg, 8.33 mmol, 617.07 μL, 48.91 eq) was added dropwise to a solution of 23-2 (84.00 mg, 170.40 μmol, 1.00 eq) in dichloromethane (2.00 mL). The resulting solution was stirred at 10°C for three minutes and concentrated under reduced pressure to give Compound 23-3.

**Step C:** Methyl chloroformate (37.16 mg, 393.26 μmol, 30.46 μL, 2.00 eq) was slowly added dropwise to a solution of 23-3 (80.00 mg, 196.63 μmol, 1.00 eq) and triethylamine (50.74 mg, 501.41 μmol, 69.50 μL, 2.55 eq) in dichloromethane (2.00 mL) at 0°C under nitrogen atmosphere within 5 minutes. The resulting solution was stirred at 0-14°C for 30 minutes, and separated by high performance liquid chromatography (column: Phenomenex Synergi C18 150 mm * 25 mm * 10 μm; mobile phase: [water (0.225% formic acid) - acetonitrile]; elution gradient: 33% - 53%, 10 minutes) to give Embodiment 23.

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, $CDCl_3$) $\delta$ = 15.61 (br s, 1H), 8.38 (br s, 1H), 7.45 (s, 1H), 6.78 (s, 1H), 6.56 (s, 1H), 5.08 (br s, 1H), 4.65 - 4.45 (m, 2H), 4.07 (br s, 2H), 3.79 (br s, 1H), 3.61 (s, 3H), 3.39 (br d, *J*=5.1 Hz, 2H), 2.04 (br s, 2H), 1.18 (s, 1H), 1.02 (br d, *J*=5.9 Hz, 3H), 0.81 (br d, *J*=6.1 Hz, 3H).

**Embodiment 24**

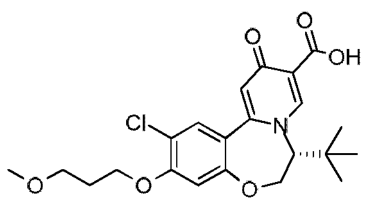

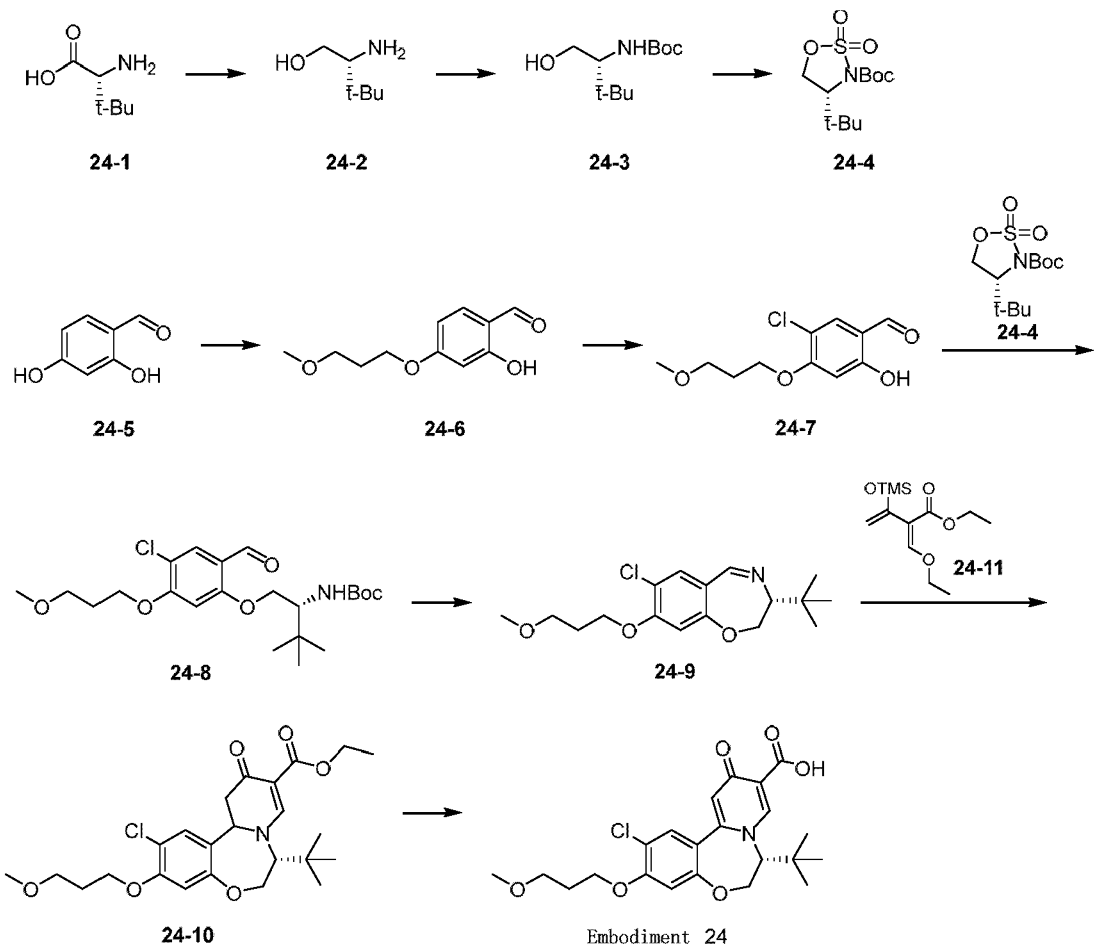

24-1 24-2 24-3 24-4

24-4

24-5 24-6 24-7

24-11

24-8 24-9

24-10 Embodiment 24

**Compound 24-4 can be prepared by the method referring to the preparation method of Compound 6-4:**

**Step A:** 24-5 (100.00 g, 724.01 mmol, 1.00 eq) was dissolved in *N*,*N*-dimethylformamide (600.00 mL). After cooling to 0°C, potassium carbonate (100.06 g, 724.01 mmol, 1.00 eq) was added thereto. After the mixture was heated to 90°C, 1-bromo-3-methoxy-propane (110.79 g, 724.01 mmol, 1.00 eq) in *N*,*N*-dimethylformamide (400.00 mL) was added slowly to the solution within 1 hour. The mixed solution was further stirred at 90°C for one hour. The solution was then poured into 400.00 mL of water and extracted with ethyl acetate (800.00 mL*3). The organic phases were combined, washed with water (500.00 mL) and saturated brine (200.00 mL * 2), concentrated under reduced pressure to give a white solid and purified by silica gel column chromatography (silica, petroleum ether / ethyl acetate = 100/1 to 80/1) to give Compound 24-6.

**Step B:** 24-6 (50.00 g, 237.84 mmol, 1.00 eq) was dissolved in acetonitrile (300.00 mL) and cooled to 0°C, followed by addition of chlorosuccinimide (32.08 g, 240.22 mmol, 1.01 eq). The mixture was heated to 25°C and stirred for 10 hours. The solution was concentrated under reduced pressure to give a colorless liquid. 500 mL of ethyl acetate was poured into the liquid, and the mixture was washed with water (100.00 mL*3) and saturated brine (100.00 mL *3). The organic phase was dried over anhydrous sodium sulfate and concentrated under reduced pressure to give a white solid. The given white solid was triturated with methanol to give Compound 24-7.

**Step C:** Potassium carbonate (21.59 g, 156.21 mmol, 2.00 eq) was added to a solution of 24-7 (19.11 g, 78.10 mmol, 1.00 eq) and 24-4 (24.00 g, 85.91 mmol, 1.10 eq) in *N,N*-dimethylformamide (300.00 mL) in one portion. The solution was stirred at 50°C for two hours. The reaction mixture was poured into 100.00 mL of water, and extracted with ethyl acetate (1000.00 mL). The organic phase was separated, washed with water (100.00 mL) and saturated brine (100.00 mL), dried over anhydrous sodium sulfate and concentrated under reduced pressure to give Compound 24-8.

**Step D:** Trifluoroacetic acid (68.68 g, 602.31 mmol, 44.59 mL, 8.10 eq) was added to a solution of 24-8 (33.00 g, 74.33 mmol, 1.00 eq) in dichloromethane (30.00 mL) in one portion. The solution was stirred at 10°C for 1 hour, and concentrated under reduced pressure to give a brown oil. 100.00 mL of saturated aqueous sodium bicarbonate solution was added thereto, then the mixture was stirred at 10°C for 1 hour and extracted with 600.00 mL of ethyl acetate. The organic phase was separated, dried over anhydrous sodium sulfate and concentrated to give compound 24-9.

**Step E:** 24-9 (10.00 g, 30.69 mmol, 1.00 eq) was added to a solution of 24-11 (15.86 g, 61.38 mmol, 2.00 eq) in toluene (100.00 mL) in one portion. After the suspension was purged with nitrogen gas several times, it was heated to 120°C and stirred for 28 hours. The solution was concentrated under reduced pressure to give a brown oil. The brown oil was subjected to flash silica gel column chromatography (ISCO®; 330 g SepaFlash® flash silica gel column, 5% trifluoroacetic acid/acetonitrile as eluent, flow rate: 100 mL/min) to give Compound 24-10.

**Step F:** A solution of 24-10 (4.20 g, 9.01 mmol, 1.00 eq) and tetra-chloro-benzoquinone (5.54 g, 22.53 mmol, 2.50 eq) in toluene (60.00 mL) and glycol dimethyl ether (60.00 mL) was stirred at 120°C for 2 hours. The solution was concentrated under reduced pressure to give a brown oil. The brown oil was subjected to flash silica gel column chromatography (ISCO®; 330 g SepaFlash® flash silica gel column, mobile phase: 0-70% acetonitrile / 5% trifluoroacetic acid, flow rate: 100 mL/min) to give a brown oil. The residue was subjected to high performance liquid column chromatography (column: Phenomenex luna C18 250*50 mm*10 μm; mobile phase: [water (0.225% formic acid)-acetonitrile]; elution gradient: 38%-68%, 30 min) to give Embodiment 24.

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1H$ NMR (400MHz, $CDCl_3$) $\delta$ = 9.04 - 8.37 (m, 1H), 7.66 - 7.51 (m, 1H), 7.07 - 6.54 (m, 2H), 4.90 - 4.48 (m, 2H), 4.18 (br s, 3H), 3.62 (br s, 2H), 3.39 (s, 3H), 2.15 (quin, $J$=5.9 Hz, 2H), 1.30 - 0.91 (m, 9H).

## Embodiment 25

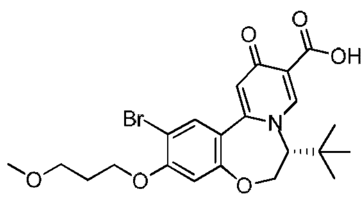

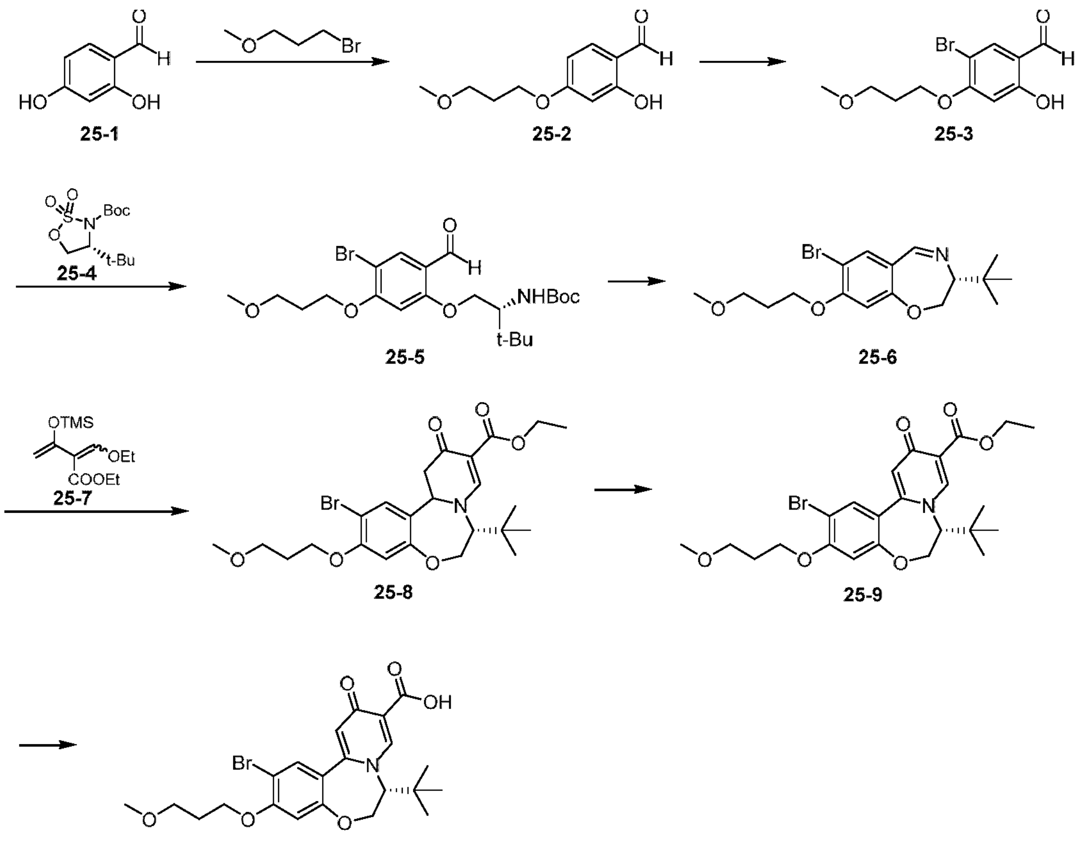

25-1 25-2 25-3

25-4

25-5 25-6

25-7

25-8 25-9

Embodiment 25

**Compound 25-4 can be prepared by the method referring to the preparation method of Compound 6-4:**

**Step A:** A mixture of 25-1 (140.00 g, 1.01 mol), potassium carbonate and *N,N*-dimethylformamide (500.00 mL) was heated at 90°C for 1 hour. A solution of 1-bromo-3-methoxy-propanol (147.34 g, 962.93 mmol) in *N,N*-dimethylformamide (100.00 mL) was then added dropwise to the mixture at 90°C and then stirred at 90°C for 5 hours. The mixture was poured into water (1500.00 mL), and extracted with ethyl acetate (1000.00 mL*2). The organic phases were combined, washed with saturated brine (1000.00 mL*3), dried over anhydrous sodium sulfate and concentrated under reduced pressure at 45°C to give a crude product. The crude product was purified by silica gel column chromatography (petroleum ether / ethyl acetate = 50/1 to 10/1) to give Compound 25-2.

**Step B:** A solution of bromine (33.34 g, 208.65 mmol, 10.76 mL) in dichloromethane (100.00 mL) was dropwise added to a solution of 25-2 (40.00 g, 189.68 mmol) in dichloromethane (300.00 mL) at 0°C under nitrogen atmosphere. After the mixture was stirred at 15°C for 1 hour, it was quenched with saturated sodium thiosulfate solution (200.00 mL), and extracted with ethyl acetate (100.00 mL*2). The organic phases were combined, washed with saturated brine (100.00 mL*2), dried over anhydrous sodium sulfate, concentrated under reduced pressure at 45°C to give Compound 25-3.

$^1$H NMR (400MHz, $CDCl_3$) $\delta$ = 11.43 (s, 1H), 9.78 - 9.63 (m, 1H), 7.69 (s, 1H), 6.50 (s, 1H), 4.20 (t, J=6.1 Hz, 2H), 3.73 - 3.52 (m, 2H), 3.39 (s, 3H), 2.23 - 2.08 (m, 2H).

**Step C:** Potassium carbonate (26.77 g, 193.69 mmol) and 25-4 were added to a solution of 25-3 (28.20 g, 96.84 mmol) in dimethylformamide (280.00 mL) at 15°C. The mixture was stirred at 50°C for 2 hours. The mixture was poured into saturated aqueous ammonium chloride solution (300.00 mL) and extracted with ethyl acetate (200.300 mL*2). The organic phase was washed with saturated brine (100.00 mL * 2), dried over anhydrous sodium sulfate, and concentrated under reduced pressure at 45°C to give compound 25-5.

**Step D:** Trifluoroacetic acid (229.01 g, 2.01 mol, 148.71 mL) was added to a solution of 25-5 (45.00 g, 91.34 mmol) in dichloromethane (150.00 mL) at 0°C. The mixture was stirred for 20 hours. The solvent was removed under reduced pressure at 45°C. The crude product was dissolved in saturated sodium bicarbonate solution (400.00 mL), extracted with ethyl acetate (200.00 mL * 4). The organic phases were combined, washed with saturated brine (200.00 mL *3), dried over anhydrous sodium sulfate and concentrated under reduced pressure at 45°C to give Compound 25-6.

$^1$H NMR (400MHz, $CDCl_3$) $\delta$ = 8.91 (s, 1H), 7.80 (s, 1H), 6.59 (s, 1H), 4.69 (br d, *J*=10.6 Hz, 1H), 4.16 (dt, *J*=1.3, 6.2 Hz, 2H), 4.04 - 3.98 (m, 1H), 3.85 (br d, *J*=2.7 Hz, 1H), 3.53 (t, *J*=5.9 Hz, 2H), 3.29 (s, 3H), 2.13 - 2.02 (m, 2H), 1.05 (s, 9H).

**Step E:** 25-7 (34.73 g, 134.40 mmol) was added to a solution of 25-6 in toluene (120.00 mL) at 15°C. After the mixture was stirred at 120°C for 12 hours, additional 25-7 (9.98 g, 38.64 mmol) was added and the resulting mixture was stirred at 120°C for another 20 hours. Afterwards, trifluoroacetic acid (76.62 g, 672.00 mmol, 49.76 mL) was added to the reaction mixture, and the reaction mixture was stirred at 40°C for 3 hours. The reaction mixture was concentrated under reduced pressure at 45°C, the pH was adjusted to 9-10 with saturated sodium carbonate solution (300.00 mL), followed by extraction with ethyl acetate (200.00 mL * 2). The organic phases were combined, washed with saturated brine (200.00 mL*1), dried over anhydrous sodium sulfate, filtered and concentrated under reduced pressure at 45°C. The crude product was subjected to silica gel column chromatography (petroleum ether / ethyl acetate = 10/1 to 1/1) to give Compound 25-8.

**Step F:** A solution of 25-8 (3.88 g, 7.37 mmol) and 2,3,5,6-tetrachloro-1,4-benzoquinone (2.18 g, 8.85 mmol) in toluene (20.00 mL) and glycol dimethyl ether (20.00 mL) was heated to 70°C and stirred for 3 hours. The mixture was evaporated to dryness under reduced pressure at 45°C to remove the solvent, followed by addition of saturated aqueous sodium carbonate solution (300.00 mL) and extraction with ethyl acetate (100.00 mL *3) to remove the acidic impurities. The organic phases were combined, and 2.00 mol/L dilute hydrochloric acid (200.00 mL) was added and stirred for 1 hour. After the aqueous phase was separated, the pH value was adjusted to 10 with 2.00 mol/L sodium hydroxide solution, followed by extraction with dichloromethane (100.00 mL*3). The organic phase was washed with water (150.00 mL * 1) and saturated brine (150.00 mL*2), dried over anhydrous sodium sulfate, and concentrated under reduced pressure at 45°C to give Compound 25-9.

**Step G:** 4.00 mol/L sodium hydroxide solution (494.58 μL) was added to a solution of 25-9 (60.00 mg, 116.74 μmol) in methanol (1.00 mL) at 15°C. The mixture was stirred at 15°C for 0.5 hour. The mixture was concentrated under reduced pressure at 45°C. The pH of the crude product was adjusted to 6-7 with 1.00 mol/L hydrochloric acid solution, and then concentrated under reduced pressure at 45°C. The obtained crude product was purified by high performance liquid chromatography (hydrochloric acid conditions; column: Phenomenex Synergi C18 150*25*10 μm; mobile phase: [water (0.225% formic acid)-acetonitrile]; elution gradient: 40%-70%, 10 min) to give Compound of Embodiment 25.

ee value (enantiomeric excess): 96.654%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, $CD_3OD$) $\delta$ = 8.63 (br s, 1H), 8.44 (s, 1H), 7.86 (br s, 1H), 6.73 (br s, 1H), 4.62 (br s, 3H), 4.20 (br s, 2H), 3.64 (t, J=6.1 Hz, 2H), 3.38 (s, 3H), 2.17 - 2.04 (m, 2H), 1.38 - 0.85 (m, 9H).

**Embodiment 26**

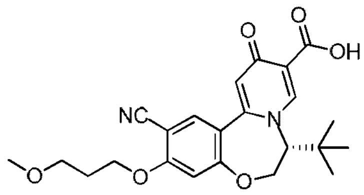

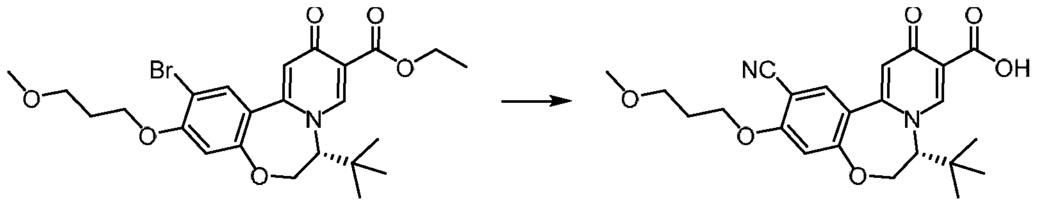

26-1 Embodiment 26

**Compound 26-1 can be prepared by the method referring to the preparation method of Compound 25-9:**

**Step A:** At 10°C, copper(I) cyanide (27.88 mg, 311.30 μmol, 68.00 μL, 2.00 eq) was added to a solution of 26-1 (80 mg, 155.65 μmol, 1.00 eq) in *N*,*N*-dimethylformamide (2.00 mL). The mixture was stirred at 140°C for 12 hours. The mixture was washed with ethyl acetate (20.00 mL) and 15% dilute aqueous ammonia. The organic phase was concentrated under reduced pressure at 45°C. The crude product was purified by high performance liquid chromatography (hydrochloric acid conditions; column: Phenomenex Synergi C18 150*25*10 μm; mobile phase: [water (0.05% hydrochloric acid)-acetonitrile]; elution gradient: 45%-65%, 7.8 min) to give Compound of Embodiment 26.

ee value (enantiomeric excess): 100%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, $CDCl_3$) $\delta$ = 9.07 - 8.18 (m, 1H), 7.74 (br s, 1H), 7.06 - 6.32 (m, 2H), 5.01 - 4.41 (m, 2H), 4.14 (br s, 3H), 3.53 (br s, 2H), 3.30 (s, 3H), 2.07 (br s, 2H), 1.30 - 0.76 (m, 9H).

**Embodiment 27**

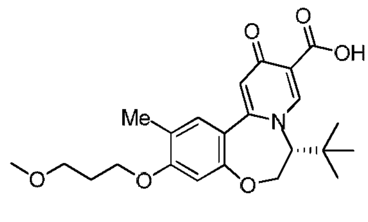

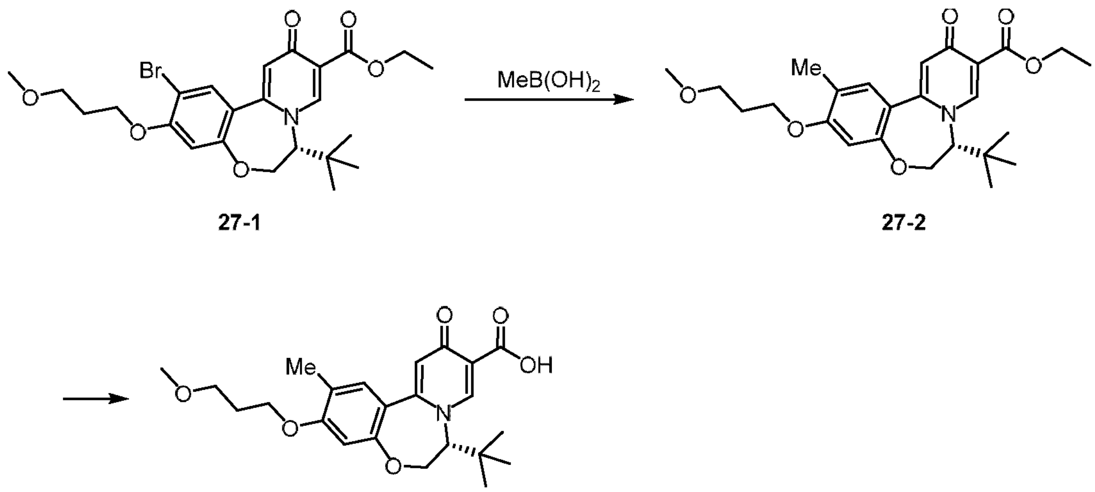


27-1

27-2

Embodiment 27

**Compound 27-1 can be prepared by the method referring to the preparation method of Compound 25-9:**

**Step A:** A suspension of 27-1 (100.00 mg, 194.56 μmol), methylboronic acid (13.98 mg, 233.47 μmol), sodium carbonate (20.62 mg, 194.56 μmol) and bis(diphenylphosphino)ferrocene]dichloropalladium dichloromethane complex (31.78 mg, 38.91 μmol) in dioxane (1.00 mL) and water (0.20 mL) was stirred at 80°C for 12 hours under nitrogen atmosphere. The mixture was filtered, and the filtrate was extracted with ethyl acetate (10.00 mL*3). The organic phase was washed with saturated brine (20.00 mL*1) and concentrated at 45°C under reduced pressure. The crude product was purified by preparative thin-layer chromatography (silica gel, dichloromethane: methanol = 15:1) to give 27-2.

**Step B:** 4.00 mol/L sodium hydroxide solution (4.00 M, 443.78 μL) was added to a solution of 27-2 (55.00 mg, 110.06 μmol) in methanol (2.00 mL) at 15°C. The mixture was stirred at 15°C for 0.5 hours. The mixture was concentrated under reduced pressure at 45°C. The pH of the crude product was adjusted to 6-7 with 1 mol/L hydrochloric acid solution, and then concentrated under reduced pressure at 45°C. The crude product was purified by high performance liquid chromatography (formic acid conditions; column: Phenomenex Synergi C18 150*25*10 μm; mobile phase: [water (0.225% hydrochloric acid)-acetonitrile]; elution gradient: 40%-70%, 10 min) to give Compound of Embodiment 27.

ee value (enantiomeric excess): 96.852%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1H$ NMR (400MHz, $CD_3OD$) $\delta$ = 8.73 (br s, 1H), 7.48 (s, 1H), 7.20 (s, 1H), 6.63 (s, 1H), 4.82 (br s, 1H), 4.70 - 4.54 (m, 2H), 4.15 (br s, 2H), 3.62 (t, *J*=6.1 Hz, 2H), 3.38 (s, 3H), 2.23 (s, 3H), 2.10 (quin, *J*=6.1 Hz, 2H), 1.22 - 0.83 (m, 9H).

**Embodiment 28**

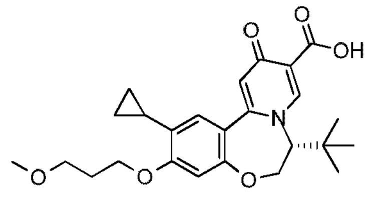

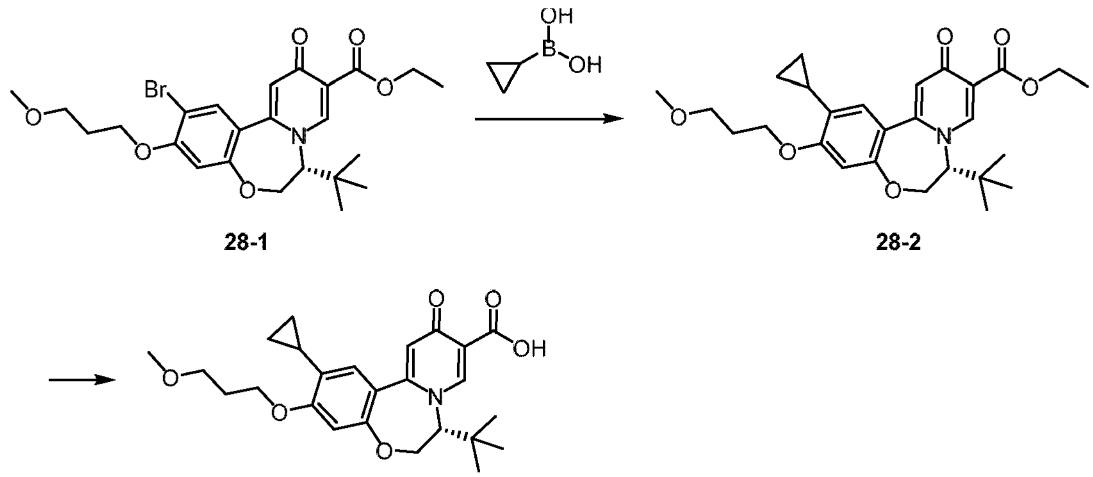

28-1

28-2

Embodiment 28

Compound 28-1 can be prepared by the method referring to the preparation method of Compound 25-9:

**Step A:** A suspension of 28-1 (100.00 mg, 194.56 μmol), cyclopropylboronic acid (33.42 mg, 389.12 μmol), potassium phosphate (123.90 mg, 583.68 μmol), palladium acetate (218.40 μg, 9.73e-1μmol) and bis(1-adamantyl)-butyl-phosphane (697.58 μg, 1.95 μmol) in toluene (2.50 mL) and water (1.00 mL) were stirred at 90°C for 12 hours under nitrogen atmosphere. The mixture was diluted with ethyl acetate (20.00 mL), and filtered through diatomite, followed by addition of 20.00 mL of water. The organic phase was separated and concentrated under reduced pressure at 45°C. The crude product was purified by preparative thin-layer chromatography (silica gel, dichloromethane: methanol = 20:1) to give 28-2.

**Step B:** 4.00 mol/L sodium hydroxide solution (4.00 M, 516.43 μL) was added to a solution of 28-2 (55.00 mg, 103.29 μmol) in methanol (2.00 mL) at 15°C. The mixture was stirred at 15°C for 0.5 hour. The pH of the mixture was adjusted to 6-7 with 1.00 mol/L hydrochloric acid solution, and the mixture was concentrated under reduced pressure at 45°C. The crude product was purified by reverse phase column chromatography (formic acid conditions; 5% acetonitrile/water to 40% acetonitrile/water) to give Compound of Embodiment 28.

ee value (enantiomeric excess): 99.202%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1H$ NMR (400MHz, $CD_3OD$) $\delta$ = 8.72 - 8.49 (m, 1H), 7.15 (s, 1H), 7.00 (br s, 1H), 6.89 - 6.47 (m, 1H), 4.62 (s, 3H), 4.16 (br s, 2H), 3.65 (t, $J$=6.2 Hz, 2H), 3.38 (s, 3H), 2.16 - 2.00 (m, 2H), 1.33 - 1.18 (m, 2H), 1.01 - 0.88 (m, 9H), 0.77 - 0.62 (m, 2H).

**Embodiment 29**

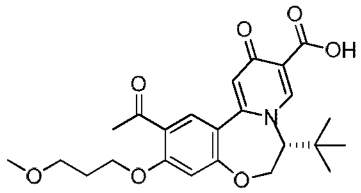

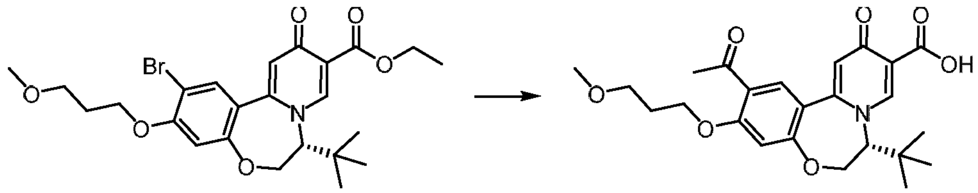

29-1 Embodiment 29

Compound 29-1 can be prepared by the method referring to the preparation method of Compound 25-9:

**Step A:** A solution of 29-1 (200.00 mg, 389.12 μmol), tributyl(1-ethoxyvinyl)stannane (0.29 g, 802.99 μmol, 271.03 μL) and bis(triphenylphosphine)palladium dichloride (10.92 mg, 15.56 μmol) in dioxane (2.00 mL) was purged with nitrogen gas three times. The mixture was stirred at 90°C for 12 hours, and concentrated under reduced pressure at 45°C to give a crude product. The crude product was purified by reverse phase column chromatography (hydrochloric acid conditions, 5% acetonitrile/water (0.05% hydrochloric acid) = 5% to 60%), and then purified by high performance liquid chromatography (basic conditions; column: Phenomenex Gemini 150*25 mm*10 μm; mobile phase: [water (0.05% ammonia)-acetonitrile]; elution gradient: 11%-38%, 12 min) to give Embodiment 29.

ee value (enantiomeric excess): 92.56%.

SFC (Supercritical Fluid Chromatography) method: Column: Chiral pak AD-3 100 mm × 4.6 mm I.D., 3 μm. Mobile phase: 5%-40% methanol (0.05% diethylamine) in carbon dioxide. Flow rate: 3 mL/min. Wavelength: 220 nm.

$^1$H NMR (400MHz, $CD_3CD$) δ = 8.59 (br s, 1H), 8.16 (s, 1H), 7.14 (br s, 1H), 6.77 (s, 1H), 5.04 - 4.95 (m, 1H), 4.76 - 4.51 (m, 2H), 4.35 - 4.22 (m, 2H), 3.63 (t, *J*=6.1 Hz, 2H), 3.38 (s, 3H), 2.64 (s, 3H), 2.17 (quin, *J*=6.1 Hz, 2H), 1.02 (s, 9H).

**Test embodiment 1: HBV *in vitro* test**

**1 Experimental objective:**

The HBV DNA content in HepG2.2.15 cells culture supernatant was determined by real-time quantitative qPCR assay (real time-qPCR), and the HBV surface antigen content was determined by enzyme linked immunosorbent assay (ELISA). The inhibitory effect of the compound on HBV was evaluated by the $EC_{50}$ value of the compound.

**2 Experimental materials:**

**2.1 Cell line: HepG2.2.15 cells**

HepG2.2.15 cell culture medium (DMEM/F12, Invitrogen-11330032; 10% serum, Invitrogen-10099141; 100 units/mL penicillin and 100 μg/mL streptomycin,Hyclone-SV30010; 1% non-essential amino acids, Invitrogen-11140050; 2 mm *L*-glutamine, Invitrogen-25030081; 300 μg/mL Geneticin, Invitrogen-10131027

**2.2 Reagents:**

Trypsin (Invitrogen-25300062)
DPBS (Corning-21031CVR)
DMSO (Sigma-D2650-100 mL)
High-throughput DNA purification kit (QIAamp 96 DNA Blood Kit, Qiagen-51162)
Quantitative Fast Start Universal Probe Reagent (FastStart Universal Probe Master, Roche-04914058001)
Hepatitis B surface antigen quantitative determination kit (Autobio Diagnostics Co., Ltd., CL 0310)

**2.3 Consumables and equipment:**

96-well cell culture plate (Corning-3599)
$CO_2$ incubator (HERA-CELL-240)
Optical sealing membrane (ABI-4311971)
Quantitative PCR 96-well plate (Applied Biosystems-4306737)
Quantitative fluorescence PCR system (Applied Biosystems-7500 real time PCR system)

**3. Experimental procedures and methods:**

3.1 HepG2.2.15 cells ($4\times10^4$ cells/well) were seeded into a 96-well plate and incubated overnight at 37°C under 5% $CO_2$.
3.2 On the 2nd day, the compound was diluted into 8 concentrations with 3-fold gradient. The compounds with different concentrations were added into the culture wells in duplicate wells. The final concentration of DMSO in the culture medium was 0.5%. 10 μM ETV was used as a 100% inhibition control, and 0.5% DMSO was used as a 0% inhibition control.
3.3 On the 5th day, the culture medium was replaced with a fresh medium containing the compound.
3.4 On the 8th day, the culture medium in the culture well was collected, and a part of the sample was taken for ELISA to determine the content of hepatitis B virus S antigen, a part of the sample was taken to extract DNA with a high-throughput DNA purification kit (Qiagen-51162).
3.5 The preparation of the PCR reaction solution was shown in Table 1:

Table 1: The preparation of the PCR reaction solution

| Item | The volume for preparing 1 hole (μL) | The volume for preparing 80 holes (μL) |
|---|---|---|
| Quantitative Fast Start Universal Probe Reagent | 12.5 | 1000 |
| Pre-primer (10 μmol) | 1 | 80 |
| Post-primer (10 μmol) | 1 | 80 |
| Probe (10 μmol) | 0.5 | 40 |

Pre-primer sequence: GTGTCTGCGGCGTTTTATCA
Post-primer sequence: GACAAACGGGCAACATACCTT
Probe sequence: 5'+ FAM + CCTCTKCATCCTGCTGCTATGCCTCATC + TAMRA -3'
3.6 15 μL of the reaction mixture was added into each well of a 96-well PCR plate, followed by addition of 10 μL of sample DNA or HBV DNA standards into each well.
3.7 PCR reaction conditions: heating at 95°C for 10 minutes; then denaturation at 95°C for 15 seconds, extension at 60°C for 1 minute, a total of 40 cycles.
3.8 Determination of hepatitis B virus S antigen content by ELISA
50 μL of sample and standard sample were taken and added into a reaction plate respectively, followed by addition of 50 μL of enzyme conjugate into each well, the mixture was shaken and well-mixed, and placed in a bath at 37°C for 60 minutes. The plate was then washed with washing solution 5 times, followed by addition of 50 μL of illuminating substrate into each well, the mixture was well-mixed and allowed to react at room temperature for 10 minutes in the dark. Chemiluminescence intensity was determined by a ELISA.
3.9 Data Analysis:

$$\text{Calculation of the percentage of inhibition: } \%\ \text{Inh.} = (1 - \text{value of sample / value of DMSO control}) \times 100$$

Calculation of $EC_{50}$: The 50% inhibitory concentration ($EC_{50}$) value of the compound on HBV was calculated by GraphPad Prism software.
4. The experimental results were shown in Table 2 and Table 3:

Table 2: HBV-DNA experimental results

| Embodiment | ECso (nM) | Embodiment | ECso (nM) |
|---|---|---|---|
| 1 | 44.68 | 16 | 11.41 |
| 2* | 8.75 | 17 | 4.77 |
| 3 | 104.7 | 18 | 34.73 |
| 4 | 11.15 | 19 | 11.73 |
| 5 | 21.46 | 20 | 44.97 |
| 6 | 2.55 | 21 | 23.20 |
| 7 | 35.31 | 22 | 11.16 |
| 8 | 22.21 | 23 | 24.07 |
| 9 | 16.23 | 24 | 0.457 |
| 10 | 3.668 | 25 | <0.457 |
| 11 | 14.25 | 26 | 2.187 |
| 12 | 19.09 | 27 | 1.103 |
| 13 | 24.07 | 28 | 0.508 |
| 14* | 23.43 | 29 | 0.507 |
| 15 | 8.66 | | |
| * reference | | | |

Conclusion: The compounds of the present disclosure are effective in inhibiting HBV-DNA *in vitro.*

Table 3: HBsAg experimental results

| Embodiment | ECso (nM) | Embodiment | ECso (nM) |
|---|---|---|---|
| 1 | 47.22 | 16 | 9.73 |
| 2* | 6.59 | 17 | 5.05 |
| 3 | 66.2 | 18 | 34.78 |
| 4 | 65.77 | 19 | 8.155 |
| 5 | 28.82 | 20 | 41.90 |
| 6 | 3.88 | 21 | 25.94 |
| 7 | 59.77 | 22 | 11.36 |
| 8 | 18.03 | 23 | 33.59 |
| 9 | 22.37 | 24 | 0.743 |
| 10 | 5.047 | 25 | 0.769 |
| 11 | 15.51 | 26 | 3.327 |
| 12 | 25.5 | 27 | 2.507 |
| 13 | 43.93 | 28 | 0.718 |
| 14* | 22.75 | 29 | 1.342 |
| 15 | 10.4 | | |
| * reference | | | |

Conclusion: The compounds of the present disclosure are effective in inhibiting hepatitis B surface antigen

(HBsAg).

**Test embodiment 2: Study on the plasma protein binding rate**

The protein binding rate of Embodiment 6 in the plasma of human, CD-1 mouse and SD rat was determined. 796 μL of blank plasma was taken from human, CD-1 mice and SD rats, and 4 μL of test compound working solution (400 μM) or warfarin working solution (400 μM) was added to achieve a final concentration of the test compound and the warfarin in plasma samples of 2 μM. The samples were mixed thoroughly. The final concentration of organic phase DMSO was 0.5%. 50 μL of the test compound and warfarin plasma sample were transferred into sample receiving plates (three parallels), and a relative volume of corresponding blank plasma or buffer was immediately added, ensuring that the final volume of each sample well was 100 μL, and the volume ratio of plasma to dialysis buffer was 1:1. 400 μL of stop solution was added to these samples, which was used as a $T_0$ sample for the determination of recovery and stability. The $T_0$ sample was stored at 2-8°C, waiting for subsequent processing with other dialyzed samples. 150 μL of test compound and warfarin plasma sample were added into the drug delivery end of each dialysis well, and 150 μL of blank dialysis buffer were added into the receiving end of the dialysis well. The dialysis plate was then sealed with a gas permeable membrane, placed in a humidified 5% $CO_2$ incubator and incubated at 37°C while shaking at about 100 rpm for 4 hours. After completion of the dialysis, 50 μL of the dialyzed buffer sample and the dialyzed plasma sample were pipetted into a new sample receiving plate. A relative volume of corresponding blank plasma or buffer was added to the sample, ensuring that the final volume of each sample well was 100 μL and the plasma to dialysis buffer volume ratio was 1:1. All samples were subjected to protein precipitation, followed by LC/MS/MS analysis. The protein binding rate and the recovery rate were calculated by the formulas: %Protein unbinding rate (%) = 100 * Drug concentration through the dialysis membrane / drug concentration not through the dialysate; Protein binding rate (%) = 100 - % protein unbinding rate; % Recovery = 100 * (Drug concentration through the dialysis membrane + Drug concentration not through the dialysate) / Total drug concentration before undialysis. Protein binding rate and recovery rate were calculated.

**Experimental results:** The protein binding rate of Embodiment 6 in the plasma of human, CD-1 mouse and SD rat was 55.7%, 50.2% and 59.4% respectively.

**Conclusion:** The compound of the present disclosure has a moderate plasma protein binding rate, and a high portion of the drug unbinds to protein, thereby exhibiting higher plasma exposure.

**Test embodiment 3: Study on the inhibition of cytochrome P450 isoenzyme**

The inhibitory effect of the test compound on different subtypes of human cytochrome P450 isoenzyme was determined. Working solutions of the test compound, standard inhibitor (100× final concentration) and a mixed substrate were prepared. Microsomes frozen in a -80°C refrigerator were thawed. 2 μL of test compound and the standard inhibitor solution were added into the corresponding wells, while 2 μL of the corresponding solvent was added into the non-inhibitor control well (NIC) and the blank control well (Blank). 20 μL of the mixed substrate solution was added into the corresponding wells except for the Blank well (20 μL of PB was added into the Blank well). Human liver microsomes solution was prepared (it was put back to the refrigerator immediately after use and the date was marked) and 158 μL of human liver microsomes solution were added into all of the wells immediately. The sample plate was pre-incubated under a 37°C water bath, during which coenzyme factor (NADPH) solution was prepared immediately. After 10 minutes, 20 μL of coenzyme factor (NADPH) solution was added into all of the wells. After the sample plate was shaken evenly, it was incubated under a 37°C water bath for 10 minutes. 400 μL of cold acetonitrile solution (the internal standard was 200 ng/mL Tolbutamide and Labetalol) was added to terminate the reaction at the corresponding time. After the sample plate was well-mixed, it was centrifuged at 4,000 rpm for 20 minutes to precipitate the protein. 200 μL of the supernatant was collected and added to 100 μL of water, and subjected to LC/MS/MS measurement after being shaken evenly.

**The experimental results** were shown in Table 4:

**Conclusion:** The test compounds have no inhibitory effect on CYP enzyme.

Table 4. Experimental results of the inhibition on cytochrome P450 isoenzyme

| Compound | $IC_{50}$ (μM) | | | | |
|---|---|---|---|---|---|
| | CYP1A2 | CYP2C9 | CYP2C19 | CYP2D6 | CYP3A4-M |
| Embodiment 6 | >50 | >50 | >50 | >50 | >50 |

**Test embodiment 4: Microsomal metabolism stability**

**Experimental objective:** The microsomal metabolism stability of the test compound (Embodiment 6) in three

species was determined.

**Experimental procedures:** 1 μM test compound and microsomes (0.5 mg/mL) supplemented with NADPH regeneration system were incubated at 37°C. The positive controls were testosterone (3A4 substrate), propafenone (2D6 substrate) and diclofenac (2C9 substrate), respectively. The positive control and microsomes (0.5 mg/mL) supplemented with NADPH regeneration system were incubated at 37°C. Samples at different time points (0, 5, 10, 20, 30, and 60 minutes) were mixed directly with cold acetonitrile containing an internal standard to terminate the reaction. The compound and microsomes were incubated for 60 minutes without the NADPH regeneration system. One parallel was set at each time point (n = 1). The samples were analyzed by LC/MS/MS. The concentration of the compound was indicated by the ratio of the peak area of the analyte to the peak area of the internal standard.

**Experimental results:** The remaining ratio of the compound of the present disclosure in rat, human and mouse liver microsomes at T=60 min was: 113.0%, 109.1% and 102.5%, respectively.

**Experimental conclusion:** The compound of the present disclosure has good stability in all of three species: rats, humans and mice.

**Test embodiment 5: single-dose pharmacokinetic study on Mouse/Rat**

**Experimental objective:** Male C57BL/6 mice or SD rats were used as test animals, and the drug concentrations of the compound in the plasma, liver and cerebrospinal fluid were determined after single-dose administration and the pharmacokinetic behavior was evaluated.

**Experimental procedures:** Healthy adult male C57BL/6 mice or SD rats were selected and intragastrically administrated. The candidate compound was mixed with an appropriate amount of 5% DMSO/95% (10% hydroxypropyl-β-cyclodextrin), vortexed and sonicated to prepare a 0.2 mg/mL clear solution for use. After the mice were orally administered at the dose of 2 mg/kg, the whole blood was collected for a certain period of time to prepare the plasma, and liver and cerebrospinal fluid were collected. After pretreatment of the sample, the drug concentration was analyzed by LC-MS/MS method. Phoenix WinNonlin software was used to calculate the pharmacokinetic parameters.

**Experimental results:** Table 5.

**Experimental conclusion:** The test compound of Embodiment 5 has good $AUC_{0\text{-}last}$ and bioavailability in both mouse and rat species.

Table 5: Experimental results of the pharmacokinetic of test compound on mouse and rat

| Embodiment 6 (IV: lmg/kg PO: 2 mg/kg) | Mouse | Rat |
|---|---|---|
| Clearance rate (mL/min/kg) | 75.0 | 28.1 |
| Apparent distribution volume (L/kg) | 5.79 | 1.72 |
| $AUC_{0\text{-}last}$ (Intravenous injection, nM. hr) | 502 | 1365 |
| $AUC_{0\text{-}last}$ (Oral, nM. hr) | 876 | 1832 |
| Half-life (h) | 2.46 | 1.98 |
| Highest concentration (nM) | 1055 | 659 |
| Bioavailability (%) | 91.6 | 71.1 |

**Test embodiment 6: *In vivo* pharmacodynamic study of hydrodynamic injection mouse HBV model (HDI-HBV) via tail vein**

**Experimental objective:** Anti-hepatitis B virus activity of the compound of the embodiment *in vivo* was evaluated by HDI mice model.

**Experimental materials:** Balb/c mice, 10% HP-β-CD as vehicle, test compound, RG7834, ETV (Entecavir), pAAV2-HBV1.3mer plasmid (extracted with Qiagen EndoFree Plasmid Giga kit), main reagents including QIAamp96 DNA kit and FasStart Universal Probe Mast (ROX). The main instruments used in this experiment included centrifuge (Beckman Allegra X-15R), tissue grinder (QIAGEN-Tissue lyser II) and spectrophotometer (Thermo-NANODROP 1000).

**Experimental methods:**

a) The experimental design was shown in Table 6 below:

**Table 6: *In vivo* experimental design**

| Group | Number of animals | Compound | Dose (mg/kg) | Dosing volume (mL/kg) | Dosage regimen | Blood collection time |
|---|---|---|---|---|---|---|
| 1 | 5 | Blank | / | 10 | Gavage, from the 1st day to the 7th day, once a day | 4 hours after administration on the 1st day, the 3rd day, the 5th day and the 7th day |
| 2 | | RG7834 | 30 | | | |
| 3 | | Embodiment 6 | 30 | | | |
| 4 | | Entecavir | 0.1 | | | |

b) On day 0, all mice were subjected to hydrodynamic injection with HBV plasmid DNA solution via tail vein. The plasmid DNA was pre-treated with sterile physiological saline before injection and stored at 4°C until use. Plasmid DNA solution was injected via the tail vein at a dose of 8% of the body weight of the mouse within 5 seconds.

c) On the 1st day to the 7th day, the mice were administered with the compound or solvent by oral gavage for 7 days. The specific administration methods were shown in Table 15-16.

d) On the 1st day, 3rd day and 5th day, the blood was collected from the submandibular vein of the mice, and heparin sodium was used as an anticoagulant. The blood sample was centrifuged at 7,000×g,4°C to prepare the plasma for the determination of HBV DNA.

e) On the 7th day, all mice were euthanized by $CO_2$. The blood was collected from the heart to prepare plasma, and the liver tissue was collected. Two left hepatic lobe were isolated, the size of which was 70-100 mg, and were frozen by liquid nitrogen immediately after collection. One of the hepatic lobe was used for HBV DNA detection, and the other was used for backup.

f) All of the plasma samples and the liver samples were stored in a -80°C refrigerator before being sent for analysis.

**Sample treatment:** The content of HBsAg in the serum of the mice was determined by ELISA (enzyme linked immunosorbent assay). The experimental procedure refers to the specification of HBsAg ELISA kit.

**Experimental results:** The inhibitory activity of the test compound on HBV replication on the mouse HDI model was determined by measuring the content of HBsAg in the plasma of the mice. The content of HBsAg in the plasma of the mice at different dosing time was shown in Table 7 and Figure 1.

Table 7: HBsAg content in the plasma of the mice after dosing at different days

| Group | Dosing time | Log HBsAg (IU/mL) (Ave. value) |
|---|---|---|
| 1 Blank QD | 1 | 3.57 |
| | 3 | 4.30 |
| | 5 | 4.20 |
| | 7 | 2.22 |
| 2 RG7834 30 mg/kg QD | 1 | 3.75 |
| | 3 | 4.08 |
| | 5 | 3.59 |
| | 7 | 1.10 |
| 3 Embodiment 6 30mg/kg QD | 1 | 3.86 |
| | 3 | 4.09 |
| | 5 | 3.27 |
| | 7 | 1.07 |

(continued)

| Group | Dosing time | Log HBsAg (IU/mL) (Ave. value) |
|---|---|---|
| 4 Entecavir 0.1mg/kg QD | 1 | 3.66 |
| | 3 | 4.32 |
| | 5 | 4.01 |
| | 7 | 2.46 |

**Conclusion:**

From the data of HBsAg content on the $5^{th}$ day, the compound of Embodiment 6 shows a better effect in lowering the surface antigen than RG7834 and Entecavir at the same dose, exhibiting a better efficacy.

**Test embodiment 7: Anti-HBV Activity on the hepatitis B virus mouse model (AAV-HBV) mediated by recombinant adeno-associated type 8 virus vectors**

**Experimental objective:**

The AAV vector-mediated HBV infected mouse model is a fast and efficient HBV model. Using the high hepatotropism of the AAV vector, the recombinant adeno-associated type 8 virus carrying 1.3 copies of the HBV genome (rAAV8-1.3HBV) is injected into the tail vein of mice, which can efficiently introduce the carried 1.3 copy HBV genome into hepatocytes. Due to the characteristics of the AAV viral vector, the vector mediated by it can express for a long time. The AAV/HBV model can continuously replicate HBV DNA and express HBsAg and HBeAg in the liver of the mice.
The anti-HBV efficacy of the test compound *in vivo* was evaluated by determining the content of HBsAg in the serum of the mice after treatment with the test compound on AAV/HBV mouse mode.

**Experimental materials:**

C57BL/6 mice, 10% HP-β-CD as vehicle, reference compound TDF (Tenofovir), test compound, recombinant virus rAAV8-1.3HBV, main reagents of the experiment including QIAamp96 DNA kit and TaqMan® Universal PCR Master Mix, Hepatitis B Virus Surface Antigen Detection Kit, instruments including: centrifuge (Beckman Allegra X-15R), tissue grinder (QIAGEN-Tissue lyser II) and spectrophotometer (Thermo-NANODROP 1000).

**Experimental procedures:**

a) All mice were orally administered on the $28^{th}$ day after the virus injection, and the day was set as day 0. Submaxillary blood of all mice was collected for serum collection before administration. The mice were administered once a day for four weeks. The specific dosage regimen was shown in Table 8.
b) Submaxillary blood of all mice was collected for serum collection twice a week, the volume of the blood collected each time was approximately 100 μL. The specific blood collection time was shown in Table 8.
c) On the $28^{th}$ day, all mice were euthanized and the blood was collected from the heart for serum collection.
d) All serum samples were sent for analysis.

**Table 8: *In vivo* experimental scheme**

| Group | No. of the mice | Dosing design | | | | Serum collection regimen |
|---|---|---|---|---|---|---|
| | | Compound | Dose (mg/kg) | Dosing volume (mL/kg) | Dosage regimen | |
| 1 | 5 | Vehicle | / | 10 | The 28th day after virus injection was set as day 0. Administration once a day for four weeks, that is, each administration period was 0-the | The 28th day after virus injection was set as day 0. The blood was collected twice a week, and the volume of the blood collected each time was |
| 2 | 5 | Tenofovir | 1 | | | |
| 3 | 5 | RG7834 | 10 | | | |
| 4 | 5 | Embodiment 6 | 3 | | | |
| 5 | 5 | Embodiment 6 | 10 | | | |
| 6 | 5 | Embodiment 6 | 30 | | | |
| 7 | 5 | Embodiment 6 + TDF | (10 mg/kg + 1 mg/kg) | | 27th day. | about 100 $\mu$L. The blood was collected on the 3rd day, the 7th day, the 10th day, the 14th day, the 17th day, the 21st, the 24th day, the 28th day. |

**Sample analysis:**

The content of HBsAg in the serum of the mice was determined by ELISA. The experimental procedure refers to the specification of HBsAg ELISA kit.

**Experimental results:**

a) The anti-HBV activity of the test compound on AAV/HBV mice model was evaluated by determining the content of HBsAg in the plasma of the mice. The results were shown in Table 9 and Figure 2.

Table 9: HBsAg content in the plasma of the mice after administration at different days (IU/mL)

| Date (day) | Blank (PO,QD) | TDF (1 mg/kg, PO,QD) | RG783 4 (10mg/ kg, PO) | Embodim ent 6 (3mg/kg, PO) | Embodimen t 6 (10mg/kg, PO) | Embodimen t 6 (30mg/kg, PO) | Embodiment 6 (10mg/kg, PO) + TDF (1 mg/kg, PO,QD) |
|---|---|---|---|---|---|---|---|
| -1 | 4.54 | 4.59 | 4.56 | 4.54 | 4.51 | 4.48 | 4.48 |
| 4 | 4.27 | 4.56 | 3.54 | 3.68 | 3.56 | 3.38 | 3.62 |
| 7 | 4.46 | 4.59 | 3.58 | 3.70 | 3.69 | 3.44 | 3.76 |
| 11 | 4.52 | 4.66 | 3.70 | 3.76 | 3.92 | 3.64 | 3.82 |
| 14 | 4.41 | 4.50 | 3.49 | 3.70 | 3.61 | 3.40 | 3.59 |
| 18 | 4.56 | 4.61 | 3.70 | 3.85 | 3.90 | 3.55 | 3.61 |
| 21 | 4.58 | 4.52 | 3.52 | 3.78 | 3.75 | 3.46 | 3.58 |
| 25 | 4.52 | 4.33 | 3.50 | 3.76 | 3.83 | 3.37 | 3.50 |
| 28 | 4.34 | 4.34 | 3.66 | 3.96 | 3.87 | 3.53 | 3.76 |

b) The changes in body weight of mice were shown in Figure 3.

**Experimental conclusion:**

In this experiment, the test compound Embodiment 6 was able to significantly reduce the content of HBsAg on the AAV/HBV mouse model, and exhibited a certain dose-effect relationship. During the course of treatment with Embodiment 6, the mice showed good tolerance and the changes of the body weight were better than RG7834.

**Test embodiment 8: 14-day pre-toxicology tolerance test on rat**

In this experiment, the test compound RG7834 and Embodiment 6 were intragastrically administered once a day for 14 consecutive days to test the potential toxicity. The experimental design was shown in Table 10.

**Table 10: Experimental design group and dosage**

| Group | No. of animals (gender) | Test compound | Dose (mg/kg) | Volume (mL/kg) | Concentration (mg/mL) | Solvent |
|---|---|---|---|---|---|---|
| 1 | 3 (male) | Solvent | 0 | 10 | 0 | 0.5% hydroxypropyl methylcellulos e / 0.2% Tween 80 dissolved in water, pH 8-9 |
| 2 | 3 (male) | RG7834 | 100 | 10 | 10 | |
| 3 | 3 (male) | RG7834 | 300 | 10 | 30 | |
| 4 | 3 (male) | RG7834 | 1000 | 10 | 100 | |
| 5 | 3 (male) | Embodiment 6 | 100 | 10 | 10 | |
| 6 | 3 (male) | Embodiment 6 | 300 | 10 | 30 | |
| 7 | 3 (male) | Embodiment 6 | 1000 | 10 | 100 | |

A total of 21 animals in this experiment were randomly divided into 7 groups. The animals were orally administered with the test compounds once a day for 14 consecutive days, and the toxicity was evaluated. Animal necropsy and tissue would be taken from the fixative, repaired, dehydrated, embedded, sectioned, stained and microscopically examined. These tissues included the liver, heart and lung of all animals, and spleen, stomach, duodenum, jejunum, ileum, and kidney of partial animals.

**Experimental results:**

RG7834: Only the heart, liver and lung were examined in the medium and low dose groups, and no histopathological changes associated with the test compound were observed. The high dose group showed that the histopathological changes associated with the test compoundappeared as mild myocardial degeneration in the heart, mild degeneration of central hepatic cells in the small lobules of the liver, mild decrease of white pulp lymphocytes in the spleen, and moderate acute inflammation of the duodenal mucosa.

Embodiment 6: Only the heart, liver, and lung were examined in the medium and low dose groups, and no histopathological changes associated with the test compound were observed. The high dose group showed that the histopathological changes associated with the test compoundappeared as little or mild decrease of white pulp lymphocytes in the spleen.

**Experimental conclusion:** It can be seen from the experimental results that Embodiment 6 has higher safety than RG7834.

**Test embodiment 9: Single dose neurotoxicity test on rats**

**Experimental objective:** In this experiment, SD rats were intragastrically administrated with RG7834 and Embodiment 6 in a single dose, and the potential neurobehavioral toxicities to SD rats were evaluated by functional observation combination test (FOB).

**Experimental materials and procedures:** A total of 25 SD male rats were used in this experiment. At the start of the administration, the animals were about 7 to 8 weeks old, males weighed between 225.50 and 285.70 g, and females weighed between 177.68 and 219.89 g. The animals in the test compound group were intragastrically administered with 300 or 1000 mg/kg of RG7834 and 300 or 1000 mg/kg of Embodiment 6 dissolved in the menstruum: 0.5% (w/v) hydroxypropyl methylcellulose / 0.2% (v/v) Tween 80 purified aqueous solution (pH 8.0-9.0). The animals in the control group were administered with the menstruum. All animals were administered at a volume of 10 mL/kg, and subjected to the FOB test.

**Experimental results:** SD rats were intragastrically administered with RG7834 and Embodiment 6 at a single dose of 0, 300, 1000 mg/kg. 24 hours after administration, a magnification of the pupil associated with the test compound appeared on the male animals administrated with 1000 mg/kg and 300 mg/kg of RG7834A. A magnification of the pupil associated with the test compound did not appear on the male animals administrated with 1000 mg/kg and 300 mg/kg of Embodiment 6.

**Experimental conclusion:** RG7834 has a certain neurotoxic effect at high dose. In contrast, the compound of the present disclosure has more excellent safety.

## Claims

1. A compound of formula (I):

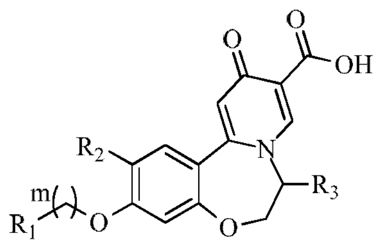

(I)

,

or a compound selected from the group consisting of:

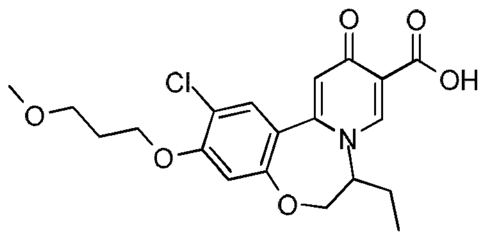

,

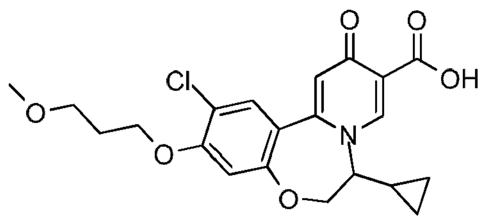

,

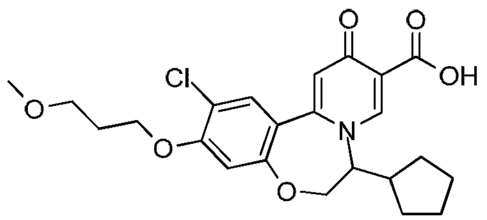

,

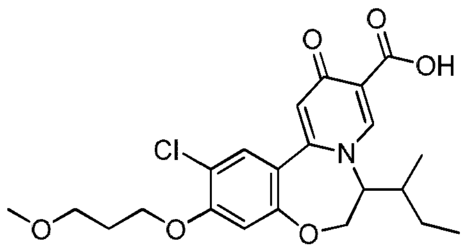

,

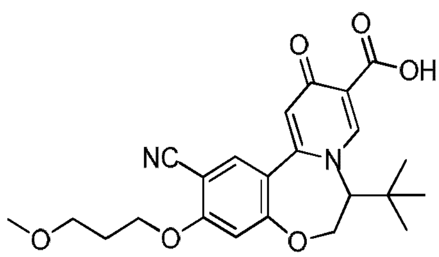

,

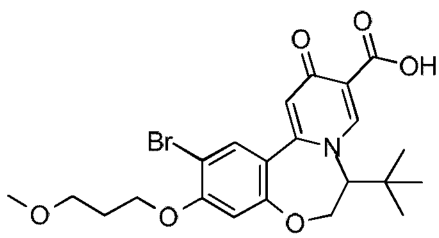

,

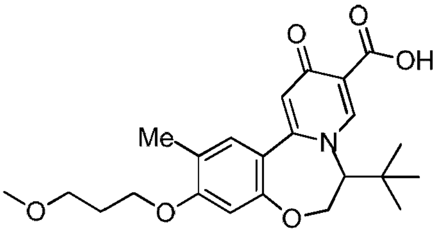,
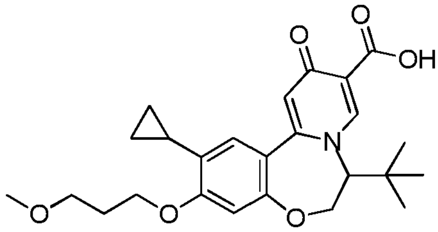,

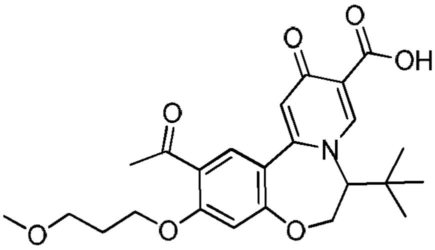;
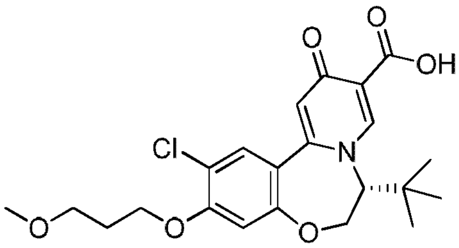

and

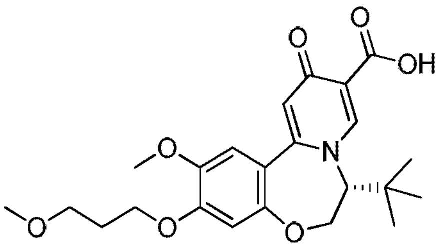;

or a pharmaceutically acceptable salt of the compound;
wherein, in the formula (I):
$R_1$ is OH, CN, $NH_2$,

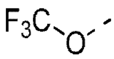,

or selected from the group consisting of

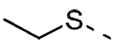, 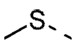,

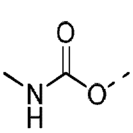, 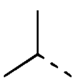, 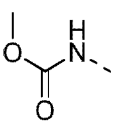, 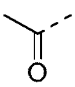, 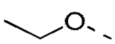, 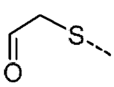, 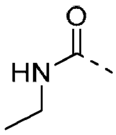,

$C_{2-5}$ alkenyl, $C_{2-5}$ heteroalkenyl, $C_{3-6}$ cycloalkyl and 3-6 membered heterocycloalkyl, wherein each of the

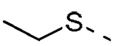,

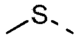, 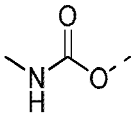, 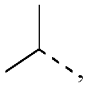, 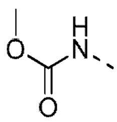, 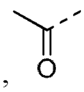, 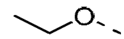, 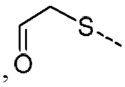, 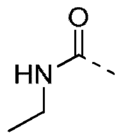,

$C_{2-5}$ alkenyl, $C_{2-5}$ heteroalkenyl, $C_{3-6}$ cycloalkyl and 3-6 membered heterocycloalkyl is optionally substituted by 1, 2 or 3 R;

m is 0, 1, 2, 3, 4 or 5; and
$R_1$ is not OH, CN, $NH_2$ provided m is 0;
or,

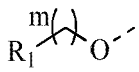 is 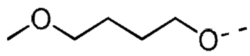;

$R_2$ is H, OH, CN, $NH_2$, halogen, or selected from the group consisting of $C_{1-3}$ alkyl, $C_{1-3}$ heteroalkyl, $C_{3-6}$ cycloalkyl and 3-6 membered heterocycloalkyl, wherein each of the $C_{1-3}$ alkyl, $C_{1-3}$ heteroalkyl, $C_{3-6}$ cycloalkyl and 3-6 membered heterocycloalkyl is optionally substituted by 1, 2 or 3 R;
$R_3$ is selected from the group consisting of $C_{1-6}$ alkyl and $C_{3-6}$ cycloalkyl, wherein each of the $C_{1-6}$ alkyl and $C_{3-6}$ cycloalkyl is optionally substituted by 1, 2 or 3 R;
R is H, halogen, OH, CN, $NH_2$, or selected from the group consisting of $C_{1-3}$ alkyl and $C_{1-3}$ heteroalkyl, wherein each of the $C_{1-3}$ alkyl and $C_{1-3}$ heteroalkyl is optionally substituted by 1, 2 or 3 R';
R' is selected from the group consisting of F, Cl, Br, I, OH, CN, $NH_2$, $CH_3$, $CH_3CH_2$, $CH_3O$, $CF_3$, $CHF_2$ and $CH_2F$;
the "hetero" refers to heteroatom or heteroatomic group; the "hetero" in the $C_{2-5}$ heteroalkenyl, 3-6 membered heterocycloalkyl, $C_{1-3}$ heteroalkyl is independently selected from the group consisting of -C(=O)N(R)-, -N(R)-, -C(=NR)-, -(R)C=N-, $-S(=O)_2N(R)-$, -S(=O)N(R)-, N, -O-, -S-, =O, =S, - C(=O)O-, -C(=O)-, -C(=S)-, -S(=O)-, $-S(=O)_2-$ and -N(R)C(=O)N(R)-;
in any of the above cases, the number of the heteroatom or the heteroatomic group is independently 1, 2 or 3.

2. The compound, or the pharmaceutically acceptable salt according to claim 1, wherein, R is H, F, Cl, Br, I, OH, CN, $NH_2$, $CH_3$, $CH_3CH_2$, $CH_3O$, $CF_3$, $CHF_2$ or $CH_2F$.

3. The compound, or the pharmaceutically acceptable salt according to claim 1 or 2, wherein, $R_1$ is OH, CN, $NH_2$, or selected from the group consisting of $C_{2-3}$ alkenyl, $C_{2-3}$ heteroalkenyl, $C_{3-6}$ cycloalkyl and 3-6 membered heterocycloalkyl, wherein each of the $C_{2-3}$ alkenyl, $C_{2-3}$ heteroalkenyl, $C_{3-6}$ cycloalkyl and 3-6 membered heterocycloalkyl is optionally substituted by 1, 2 or 3 R.

4. The compound, or the pharmaceutically acceptable salt according to claim 1 or 2, wherein, $R_1$ is OH, CN, $NH_2$, or selected from the group consisting of

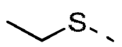, 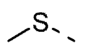, 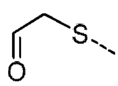,

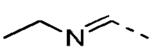, 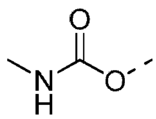, 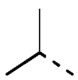, 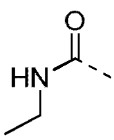, 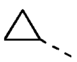, 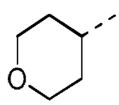, 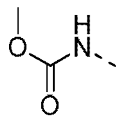, 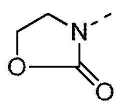,

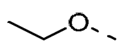 and 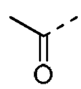,

wherein each of the

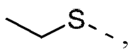, 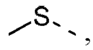, 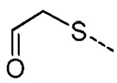, 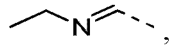,

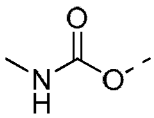, 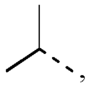, 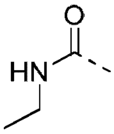, 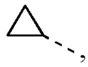, 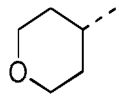, 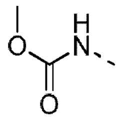, 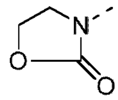, 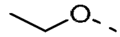

and

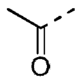

is optionally substituted by 1, 2 or 3 R.

**5.** The compound, or the pharmaceutically acceptable salt according to claim 1 or 2, wherein, $R_1$ is selected from the group consisting of OH, CN, $NH_2$,

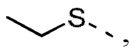, 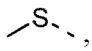, 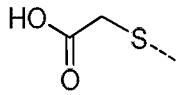,

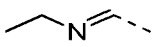, 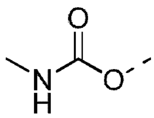, 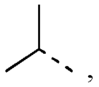, 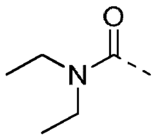, 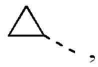, 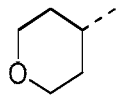, 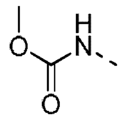,

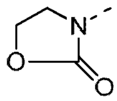, 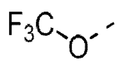, 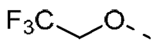 and 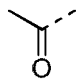.

**6.** The compound, or the pharmaceutically acceptable salt according to claim 1 or 2, wherein, $R_2$ is H, OH, CN, $NH_2$, halogen, or selected from the group consisting of $C_{1-3}$ alkyl, $C_{1-3}$ heteroalkyl and $C_{3-6}$ cycloalkyl, wherein each of the $C_{1-3}$ alkyl, $C_{1-3}$ heteroalkyl and $C_{3-6}$ cycloalkyl is optionally substituted by 1, 2 or 3 R, optionally, $R_2$ is H, OH, CN, $NH_2$, F, Cl, Br, I, or selected from the group consisting of $CH_3$,

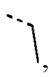,

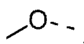, 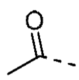 and 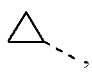,

wherein each of the

$CH_3$, 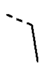, 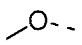, 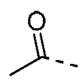 and 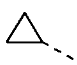 is

optionally substituted by 1, 2 or 3 R, optionally thereto, $R_2$ is selected from the group consisting of Cl, Br, CN, $CH_3$,

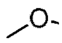, 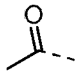

and

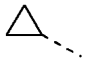

7. The compound, or the pharmaceutically acceptable salt according to claim 1 or 2, wherein, $R_3$ is selected from the group consisting of $C_{1-4}$ alkyl and $C_{3-6}$ cycloalkyl, wherein each of the $C_{1-4}$ alkyl and $C_{3-6}$ cycloalkyl is optionally substituted by 1, 2 or 3 R, optionally, $R_3$ is selected from the group consisting of

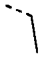, 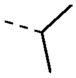, 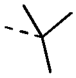, 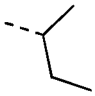, 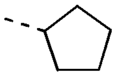

and

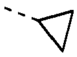.

8. The compound, or the pharmaceutically acceptable salt according to claim 1 or 2, wherein, m is 0, 1, 2, 3 or 4; and $R_1$ is not OH, CN, $NH_2$ provided m is 0.

9. The compound, or the pharmaceutically acceptable salt according to claim 1 or 2, wherein, the moiety

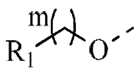

is selected from the group consisting of

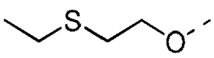, 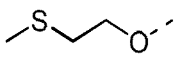,

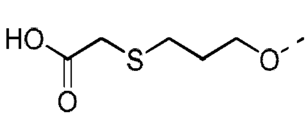, 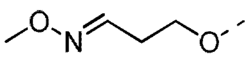, 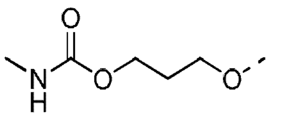,

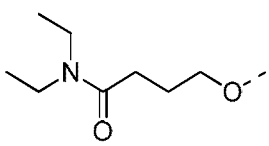, 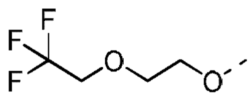, 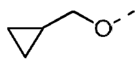, 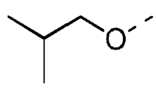, 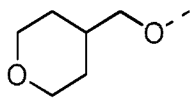,

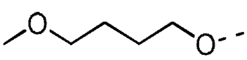, 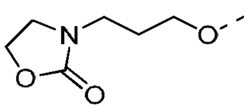, 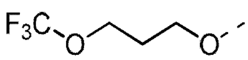, 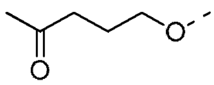

and

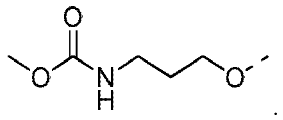

10. A compound of formula (II), (III) or (IV), or a pharmaceutically acceptable salt of the compound:

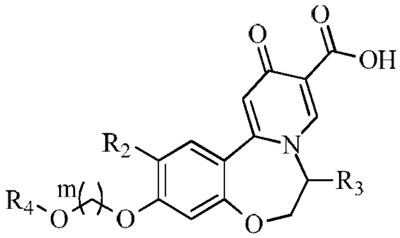

(II)

,

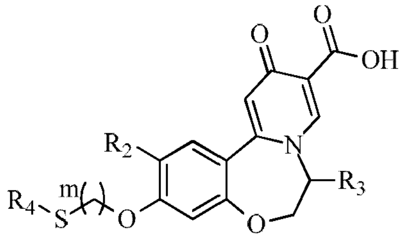

(III)

and

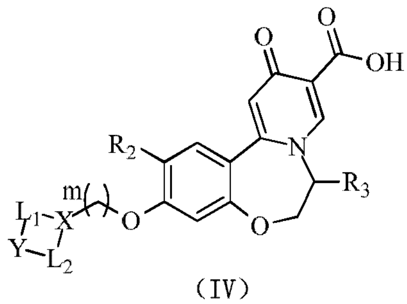

(IV)

,

wherein,

$R_4$ is H, or selected from the group consisting of $C_3$ alkyl and $C_{1-3}$ heteroalkyl, wherein each of the $C_3$ alkyl and $C_{1-3}$ heteroalkyl is optionally substituted by 1, 2 or 3 R;
X is selected from the group consisting of C and N;
Y is selected from the group consisting of O and C;
each of $L_1$ and $L_2$ is independently selected from the group consisting of a single bond, $-(CH_2)_n-$ and $-C(=O)-$; with the provision that $L_1$ and $L_2$ are not both a single bond;
n is 1 or 2;
m, R, $R_2$, $R_3$ and the "hetero" in $C_{1-3}$ heteroalkyl are as defined in claims 1-8; and $R_4$ is not H provided m is 0.

11. The compound, or the pharmaceutically acceptable salt according to claim 1, wherein, the compound is selected from the group consisting of:

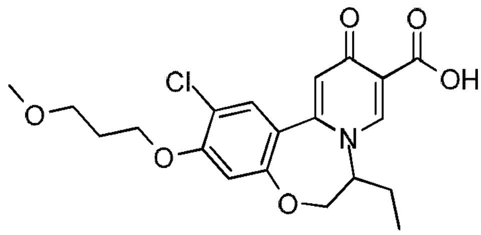

,

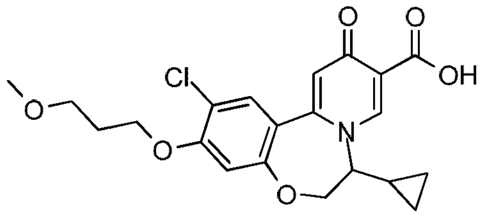,

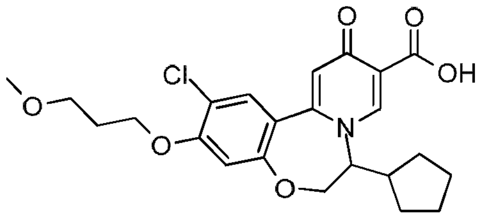,

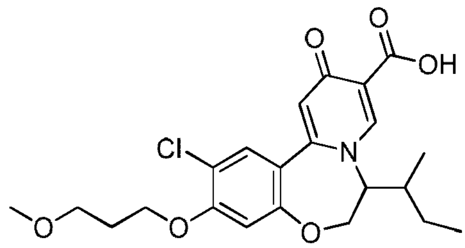,

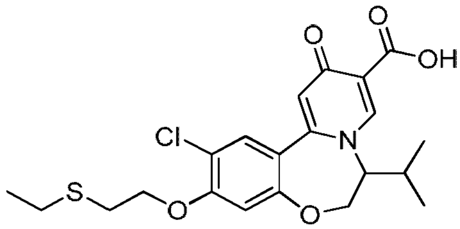,

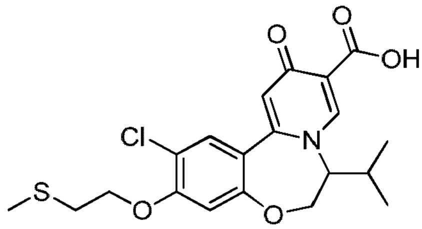,

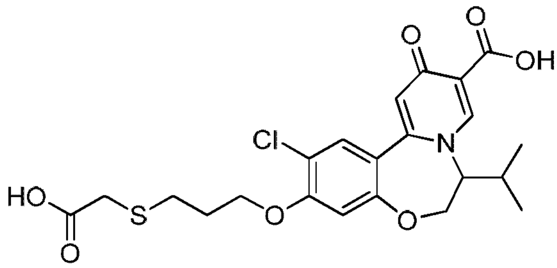,

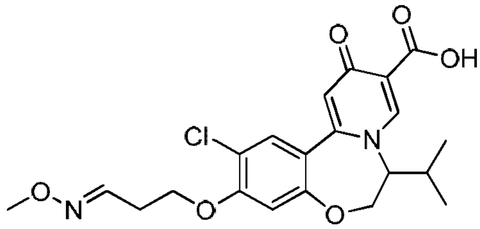,

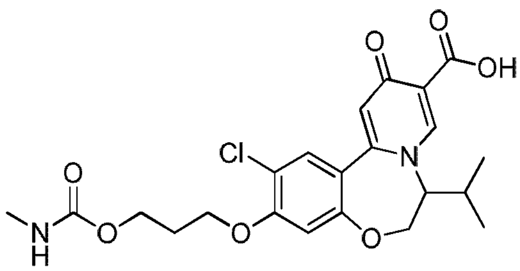,

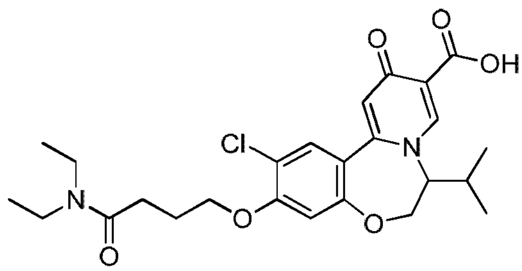,

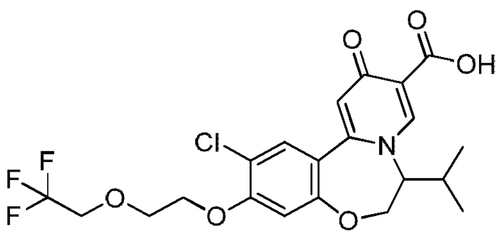,

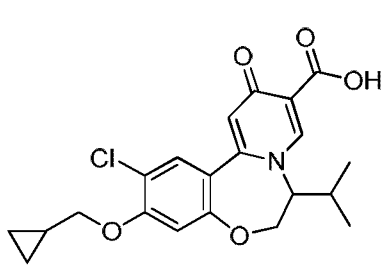
,
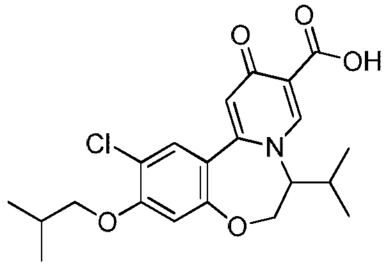
,

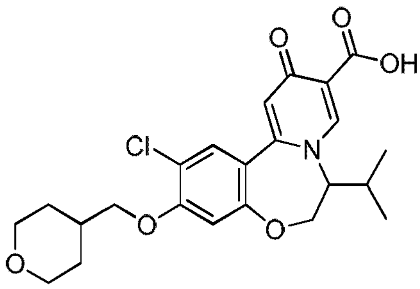
,

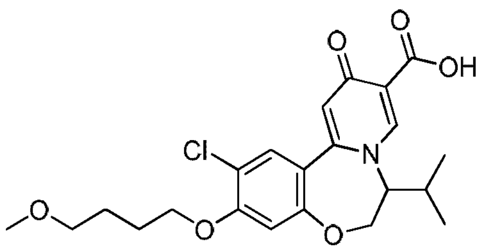
,
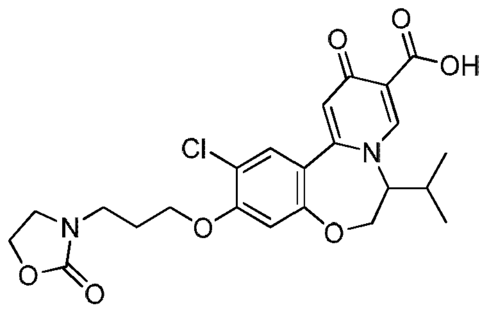
,

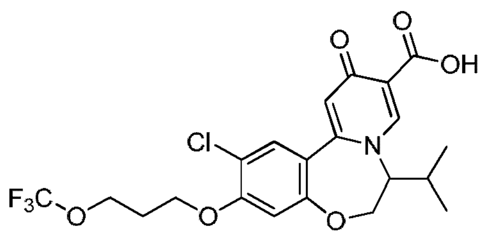
,
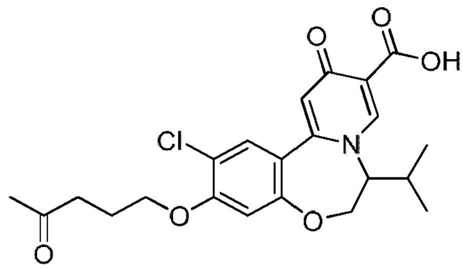
,

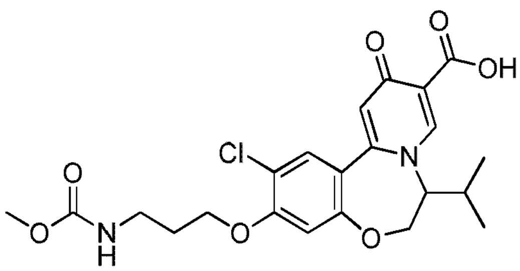
,

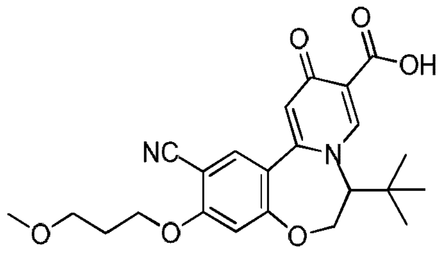
,
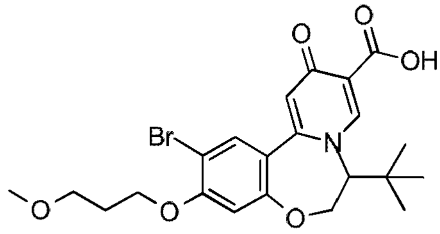
,

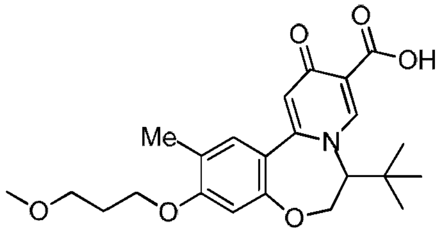
,
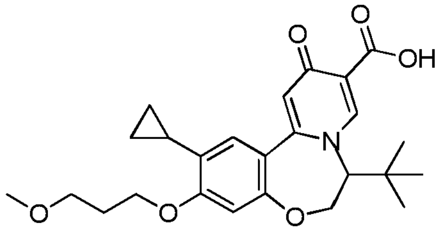
,

and

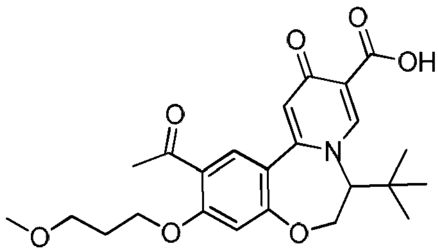
.

**12.** The compound, or the pharmaceutically acceptable salt according to claim 1, wherein the compound is selected from the group consisting of:

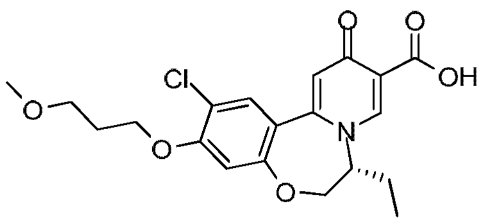
,

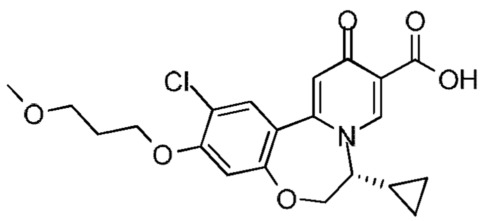
,
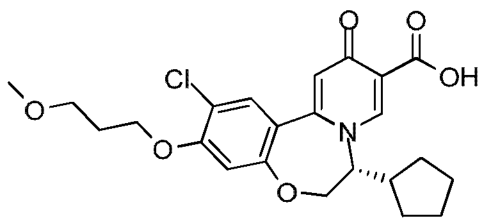
,

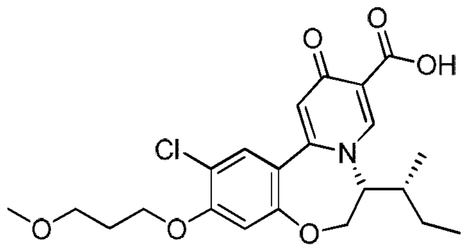,
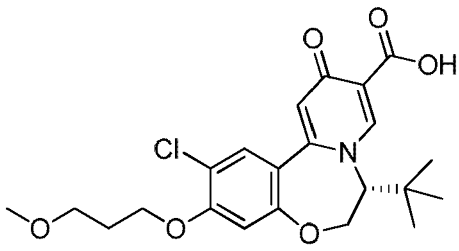,

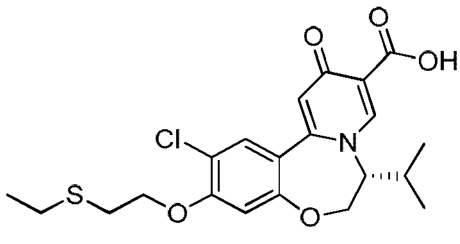,
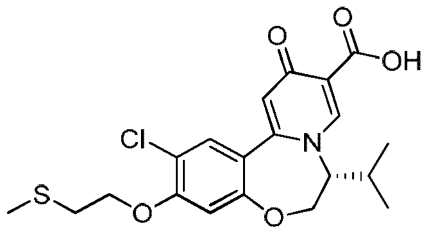,

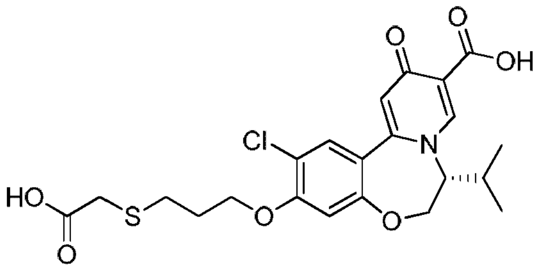,
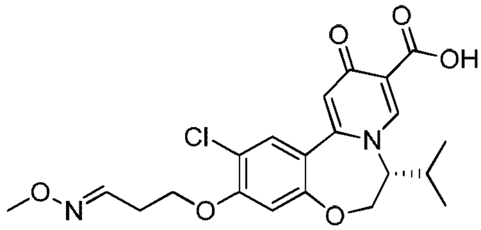,

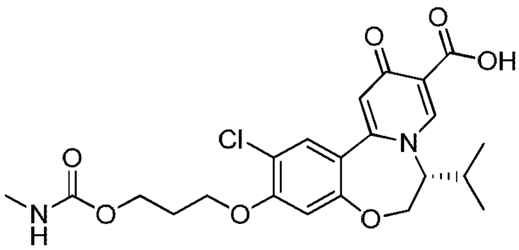,
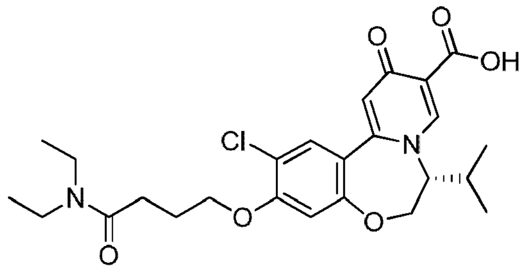,

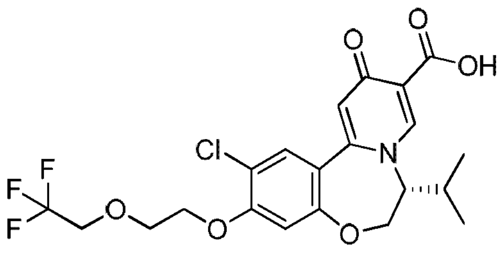,

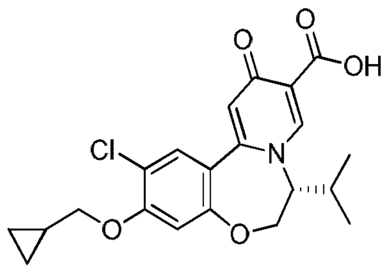,
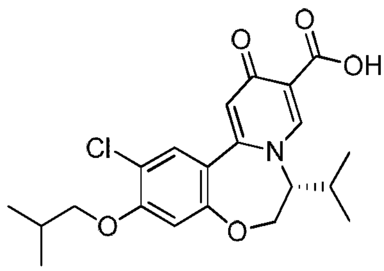,

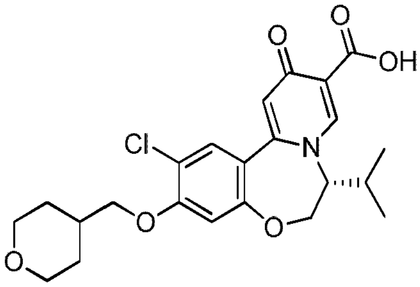

,

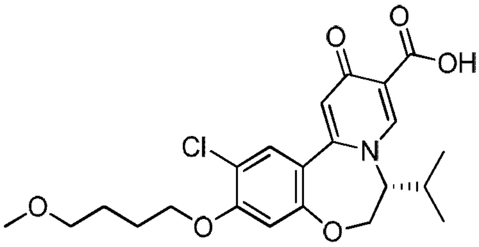

,

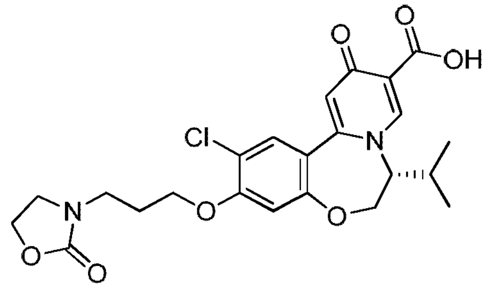

,

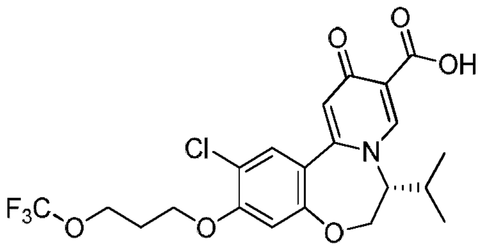

,

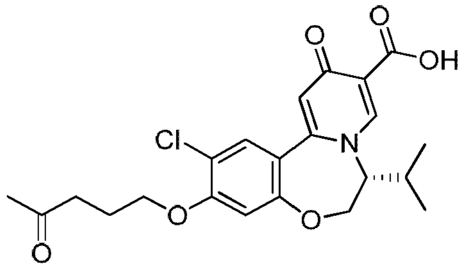

,

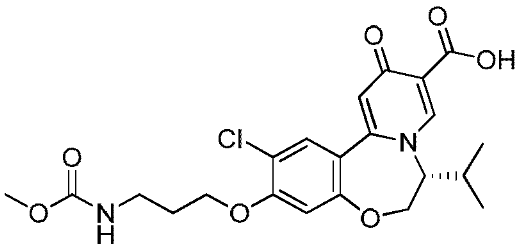

,

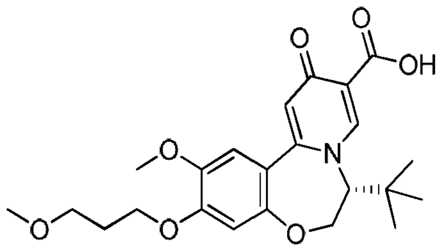

,

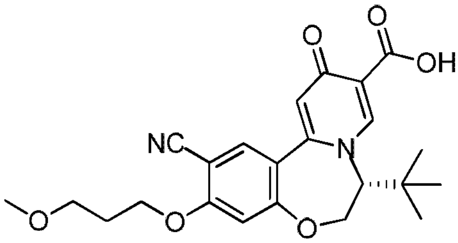

,

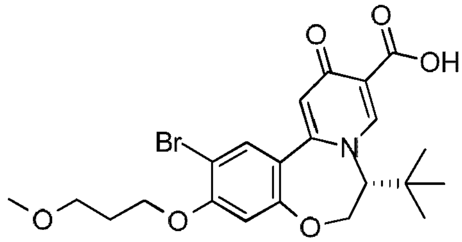

,

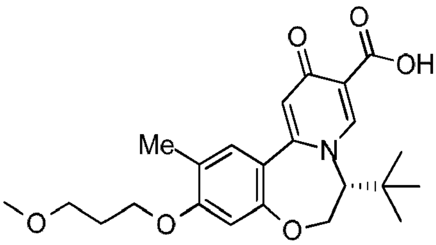,
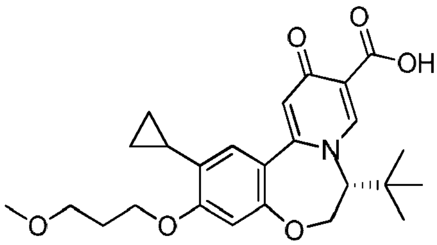

and

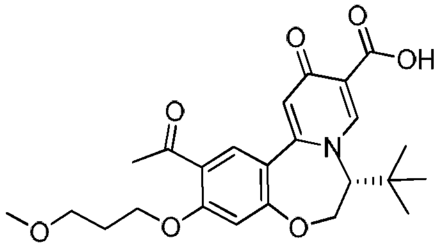.

13. A pharmaceutical composition comprising a therapeutically effective amount of the compound, or the pharmaceutically acceptable salt according to any one of claims 1-12, and a pharmaceutically acceptable carrier.

14. The compound, or the pharmaceutically acceptable salt according to any one of claims 1-12 or the pharmaceutical composition according to claim 13 for use in treating hepatitis B.

**Patentansprüche**

1. Verbindung der Formel (I):

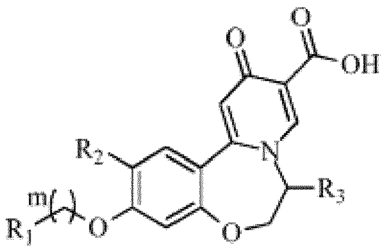

(I)

,

oder Verbindung, die aus der aus den folgenden bestehenden Gruppe ausgewählt ist:

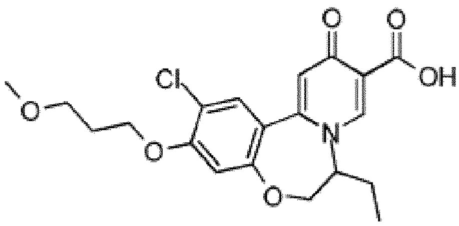,
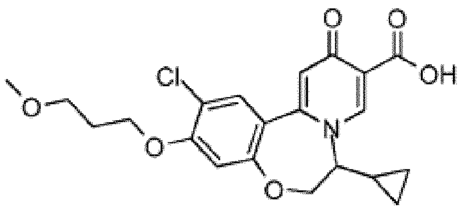,

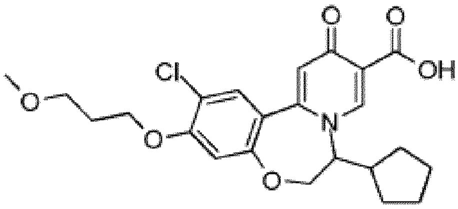
,
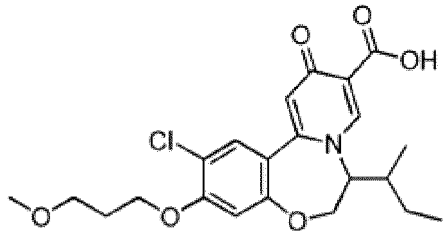
,

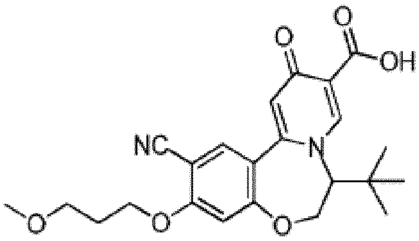
,
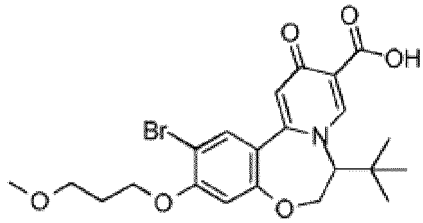
,

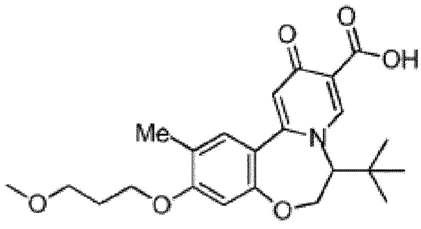
,
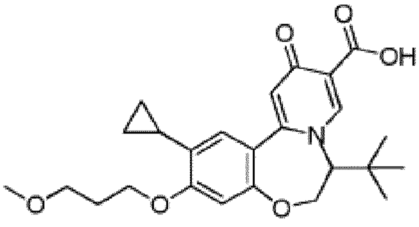
,

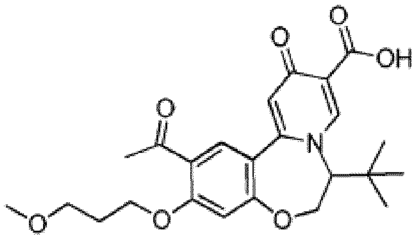
;
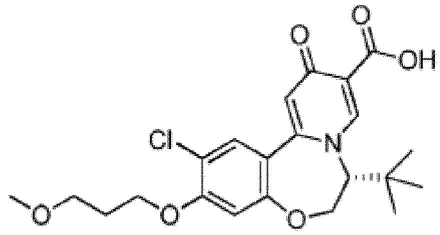

und

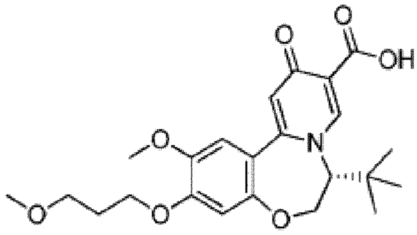
;

oder ein pharmazeutisch annehmbares Salz der Verbindung; wobei in Formel (I):

$R_1$ OH, CN, $NH_2$ oder

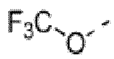

ist oder aus der aus

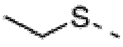, 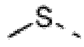,

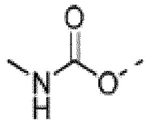, 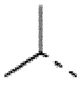, 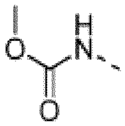, 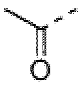, 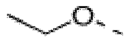, 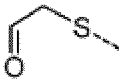, 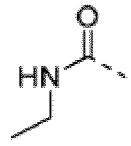,

$C_{2-5}$-Alkenyl, $C_{2-5}$-Heteroalkenyl, $C_{3-6}$-Cycloalkyl und 3- bis 6-gliedrigem Heterocycloalkyl bestehenden Gruppe ausgewählt ist, wobei

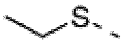, 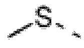,

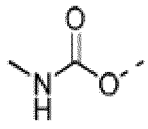, 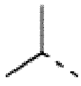, 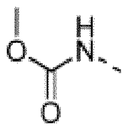, 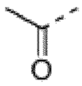, 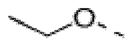, 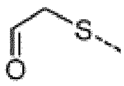, 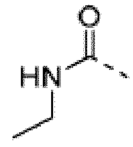,

das $C_{2-5}$-Alkenyl, $C_{2-5}$-Heteroalkenyl, $C_{3-6}$-Cycloalkyl und 3- bis 6-gliedrige Heterocycloalkyl jeweils gegebenenfalls mit 1, 2 oder 3 R substituiert sind;
m = 0, 1, 2, 3, 4 oder 5 ist; und
$R_1$ nicht OH, CN oder $NH_2$ ist, wenn m = 0 ist;
oder

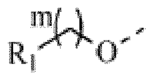 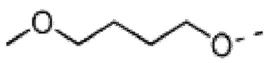

ist;
$R_2$ H, OH, CN, $NH_2$ oder Halogen ist oder aus der aus $C_{1-3}$-Alkyl, $C_{1-3}$-Heteroalkyl, $C_{3-6}$-Cycloalkyl und 3- bis 6-gliedrigem Heterocycloalkyl bestehenden Gruppe ausgewählt ist, wobei das $C_{1-3}$-Alkyl, $C_{1-3}$-Heteroalkyl, $C_{3-6}$-Cycloalkyl und 3- bis 6-gliedrige Heterocycloalkyl jeweils gegebenenfalls mit 1, 2 oder 3 R substituiert sind;
$R_3$ aus der aus $C_{1-6}$-Alkyl und $C_{3-6}$-Cycloalkyl bestehenden Gruppe ausgewählt ist, wobei das $C_{1-6}$-Alkyl und $C_{3-6}$-Cycloalkyl jeweils gegebenenfalls mit 1, 2 oder 3 R substituiert sind;
R H, Halogen, OH, CN oder $NH_2$ ist oder aus der aus $C_{1-3}$-Alkyl und $C_{1-3}$-Heteroalkyl bestehenden Gruppe ausgewählt ist, wobei das $C_{1-3}$-Alkyl und $C_{1-3}$-Heteroalkyl jeweils gegebenenfalls mit 1, 2 oder 3 R substituiert sind;
R' aus der aus F, Cl, Br, I, OH, CN, $NH_2$, $CH_3$, $CH_3CH_2$, $CH_3O$, $CF_3$, $CHF_2$ und $CH_2F$ bestehenden Gruppe ausgewählt ist; und
das "Hetero" sich auf ein Heteroatom oder eine Heteroatomgruppe bezieht; wobei das "Hetero" in $C_{2-5}$-Heteroalkenyl, 3- bis 6-gliedrigem Heterocycloalkyl und $C_{1-3}$-Heteroalkyl jeweils unabhängig aus der aus -C(=O)N(R)-, -N(R)-, -C(=NR)-, -(R)C=N-, $-S(=O)_2N(R)-$, -S(=O)N(R)-, N, -O-, -S-, =O, =S, -C(=O)O-, -C(=O)-, -C(=S)-, -S(=O)-, $-S(=O)_2-$ und - N(R)C(=O)N(R)- bestehenden Gruppe ausgewählt ist;
wobei in jedem der obigen Fälle die Anzahl an Heteroatomen oder Heteroatomgruppen jeweils unabhängig 1, 2 oder 3 ist.

**2.** Verbindung oder pharmazeutisch annehmbares Salz nach Anspruch 1, worin R H, F, Cl, Br, I, OH, CN, $NH_2$, $CH_3$, $CH_3CH_2$, $CH_3O$, $CF_3$, $CHF_2$ oder $CH_2F$ ist.

**3.** Verbindung oder pharmazeutisch annehmbares Salz nach Anspruch 1 oder 2, worin $R_1$ OH, CN oder $NH_2$ ist oder aus der aus $C_{2-3}$-Alkenyl, $C_{2-3}$-Heteroalkenyl, $C_{3-6}$-Cycloalkyl und 3- bis 6-gliedrigem Heterocycloalkyl bestehenden Gruppe ausgewählt ist, wobei das $C_{2-3}$-Alkenyl, $C_{2-3}$-Heteroalkenyl, $C_{3-6}$-Cycloalkyl und 3- bis 6-gliedrige Heterocycloalkyl jeweils gegebenenfalls mit 1, 2 oder 3 R substituiert sind.

**4.** Verbindung oder pharmazeutisch annehmbares Salz nach Anspruch 1 oder 2, worin $R_1$ OH, CN oder $NH_2$ ist oder aus der aus

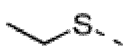, 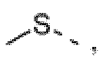, 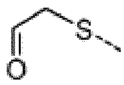,

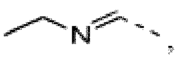, 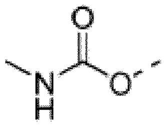, 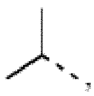, 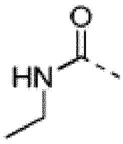, 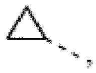, 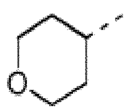, 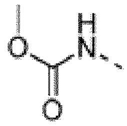, 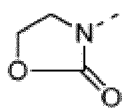,

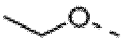 und 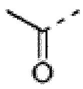

bestehenden Gruppe ausgewählt ist, wobei das

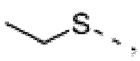, 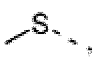, 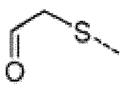, 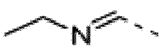,

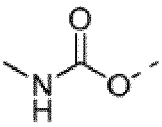, 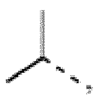, 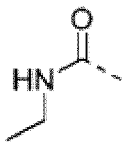, 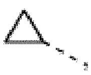, 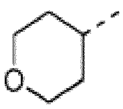, 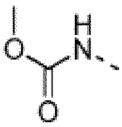, 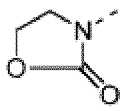, 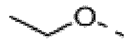

und

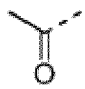

jeweils gegebenenfalls mit 1, 2 oder 3 R substituiert sind.

**5.** Verbindung oder pharmazeutisch annehmbares Salz nach Anspruch 1 oder 2, worin $R_1$ aus der aus OH, CN, $NH_2$,

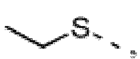, 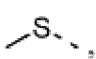, 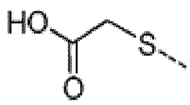,

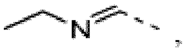, 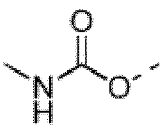, 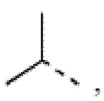, 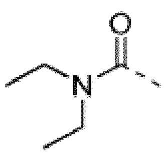, 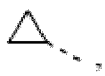, 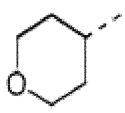, 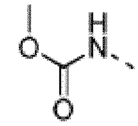,

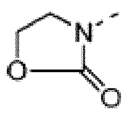, 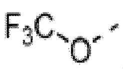, 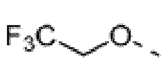 und 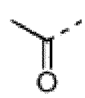

bestehenden Gruppe ausgewählt ist.

6. Verbindung oder pharmazeutisch annehmbares Salz nach Anspruch 1 oder 2, worin $R_2$ H, OH, CN, $NH_2$ oder Halogen ist oder aus der aus $C_{1-3}$-Alkyl, $C_{1-3}$-Heteroalkyl und $C_{3-6}$-Cycloalkyl bestehenden Gruppe ausgewählt ist, wobei das $C_{1-3}$-Alkyl, $C_{1-3}$-Heteroalkyl und $C_{3-6}$-Cycloalkyl jeweils gegebenenfalls mit 1, 2 oder 3 R substituiert sind, worin $R_2$ gegebenenfalls H, OH, CN, $NH_2$, F, Cl, Br oder I ist oder aus der Gruppe bestehend aus $CH_3$,

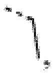, 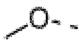, 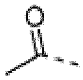 und 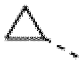

ausgewählt ist, wobei das $CH_3$,

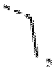, 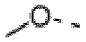, 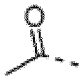 und 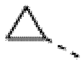

jeweils gegebenenfalls mit 1, 2 oder 3 R substituiert sind, worin $R_2$ gegebenenfalls aus der aus Cl, Br, CN, $CH_3$,

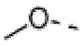, 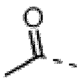 und 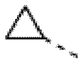

bestehenden Gruppe ausgewählt ist.

7. Verbindung oder pharmazeutisch annehmbares Salz nach Anspruch 1 oder 2, worin $R_3$ aus der aus $C_{1-6}$-Alkyl und $C_{3-6}$-Cycloalkyl bestehenden Gruppe ausgewählt ist, wobei das $C_{1-6}$-Alkyl und $C_{3-6}$-Cycloalkyl jeweils gegebenenfalls mit 1, 2 oder 3 R substituiert sind, wobei $R_3$ gegebenenfalls aus der aus

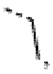, 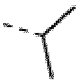, 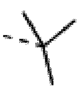, 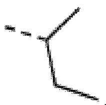, 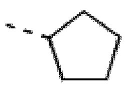 und 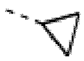

bestehenden Gruppe ausgewählt ist.

8. Verbindung oder pharmazeutisch annehmbares Salz nach Anspruch 1 oder 2, worin m = 0, 1, 2, 3 oder 4 ist; und $R_1$ nicht OH, CN oder $NH_2$ ist, wenn m = 0 ist.

9. Verbindung oder pharmazeutisch annehmbares Salz nach Anspruch 1 oder 2, worin die Gruppierung

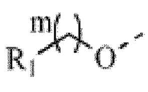

aus der aus Folgendem bestehenden Gruppe ausgewählt ist:

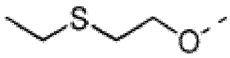, 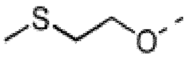,

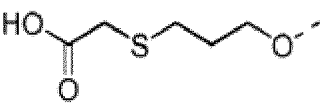, 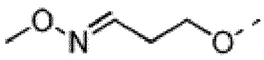, 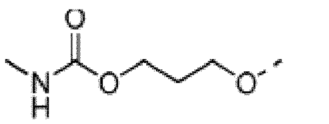,

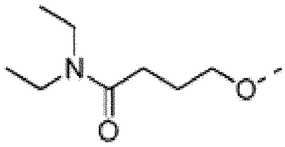, 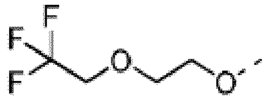, 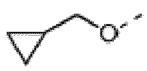, 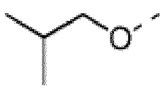, 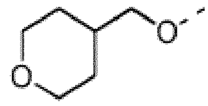,

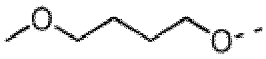, 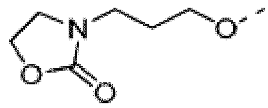, 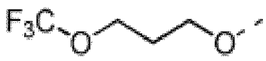, 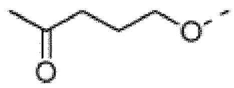

und

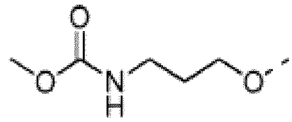.

**10.** Verbindung der Formel (II), (III) oder (IV) oder ein pharmazeutisch annehmbares Salz der Verbindung:

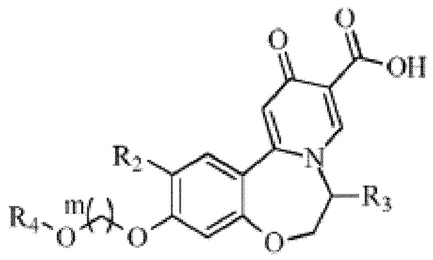

(II)

,

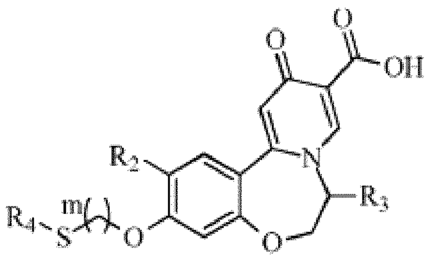

(III)

und

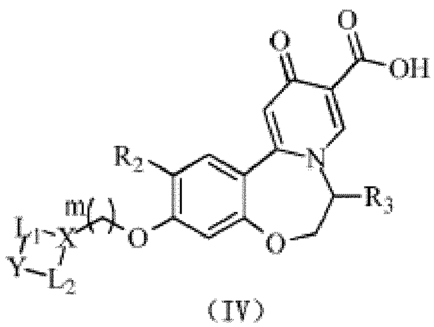

(IV)

,

worin

$R_4$ H ist oder aus der aus $C_3$-Alkyl und $C_{1-3}$-Heteroalkyl bestehenden Gruppe ausgewählt ist, wobei das $C_3$-Alkyl und $C_{1-3}$-Heteroalkyl jeweils gegebenenfalls mit 1, 2 oder 3 R substituiert sind;
X aus der aus C und N bestehenden Gruppe ausgewählt ist;
Y aus der aus O und C bestehenden Gruppe ausgewählt ist;
$L_1$ und $L_2$ jeweils unabhängig aus der aus einer Einfachbindung, $-(CH_2)_n-$ und -C(=O)- bestehenden Gruppe ausgewählt sind;
mit der Maßgabe, dass $L_1$ und $L_2$ nicht beide eine Einfachbindung sind;
n = 1 oder 2 ist;

m, R, $R_2$, $R_3$ und das "Hetero" in $C_{1-3}$-Heteroalkyl wie in den Ansprüchen 1 bis 8 definiert sind und $R_4$ nicht H ist, wenn m = 0 ist.

**11.** Verbindung oder pharmazeutisch annehmbares Salz nach Anspruch 1, wobei die Verbindung aus der aus den folgenden bestehenden Gruppe ausgewählt ist:

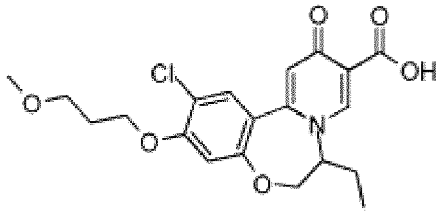
,

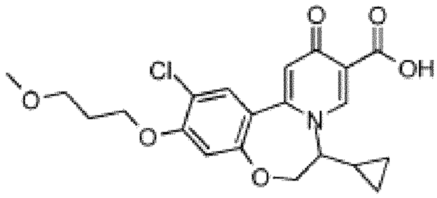
,
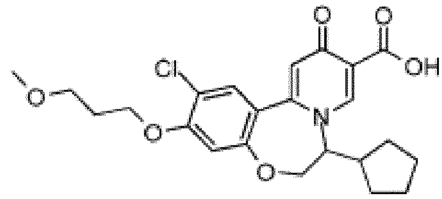
,

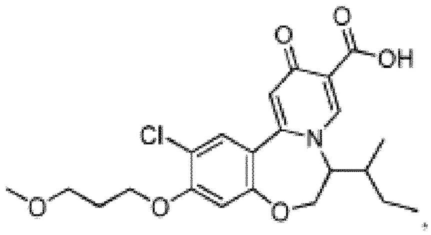
,

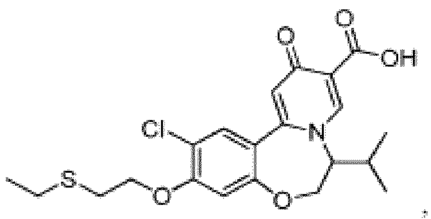
,
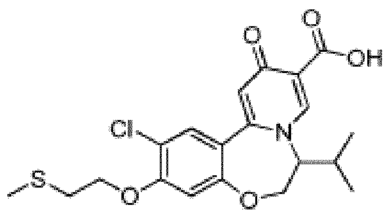
,

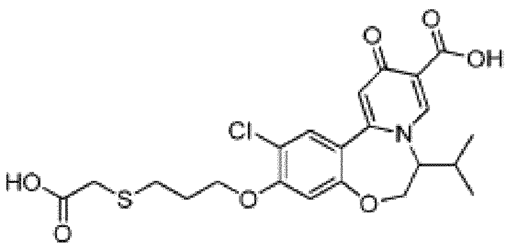
,
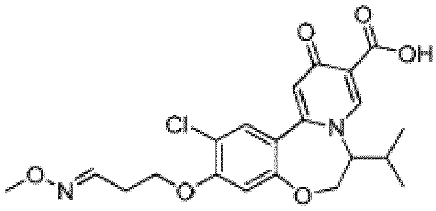
,

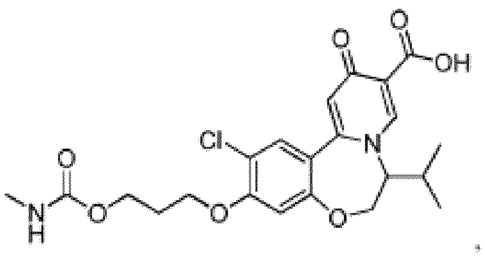,
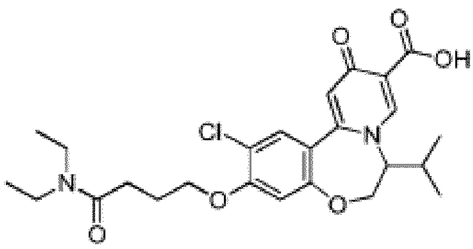,

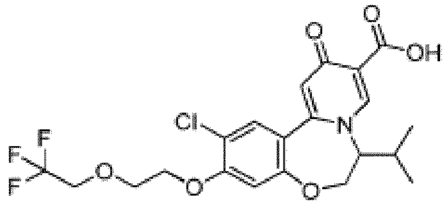,

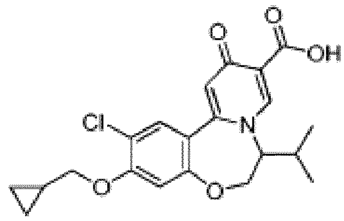,
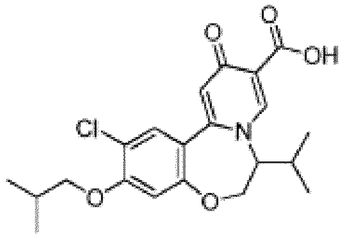,

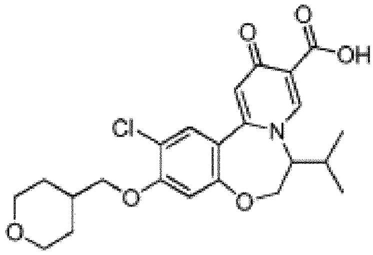.

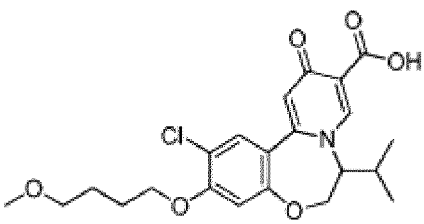,
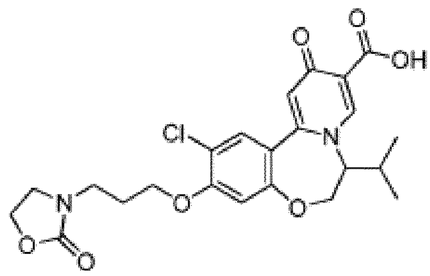,

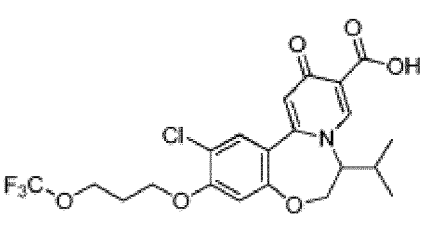,
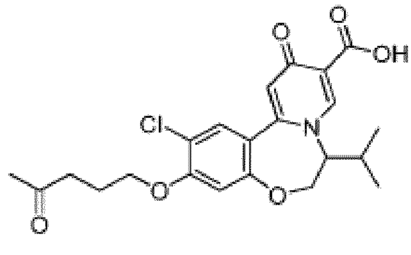,

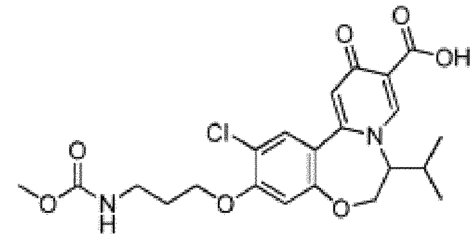

,

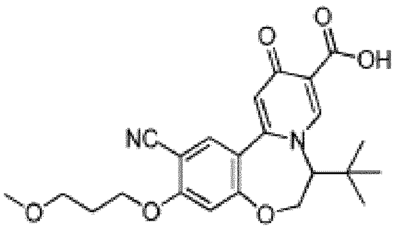

,

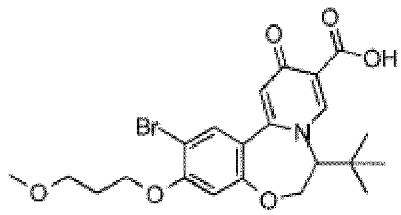

,

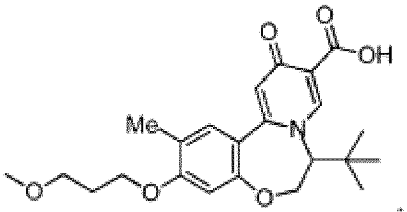

,

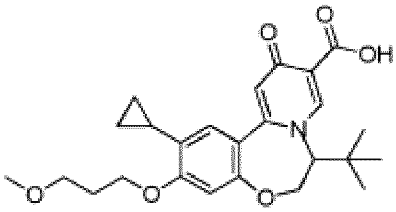

und

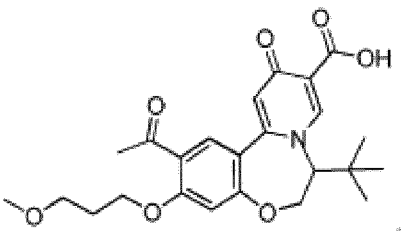

12. Verbindung oder pharmazeutisch annehmbares Salz nach Anspruch 1, wobei die Verbindung aus der aus den folgenden bestehenden Gruppe ausgewählt ist:

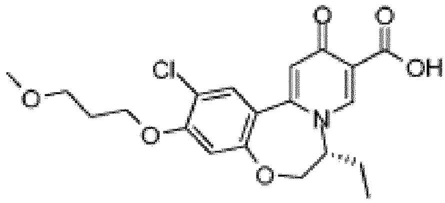

,

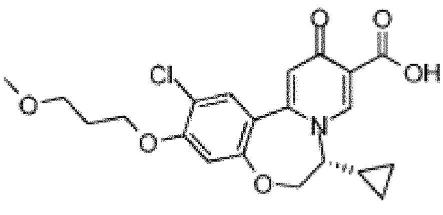

,

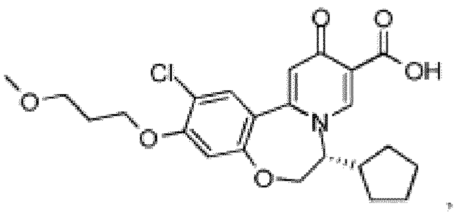

,

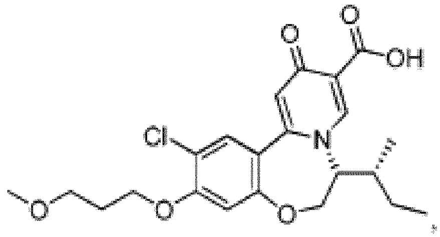,

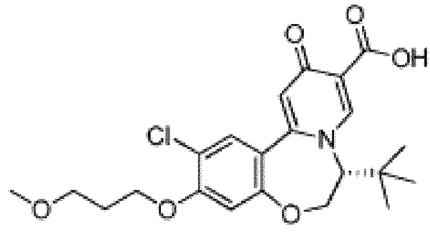,

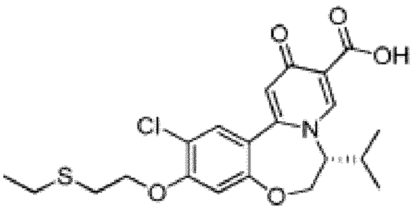,

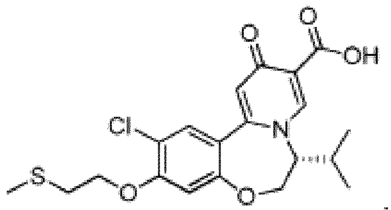,

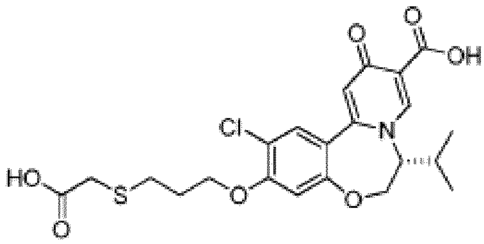,

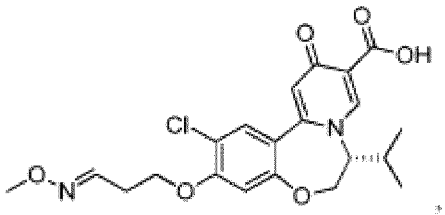,

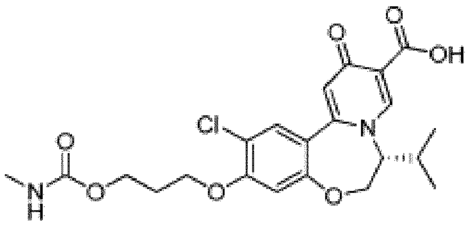,

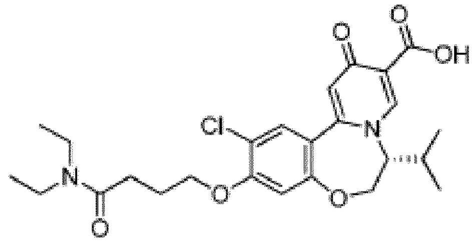,

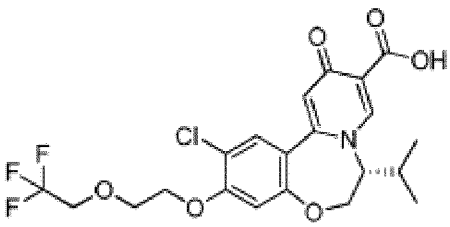,

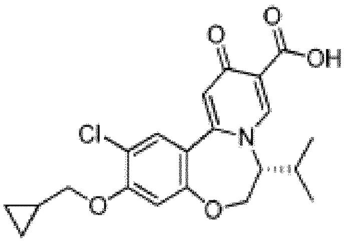,

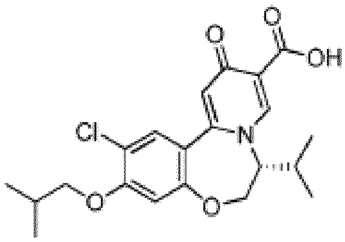,

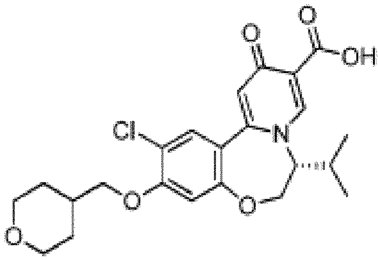,

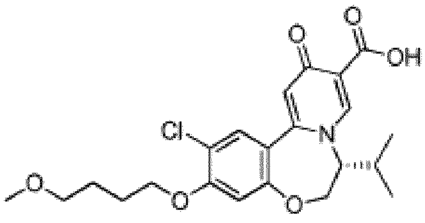, 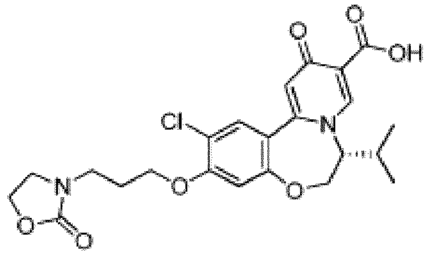,

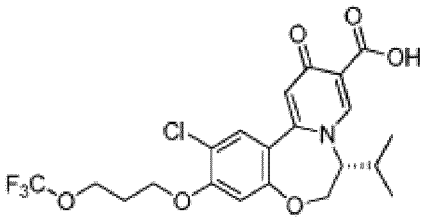, 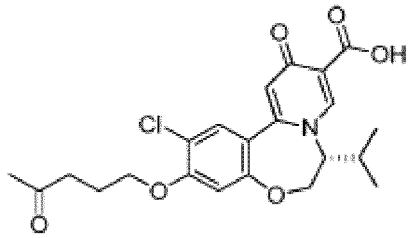,

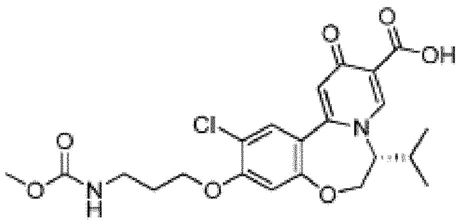, 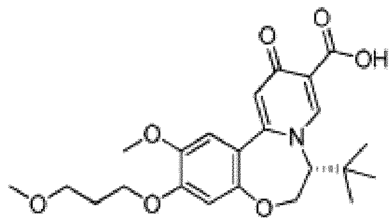,

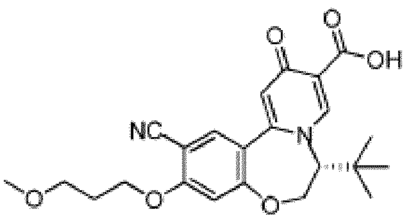, 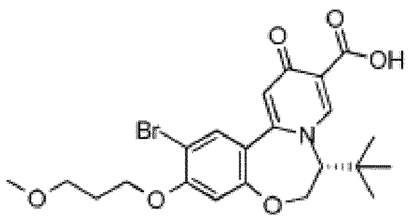,

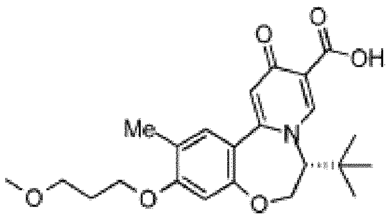, 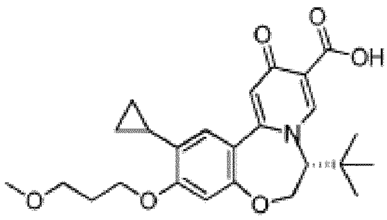

und

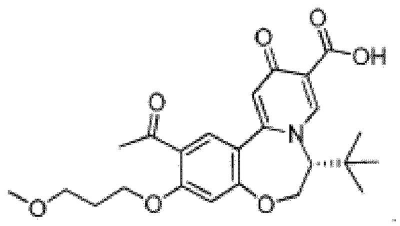

13. Pharmazeutische Zusammensetzung, die eine therapeutisch wirksame Menge einer Verbindung oder eines pharmazeutisch annehmbaren Salzes nach einem der Ansprüche 1 bis 12 und einen pharmazeutisch annehmbaren Träger umfasst.

14. Verbindung oder pharmazeutisch annehmbares Salz nach einem der Ansprüche 1 bis 12 oder pharmazeutische Zusammensetzung nach Anspruch 13 zur Verwendung bei der Behandlung von Hepatitis B.

## Revendications

1. Composé de formule (I) :

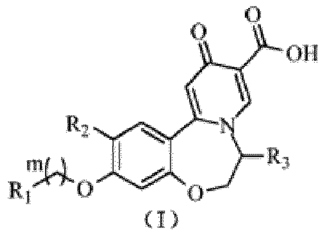

(I)

,

ou un composé choisi dans le groupe constitué de :

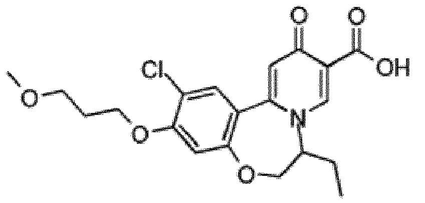

,

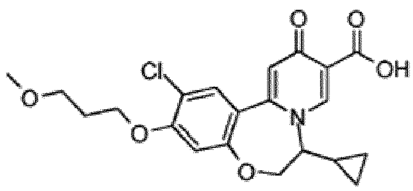

,

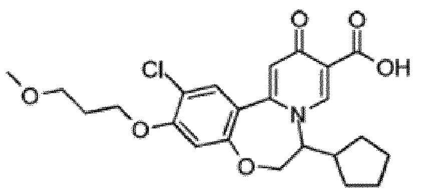

,

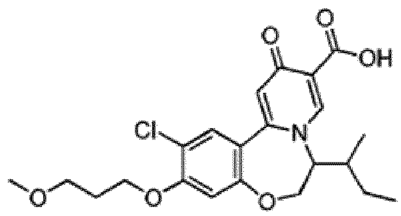

,

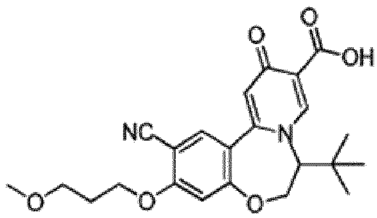

,

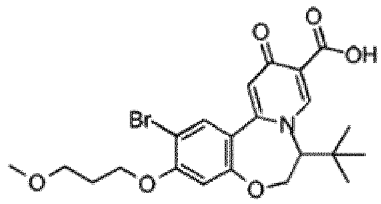

,

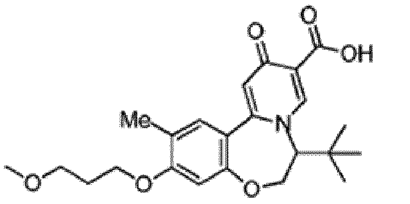 , 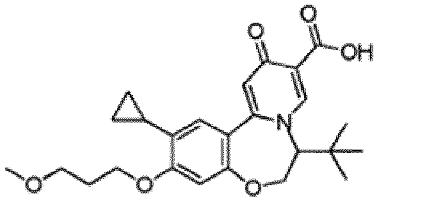 ,

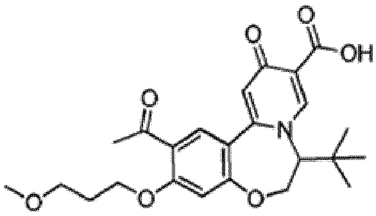 ; 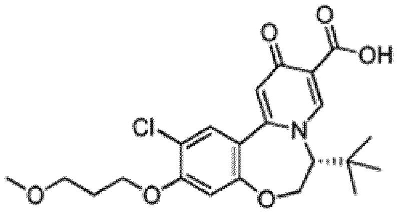

et

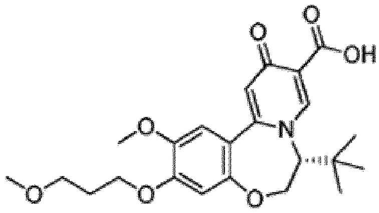 ;

ou un sel pharmaceutiquement acceptable du composé ; dans lequel, dans la formule (I) :

$R_1$ est OH, CN, $NH_2$,

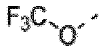 ,

ou est choisi dans le groupe constitué de

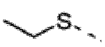 , 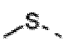 , 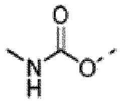 , 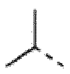 , 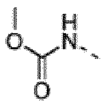 , 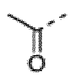 ,

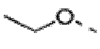 , 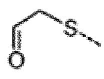 , 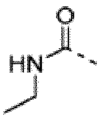 ,

un alcényle en $C_{2-5}$, un hétéroalcényle en $C_{2-5}$, un cycloalkyle en $C_{3-6}$ et un hétérocycloalkyle de 3 à 6 chaînons, dans lequel chacun des

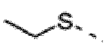 , 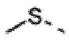 , 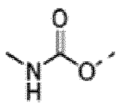 ,

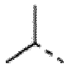, 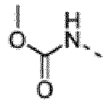, 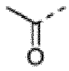, 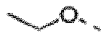, 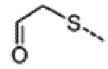, 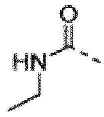,

alcényle en $C_{2-5}$, hétéroalcényle en $C_{2-5}$, cycloalkyle en $C_{3-6}$ et hétérocycloalkyle de 3 à 6 chaînons est optionnellement substitué par 1, 2 ou 3 R ;
m vaut 0, 1, 2, 3,4 ou 5 ; et
$R_1$ n'est pas OH, CN, $NH_2$ à condition que m vaille 0 ;
ou,

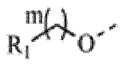 est 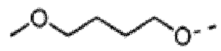 ;

$R_2$ est H, OH, CN, $NH_2$, un halogène, ou est choisi dans le groupe constitué d'un alkyle en $C_{1-3}$, d'un hétéroalkyle en $C_{1-3}$, d'un cycloalkyle en $C_{3-6}$ et d'un hétérocycloalkyle de 3 à 6 chaînons, dans lequel chacun des alkyle en $C_{1-3}$, hétéroalkyle en $C_{1-3}$, cycloalkyle en $C_{3-6}$ et hétérocycloalkyle de 3 à 6 chaînons est optionnellement substitué par 1, 2 ou 3 R ;
$R_3$ est choisi dans le groupe constitué d'un alkyle en $C_{1-6}$ et d'un cycloalkyle en $C_{3-6}$, dans lequel chacun des alkyle en $C_{1-6}$ et cycloalkyle en $C_{3-6}$ est optionnellement substitué par 1, 2 ou 3 R ;
R est H, un halogène, OH, CN, $NH_2$, ou est choisi dans le groupe constitué d'un alkyle en $C_{1-3}$ et d'un hétéroalkyle en $C_{1-3}$, dans lequel chacun des alkyle en $C_{1-3}$ et hétéroalkyle en $C_{1-3}$ est optionnellement substitué par 1, 2 ou 3 R';
R' est choisi dans le groupe constitué de F, Cl, Br, I, OH, CN, $NH_2$, $CH_3$, $CH_3CH_2$, $CH_3O$, $CF_3$, $CHF_2$ et $CH_2F$ ;
l'« hétéro » fait référence à un hétéroatome ou un groupe hétéroatomique ; l'« hétéro » dans 1' hétéroalcényle en $C_{2-5}$, l'hétérocycloalkyle de 3 à 6 chaînons, l'hétéroalkyle en $C_{1-3}$ est indépendamment choisi dans le groupe constitué de - C(=O)N(R)-, -N(R)-, -C(=NR)-, -(R)C=N-, $-S(=O)_2N(R)-$, - S(=O)N(R)-, N, -O-, -S-, =O, =S, - C(=O)O-, -C(=O)-, - C(=S)-, -S(=O)-, $-S(=O)_2-$ et -N(R)C(=O)N(R)- ;
dans l'un quelconque des cas ci-dessus, le nombre de l'hétéroatome ou du groupe hétéroatomique est indépendamment 1, 2 ou 3.

**2.** Composé ou sel pharmaceutiquement acceptable selon la revendication 1, dans lequel, R est H, F, Cl, Br, I, OH, CN, $NH_2$, $CH_3$, $CH_3CH_2$, $CH_3O$, $CF_3$, $CHF_2$ ou $CH_2F$.

**3.** Composé ou sel pharmaceutiquement acceptable selon la revendication 1 ou 2, dans lequel, $R_1$ est OH, CN, $NH_2$, ou est choisi dans le groupe constitué d'un alcényle en $C_{2-3}$, d'un hétéroalcényle en $C_{2-3}$, d'un cycloalkyle en $C_{3-6}$ et d'un hétérocycloalkyle de 3 à 6 chaînons, dans lequel chacun des alcényle en $C_{2-3}$, hétéroalcényle en $C_{2-3}$, cycloalkyle en $C_{3-6}$ et hétérocycloalkyle de 3 à 6 chaînons est optionnellement substitué par 1, 2 ou 3 R.

**4.** Composé ou sel pharmaceutiquement acceptable selon la revendication 1 ou 2, dans lequel, $R_1$ est OH, CN, $NH_2$, ou est choisi dans le groupe constitué de

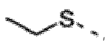, 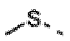, 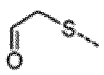,

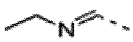, 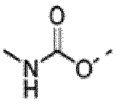, 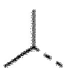, 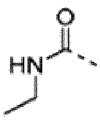, 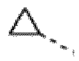, 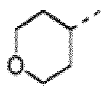, 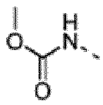, 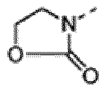,

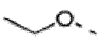 et 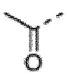,

dans lequel chacun des

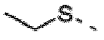, 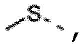, 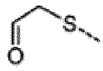,

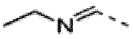, 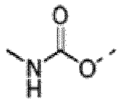, 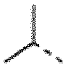, 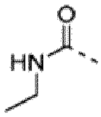, 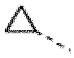, 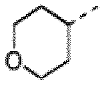, 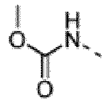, 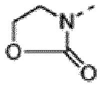,

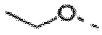 et 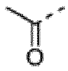

est optionnellement substitué par 1, 2 ou 3 R.

**5.** Composé ou sel pharmaceutiquement acceptable selon la revendication 1 ou 2, dans lequel, $R_1$ est choisi dans le groupe constitué de OH, CN, $NH_2$,

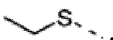, 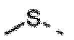, 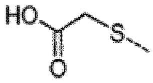,

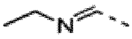, 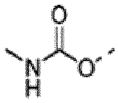, 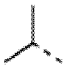, 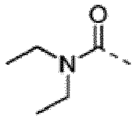, 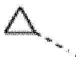, 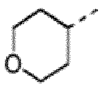, 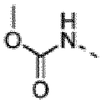, 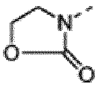,

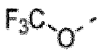, 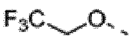 et 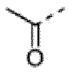.

**6.** Composé ou sel pharmaceutiquement acceptable selon la revendication 1 ou 2, dans lequel, $R_2$ est H, OH, CN, $NH_2$, un halogène, ou est choisi dans le groupe constitué d'un alkyle en $C_{1-3}$, d'un hétéroalkyle en $C_{1-3}$ et d'un cycloalkyle en $C_{3-6}$, dans lequel chacun des alkyle en $C_{1-3}$, hétéroalkyle en $C_{1-3}$ et cycloalkyle en $C_{3-6}$ est optionnellement substitué par 1, 2 ou 3 R,
optionnellement, $R_2$ est H, OH, CN, $NH_2$, F, Cl, Br, I, ou est choisi dans le groupe constitué de $CH_3$,

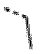, 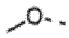, 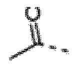

et

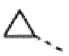,

dans lequel chacun des $CH_3$,

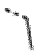, 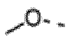, 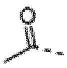 et 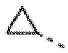

est optionnellement substitué par 1, 2 ou 3 R, optionnellement à ceci, $R_2$ est choisi dans le groupe constitué de Cl,

Br, CN, $CH_3$,

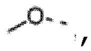, 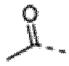 et 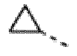.

7. Composé ou sel pharmaceutiquement acceptable selon la revendication 1 ou 2, dans lequel, $R_3$ est choisi dans le groupe constitué d'un alkyle en $C_{1-4}$ et d'un cycloalkyle en $C_{3-6}$, dans lequel chacun des alkyle en $C_{1-4}$ et cycloalkyle en $C_{3-6}$ est optionnellement substitué par 1, 2 ou 3 R, optionnellement, $R_3$ est choisi dans le groupe constitué de

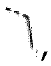, 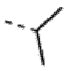, 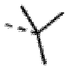, 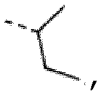, 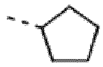 et 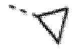.

8. Composé ou sel pharmaceutiquement acceptable selon la revendication 1 ou 2, dans lequel, m vaut 0, 1, 2, 3 ou 4 ; et $R_1$ n'est pas OH, CN, $NH_2$ à condition que m vaille 0.

9. Composé ou sel pharmaceutiquement acceptable selon la revendication 1 ou 2, dans lequel, le groupement

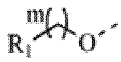

est choisi dans le groupe constitué de

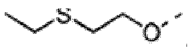, 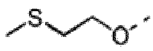,

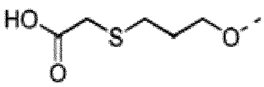, 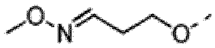, 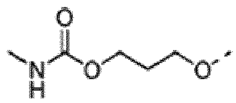,

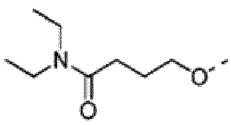, 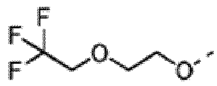, 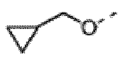, 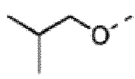, 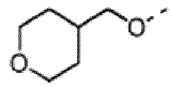,

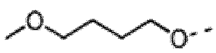, 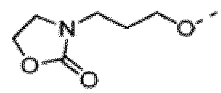, 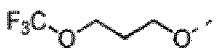, 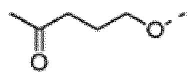

et

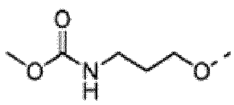.

10. Composé de formule (II), (III) ou (IV), ou un sel pharmaceutiquement acceptable du composé :

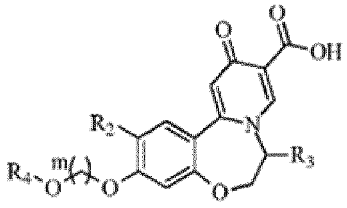

(II)

,

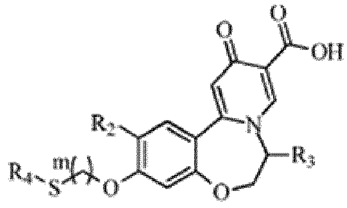

(III)

et

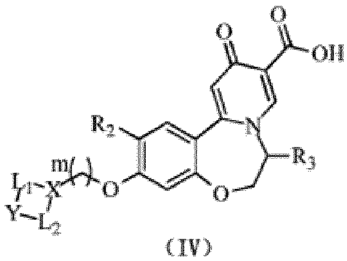

(IV)

,

dans lequel,

$R_4$ est H, ou est choisi dans le groupe constitué d'un alkyle en $C_3$ et d'un hétéroalkyle en $C_{1-3}$, dans lequel chacun des alkyle en $C_3$ et hétéroalkyle en $C_{1-3}$ est optionnellement substitué par 1, 2 ou 3 R ;
X est choisi dans le groupe constitué de C et N ;
Y est choisi dans le groupe constitué de O et C ;
chacun de $L_1$ et $L_2$ est indépendamment choisi dans le groupe constitué d'une liaison simple, de $-(CH_2)_n-$ et de -C(=O)- ;
à condition que $L_1$ et $L_2$ ne soient pas tous deux une liaison simple ;
n vaut 1 ou 2 ;
m, R, $R_2$, $R_3$ et l'« hétéro » dans l' hétéroalkyle en $C_{1-3}$ sont tels que définis dans les revendications 1 à 8 ; et $R_4$ n'est pas H à condition que m vaille 0.

**11.** Composé ou sel pharmaceutiquement acceptable selon la revendication 1, dans lequel, le composé est choisi dans le groupe constitué de :

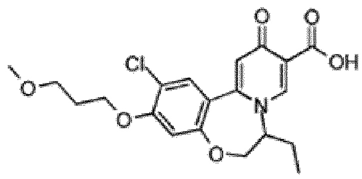

,

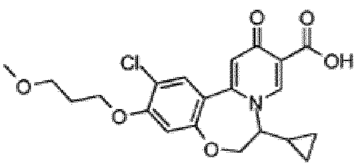

,

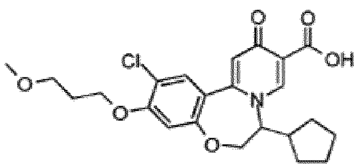

,

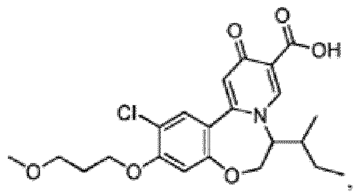,

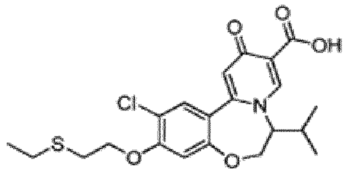, 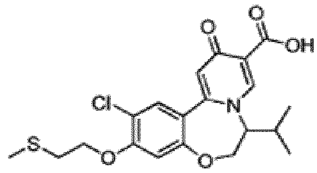,

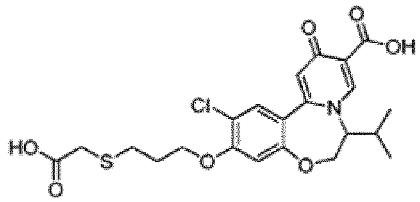, 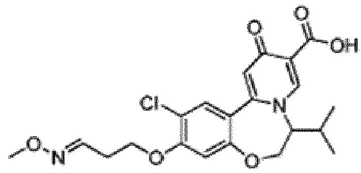,

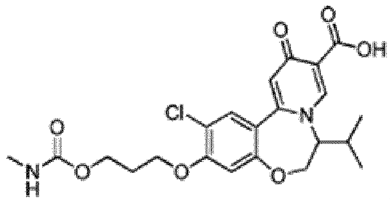, 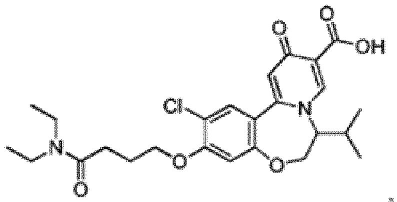,

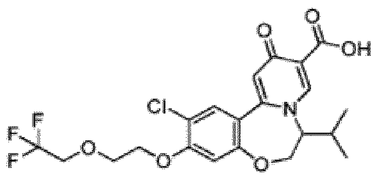,

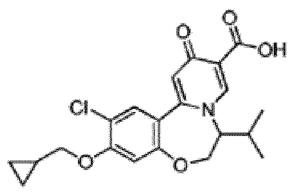, 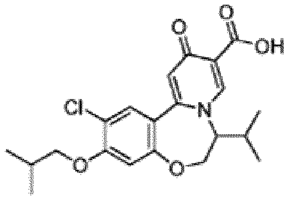,

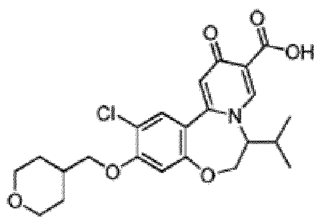,

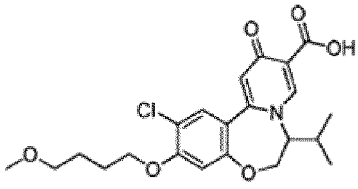,
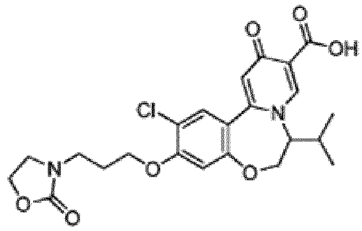,

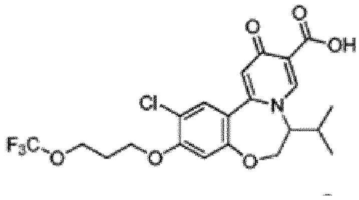,
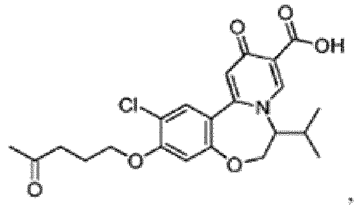,

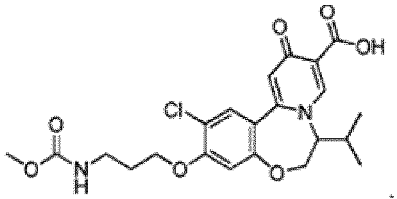.

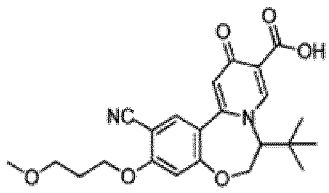,
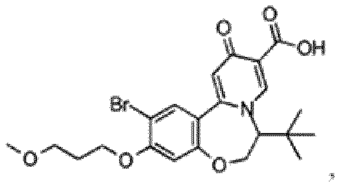,

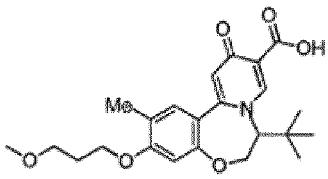,
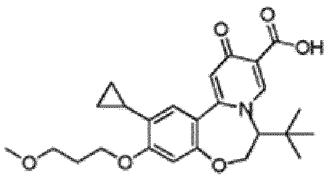,

et

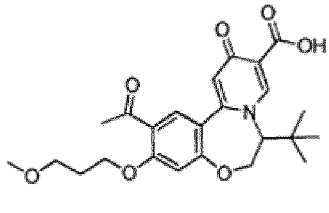.

**12.** Composé ou sel pharmaceutiquement acceptable selon la revendication 1, dans lequel le composé est choisi dans le groupe constitué de :

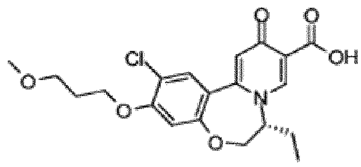,

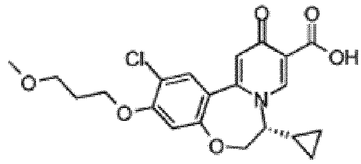, 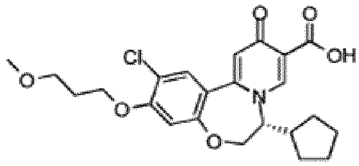,

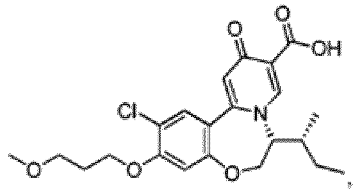, 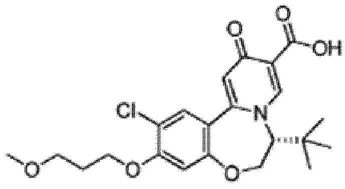,

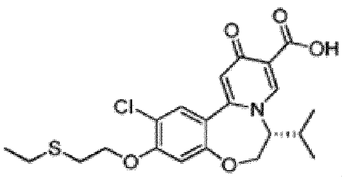, 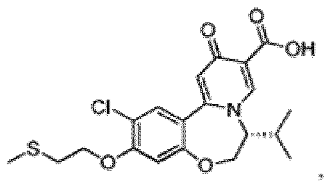,

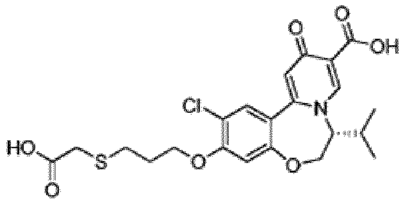, 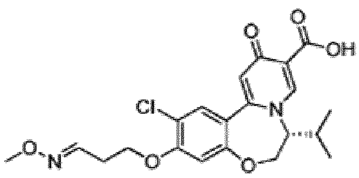,

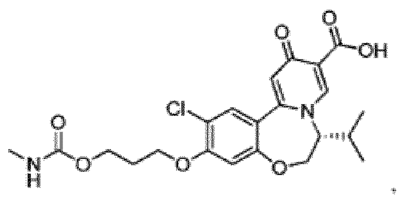, 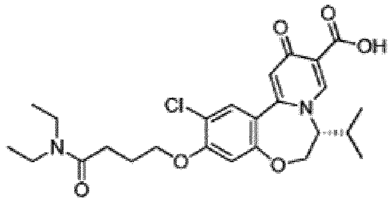,

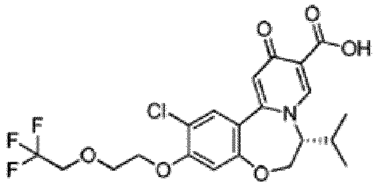,

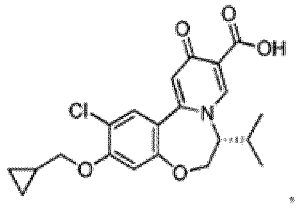,

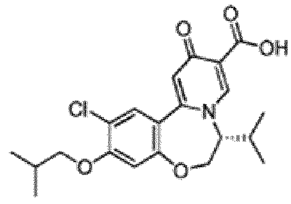,

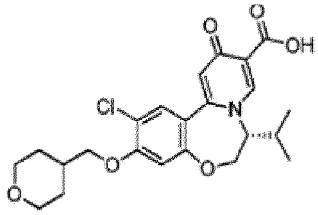,

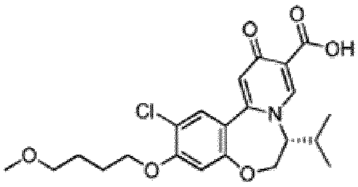,

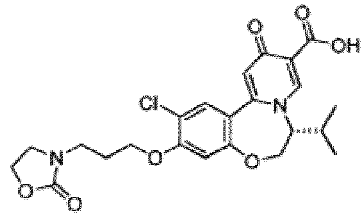,

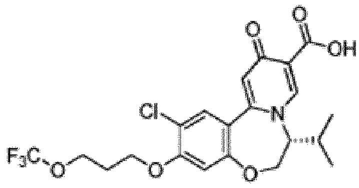,

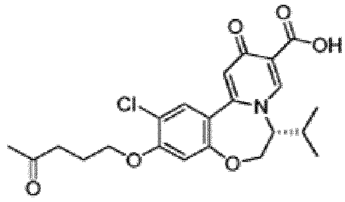,

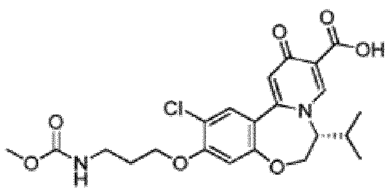,

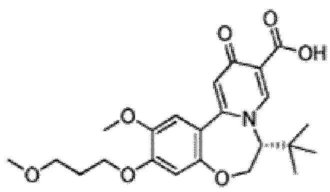,

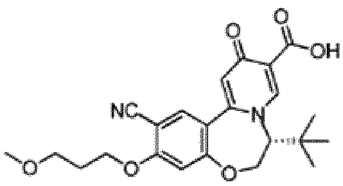,

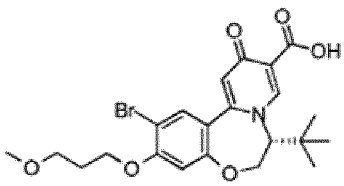,

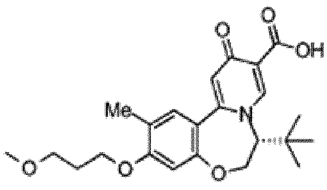,

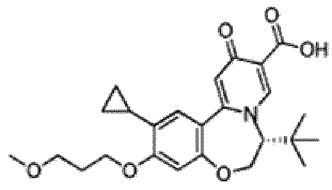

et

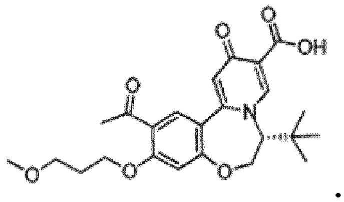

13. Composition pharmaceutique comprenant une quantité thérapeutiquement efficace du composé ou du sel pharmaceutiquement acceptable selon l'une quelconque des revendications 1 à 12, et un vecteur pharmaceutiquement acceptable.

14. Composé ou sel pharmaceutiquement acceptable selon l'une quelconque des revendications 1 à 12 ou composition pharmaceutique selon la revendication 13 pour une utilisation dans le traitement de l'hépatite B.

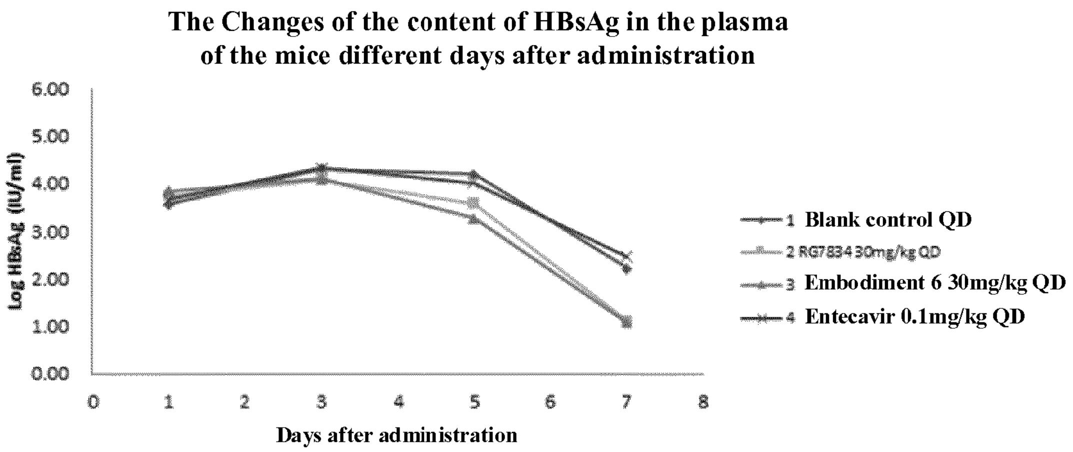


Figure 1

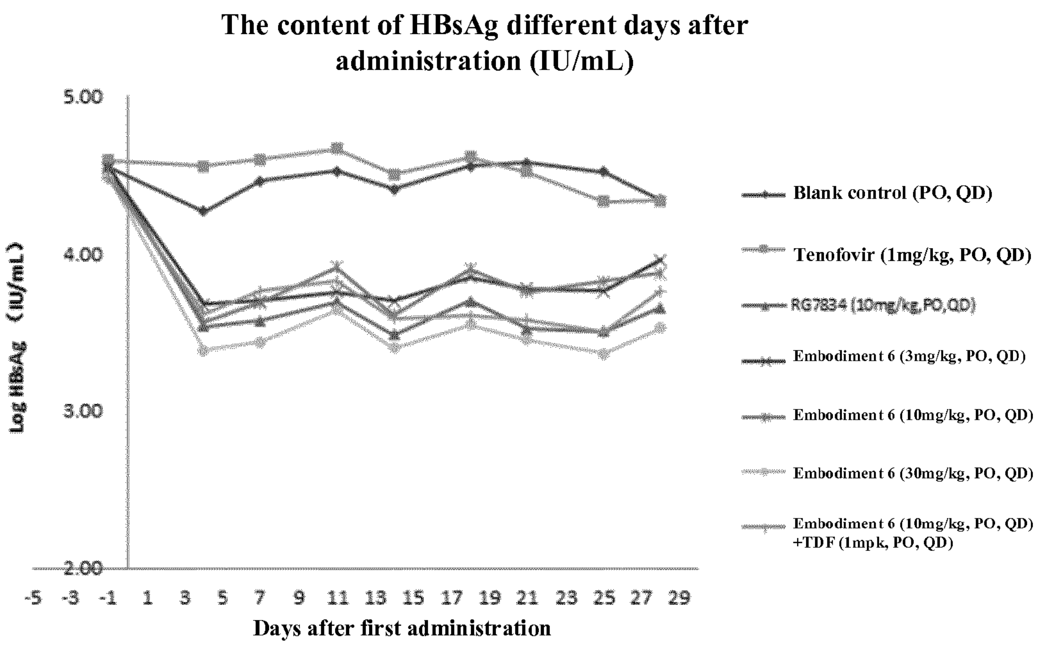


Figure 2

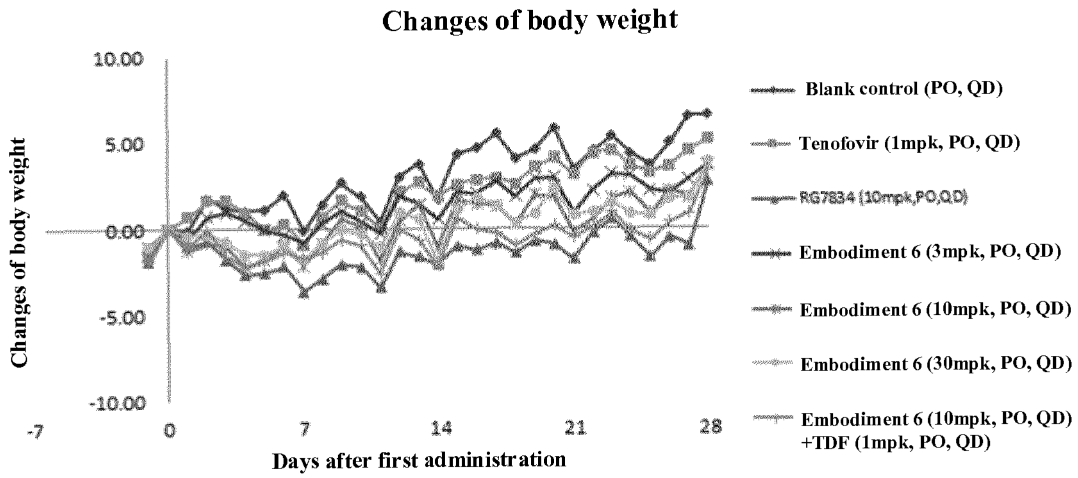
Changes of body weight
10.00
5.00
0.00
-5.00
-10.00
Changes of body weight
-7
0
7
14
21
28
Days after first administration
Blank control (PO, QD)
Tenofovir (1mpk, PO, QD)
RG7834 (10mpk,PO,QD)
Embodiment 6 (3mpk, PO, QD)
Embodiment 6 (10mpk, PO, QD)
Embodiment 6 (30mpk, PO, QD)
Embodiment 6 (10mpk, PO, QD)
+TDF (1mpk, PO, QD)

Figure 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017013046 A1
- WO 2018085619 A
- WO 2018022282 A


### Non-patent literature cited in the description


- **BERGE et al.** Pharmaceutical Salts. *Journal of Pharmaceutical Science,* 1977, vol. 66, 1-19
- **REMINGTON.** The Science and Practice of Pharmacy. Lippincott, Williams & Wilkins, 2005